(19) Europäisches Patentamt
European Patent Office
Office européen des brevets

(11) EP 1 935 409 B1

(12) EUROPEAN PATENT SPECIFICATION

(45) Date of publication and mention of the grant of the patent:
**27.06.2012 Bulletin 2012/26**

(51) Int Cl.:
***A61K 47/48*** *(2006.01)* ***A61K 9/127*** *(2006.01)*
***A61K 9/16*** *(2006.01)* ***A61K 9/51*** *(2006.01)*
***C07K 14/705*** *(2006.01)*

(21) Application number: **07017593.0**

(22) Date of filing: **07.09.2007**

# (54) Synthetic platelets

Synthetische Blutplättchen

Plaquettes synthétiques

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **07.09.2006 US 842647 P**

(43) Date of publication of application:
**25.06.2008 Bulletin 2008/26**

(73) Proprietor: **Gyongyossy-Issa, Maria I. C.**
**Vancouver, BC V6K 3V3 (CA)**

(72) Inventors:
- **Gyongyossy-Issa, Maria I.C.**
  **Vancouver, British Columbia**
  **V6K 3V3 (CA)**
- **Kizhakkedathu, Jayachandran N.**
  **Vancouver, British Columbia**
  **V6T 1Z4 (CA)**
- **Constantinescu, Iren**
  **Vancouver, British Columbia**
  **V6P 6Y1 (CA)**
- **Campell, William**
  **Richmond, British Columbia**
  **V7E 6S2 (CA)**
- **Del Carpio Munoz, Carlos A.**
  **Katahira 3-36, Aoba**
  **Sendai 980-0812 (JP)**

(74) Representative: **Engelhard, Markus**
**Boehmert & Boehmert**
**Pettenkoferstrasse 20-22**
**80336 München (DE)**

(56) References cited:
**EP-A- 0 160 266** **EP-A- 0 255 206**
**EP-A- 0 894 807** **EP-A- 1 262 490**
**WO-A-00/28972** **WO-A-92/16225**
**WO-A-03/075977** **US-A- 5 534 259**

- **KITAGUCHI T ET AL: "CHARACTERIZATION OF LIPOSOMES CARRYING VON WILLEBRAND FACTOR-BINDING DOMAIN OF PLATELET GLYCOPROTEIN IBALPHA: A POTENTIALSUBSTITUTE FOR PLATELET TRANSFUSION" BIOCHEMICAL AND BIOPHYSICAL RESEARCH COMMUNICATIONS, ACADEMIC PRESS INC. ORLANDO, FL, US, vol. 261, no. 3, 11 August 1999 (1999-08-11), pages 784-789, XP001019106 ISSN: 0006-291X**
- **NISHIYA TAKAKO ET AL: "Reconstitution of adhesive properties of human platelets in liposomes carrying both recombinant glycoproteins Ia/IIa and Ib alpha under flow conditions: specific synergy of receptor-ligand interactions." BLOOD 1 JUL 2002, vol. 100, no. 1, 1 July 2002 (2002-07-01), pages 136-142, XP002480206 ISSN: 0006-4971**
- **NISHIYA T ET AL: "TARGETING OF LIPOSOMES CARRYING RECOMBINANT FRAGMENTS OF PLATELET MEMBRANE GLYCOPROTEIN IBALPHA TO IMMOBILIZED VON WILLEBRAND FACTOR UNDER FLOW CONDITIONS" BIOCHEMICAL AND BIOPHYSICAL RESEARCH COMMUNICATIONS, ACADEMIC PRESS INC. ORLANDO, FL, US, vol. 270, no. 3, 21 April 2000 (2000-04-21), pages 755-760, XP001019105 ISSN: 0006-291X**
- **LEE D H ET AL: "Novel treatment modalities: new platelet preparations and substitutes." BRITISH JOURNAL OF HAEMATOLOGY SEP 2001, vol. 114, no. 3, September 2001 (2001-09), pages 496-505, XP002480207 ISSN: 0007-1048**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 935 409 B1

## Description

### FIELD OF THE INVENTION

The invention relates to transfusion medicine and related technologies. More specifically, it relates to synthetic platelet substitutes and antithrombotic molecules.

### BACKGROUND OF THE INVENTION

Platelets, or thrombocytes, are the blood components involved in the cellular mechanisms leading to blood clotting. Low platelet levels, as well as platelet dysfunction, predisposes an individual to bleeding, while high levels may increase the risk of thrombosis. Platelet transfusions are traditionally given to those undergoing chemotherapy for leukemia, those with aplastic anemia, AIDS, hypersplenism, idiopathic thrombocytopenic purpura (ITP), sepsis, disseminated intravascular coagulation (DIC), or to those who have undergone surgeries such as cardiopulmonary bypass.

Platelets are isolated from whole blood donations and have a very short shelf life, typically five or seven days. Since there are no effective long-term preservative solutions, platelets lose potency quickly and must be used when fresh. This results in frequent supply problems, which are further compounded by the need for donation testing which can take up a full day of shelf life time.

In view of this short supply, a synthetic platelet substitute or artificial platelet would be highly desirable as an alternate transfusion product. The advantages would be numerous, including virtually indefinite shelf-life and easy storage. Moreover, artificial platelets would not require infectious disease testing or assessment to determine whether the platelets are still viable for transfusion. Such a material could extend the numbers of platelets needed to control acute bleeding, or to reduce the donor exposure of a poly-transfused patient.

### SUMMARY OF THE INVENTION

Accordingly, an object of the present invention is to provide a synthetic platelet substitute that can interact with platelets by ligand binding and facilitate thrombus formation. As a further object, the inventors have sought to provide an antithrombotic agent.

According to an aspect of the present invention, there is provided a method for preparing a synthetic platelet substitute that interacts with platelets and the (sub)endothelium, said synthetic platelet substitute comprising a receptor molecule and a carrier molecule, said method comprising:

a. preparing a carrier molecule comprising lipidic particles with a cross-linked surface mesh by

    i. preparing lipidic particles comprising pharmaceutically acceptable lipids,
    ii. binding hydrophilic polymer chains to the surface of the lipidic particles, and
    iii. cross-linking the hydrophilic polymer chains to form the cross-linked surface mesh wherein said cross-linking comprises cross-Linking tree ends at the hydrophilic polymer chains with a cross-linking; and

b. attaching at least one receptor molecule to the surface of the carrier molecule, wherein the receptor molecule is a peptide selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, analogs thereof having 90% sequence identity, and modified peptides thereof having an insertion of a Cys residue and/or a spectrophotometrically traceable amino acid and/or a poly-Gly tag consisting of 1 to 5 Gly residues.

In an embodiment the peptide is selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO: 18, SEQ ID NO:19, SEQ ID NO:20, SEQ ID NO:21, SEQ ID NO:22 and further comprises a Cys-$(Gly)_5$ tag at the N-or C-terminus thereof.

In a further embodiment, the peptide is synthesized using D-amino acids. Alternately the peptide is synthesized using L-amino acids.

In an embodiment, the at least one receptor molecule is attached by means of a covalent linkage to the carrier molecule. The attachment may be by means of a conjugate addition reaction between an amine group of the receptor molecule and free acrylate ends of a hydrogel-coated carrier molecule.

In an embodiment, a plurality of receptor molecules are attached to the surface of the carrier molecule.

In an embodiment, the lipidic particles in step (a) comprise liposomes, vesicles, micelles, or combinations thereof. The lipidic particles in step (a) may comprise liposomes, the liposomes being prepared using 1,2 dipalmitoyl-sn-glycero 3-phosphoethanolamine (DPPE), 1,2 dipalmitoyl-sn-glycero 3-phosphocholine (DPPC) and cholesterol (CHOL). The liposomes may further be prepared in a formulation having a molar ratio of about 40:30:30, respectively, of 1,2 dipalmitoyl-sn-glycero-3-phosphoethanolamine, 1,2 dipalmitoyl-sn-glycero-3-phosphocholine, and cholesterol.

In an embodiment, the hydrophilic polymer chains in step (ii) are straight-chain non-toxic polymers comprising a crosslinkable end group. The hydrophilic polymer chains in step (ii) may comprise polyethylene glycol with an acrylate end group. The molecular weight of the polyethylene glycol may be about 3400 mw

In an embodiment, the cross-linking in step (iii) comprises cross-linking free ends of the hydrophilic polymer chains with a cross-linker. The cross-linker may comprise polyethylene glycol diacrylate, wherein the polyethylene glycol diacrylate comprises polyethylene glycol with a molecular weight ranging from about 700 to about 20,000, more particularly polyethylene glycol with a molecular weight of about 6000.

In an embodiment, the cross-linking is conducted in the presence of ammonium persulfate under ultraviolet light. In a further embodiment the polyethylene glycol diacrylate is diacryl-$PEG_{700}$ at a concentration between about 15 mM and 25 mM or diacryl-$PEG_{6000}$ at a concentration between about 0.5 mM and 5 mM.

As another aspect of the invention, there is provided a synthetic platelet substitute that interacts with platelets and the (sub)endothelium, comprising:

a. a carrier molecule comprising lipidic particles with a cross-linked surface mesh, the lipidic particles comprising: an inner lipidic particle of pharmaceutically acceptable particle-forming lipids; hydrophilic polymer chains linked to the surface of the lipidic particle, the hydrophilic polymer chains comprising a crosslinkable end group at free ends thereof; and cross-linker groups linking the end groups of the hydrophilic polymer chains to form the cross-linked surface mesh; and

b. at least one receptor molecule attached to the surface of the carrier molecule, wherein the receptor molecule is a peptide selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID a. NO:15, SEQ ID NO:16, SEQ ID NO:17, SEQ ID NO:18, SEQ ID NO:19, SEQ ID NO:20, SEQ ID NO:21, SEQ ID NO:22, analogs thereof having 90% sequence identity, and modified peptides thereof having an insertion of a Cys residue and/or a spectrophotometrically traceable amino acid and/or a poly-Gly tag consisting of 1 to 5 Gly residues.

In an embodiment, a plurality of receptor molecules are attached to the surface of the carrier molecule.

As another aspect of the invention, there is provided an antithrombotic composition that interacts with platelets and the (sub)endothelium, comprising: a peptide selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO: 10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, analogs thereof having 90% sequence identity, modified peptides thereof having an insertion of a Cys residue and/or a spectrophotometrically traceable amino acid and/or a poly-Gly tag consisting of 1 to 5 Gly residues, and combinations thereof, as defined in the appended claims.

In the antithrombotic composition, the peptide may be covalently attached to a carrier molecule at an amine group of the receptor molecule and at free acrylate ends of a hydrogel-coated carrier molecule. In such an embodiment, the carrier molecule may comprise lipidic particles with a cross-linked surface mesh, the lipidic particles comprising: an inner lipidic particle of pharmaceutically acceptable particle-forming lipids; hydrophilic polymer chains linked to the surface of the lipidic particle, the hydrophilic polymer chains comprising a crosslinkable end group at free ends thereof; and cross-linker groups linking the end groups of the hydrophilic polymer chains to form the cross-linked surface mesh.

As a further aspect of the invention, there is provided a peptide selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, analogs thereof having 90% sequence identity, modified peptides thereof having an insertion of a Cys residue and/or a spectrophotometrically traceable amino acid and/or a poly-Gly tag consisting of 1 to 5 Gly residues, and combinations thereof.

## BRIEF DESCRIPTION OF THE DRAWINGS

These and other features, aspects and advantages of the present invention will become better understood with regard to the following description and accompanying drawings wherein:

Figure 1 is a plot illustrating that the percentage of lipid PEGylated increases with the amount of DPPE available

for PEGylation as well as with the amount of PEG added. Three different levels of DPPE at 20 (squares) 30 (circles) and 40 (triangles) mol-% were incorporated into liposomes. Each of these liposomes was subjected to 1, 2 or 3 cycles of PEGylation. The error bars indicate the standard deviations obtained from 3 experiments;

Figure 2 shows the results of thin layer chromatography analysis of untreated and cross-linked liposomes. O: diacryl-$PEG_{6000}$ and ammonium persulfate, not exposed to UV; A1: unmodified liposomes alone; A2: unmodified liposomes treated with ammonium persulfate and 0.5 mM diacryl-$PEG_{6000}$; A3: liposomes treated with ammonium persulfate and 1mM diacryl-$PEG_{6000}$; B4: PEGylated liposomes treated with ammonium persulfate; B5: PEGylated liposomes treated with ammonium persulfate and 0.5 mM diacryl-$PEG_{6000}$; B6: PEGylated liposomes treated with ammonium persulfate and 1 mM diacryl-$PEG_{6000}$. The TLC was run (bottom to top) on MKC18 reverse phase plates, using a solvent mixture containing chloroform/methanol/water, 40/27/2, by volume;

Figure 3 shows the results of thin layer chromatography analysis of liposomes having received 1, 2 or 3 PEGylation cycles and then cross-linked. All the liposomes contained 30 mol-% DPPE and received 1 (A&B), 2 (C&D) or 3 (E&F) PEGylation cycles. A, C & E were treated with ammonium persulfate only, while B, D and F also received diacryl-$PEG_{6000}$. Increasing the number of PEGylation cycles resulted in a corresponding increase of the amount of cross-linked material at the origins of B, D and F, while the DPPE-PEG spots (arrows) decreased compared to its corresponding PEGylation cycle that was not cross-linked. The TLC was run (bottom to top) on MKC18 reverse phase plates, using a solvent mixture containing chloroform/methanol/water, 40/27/2;

Figure 4 is a plot of liposome mean diameter for untreated, PEGylated and cross-linked liposomes. The Gaussian distribution of the liposomes' mean diameter (nm, y axis) was determined for the untreated, PEGylated and cross-linked liposomes. PEGylation caused an apparent increase of the liposomes' size which was not increased further by cross-linking. The indicated standard deviation for the population is derived by the Nicomp Particle Sizer;

Figure 5 is a plot of the mean red fluorescence intensity of CF-liposomes incubated with the lipophilic fluorophore R18. Untreated, PEGylated, or cross-linked liposomes allowed progressively less incorporation of R18 as measured by the liposomes' fluorescence intensity in the red wavelengths. The bars indicate standard deviation, (n=3);

Figure 6 is a plot illustrating the results of lipid extraction from liposomes by Triton™ X-100. The relative amount of lipid found in the liposomes' supernatant is related to the level of protection of the liposome surface afforded by PEGylation and subsequent cross-linking. Untreated liposomes (squares), PEGylated liposomes (circles) and cross-linked liposomes (triangles) were equilibrated with increasing amounts of Triton™ X-100. The 100% lipid level was defined by the lipid concentration of the starting liposome suspension;

Figure 7 is a plot showing the fluorescence emission of EPC-FL-containing liposomes after treatment with Triton™ X-100. Untreated liposomes (squares), PEGylated liposomes (circles) and cross-linked liposomes (triangles) were treated by stepwise addition of Triton™ X-100 from 0 to 1.5% final concentration. The liposomes' supernatant was measured for released headgroup labelled lipid by fluorescence emission at 518 nm. 100% emission was obtained from the liposomes treated with a final concentration of 1.5% Triton™ X-100. The EPC-FL emission level of the liposome suspension before Triton™ X-100 addition was subtracted from all the samples;

Figure 8 is a plot depicting the effect of cross-linking on liposome cryogenic responses. Untreated (squares), PEGylated (circles) and cross-linked liposomes (triangles) were exposed to controlled rate freezing to the indicated temperatures, followed by rapid thawing. The level of CF fluorescence remaining with the liposome particles was measured by flow cytometry;

Figure 9 illustrates TEM pictures of unmodified liposomes (1 - 4), PEGylated liposomes (5 - 8) and hydrogel-liposomes (9 - 12). The reference bar is 1000 nm;

Figure 10 illustrates AFM images of dried, unstained liposomes. Unmodified liposomes (1 & 4), PEGylated liposomes (2 & 5) and hydrogel liposomes (3 &6) are shown. The bars represent 200 nm;

Figure 11 is a plot illustrating the interaction of CF-liposomes with platelets. Increasing concentrations of CF-containing liposomes untreated (squares), PEGylated (circles) or cross-linked (triangles) were allowed to interact with platelets for 2 hours at RT. The platelets were identified with a red fluorescing anti-CD42-PE and the population was assessed by flow cytometry for the proportion of green platelets. The error bars indicate standard deviation;

Figure 12 is a plot showing the interaction of CF-liposomes with erythrocytes. Increasing concentrations of CF-containing liposomes untreated (squares), PEGylated (circles) or cross-linked (triangles) were allowed to interact with red cells for 2 hours at RT. The erythrocytes were identified by their forward and side scatter characteristics in flow cytometry and assessed for the proportion of cells containing the CF marker's green fluorescence. The error bars indicate standard deviation;

Figure 13 illustrates a scheme of an exemplary route for hydrogel formation on a liposome surface. In the example, hydrogel formation starts with the amines on the lipid phosphatidylethanolamine head group. Acryl-$PEG_{3400}$-NHS is coupled to these followed by the addition of $PEG_{6000}$-diacryl to cross-link them, forming the hydrogel;

Figure 14a is a schematic illustration of a ligand-receptor interaction between a natural ligand and a natural receptor;

Figure 14b is a schematic illustration of a ligand mimic binding to a natural receptor, thus acting as an inhibitor

of the ligand-receptor interaction;

Figure 14c is a schematic illustration of a peptide-based material that mimics the function of a receptor such as, for example, an integrin receptor on the surface of a platelet and further showing a natural ligand binding to the receptor mimic;

Figure 15a is a schematic illustration of a peptide-based material that, by binding to the ligand like a receptor, can inhibit receptor-ligand interactions;

Figure 15b is a schematic illustration of a peptide-based material that, when attached to a large carrier at low coupling ratios, binds to the ligand to thus mimic a receptor, thereby providing a specific, quasi-monovalent inhibitory function such as, for example, functioning as an antithrombotic in the case of platelet-endothelium and platelet-platelet interactions;

Figure 15c is a schematic illustration of a peptide-based material that, when coupled to a large carrier at high coupling ratios, provides specific multivalent attachment possibilities, thus mimicking a receptor that is capable of binding multiple ligands;

Figure 16a is a schematic illustration of a peptide-based material comprising D-amino acids that can bind into an integrin receptor to thereby inhibit its ligand-binding function;

Figure 16b is a schematic illustration of a peptide-based material that, when attached to a large carrier at a low coupling ratio, binds to the receptor, mimicking a ligand, and thus providing a specific, quasi-monovalent inhibitory function such as, for example, functioning as an antithrombotic in the case of platelet-endothelium or platelet-platelet interactions;

Figure 17 shows a 3D computer model of a parent protein used for finding positions of particular sequences to enable the position to be related to potential vWf-GPIb interaction sites;

Figure 18 shows four cellulose membranes to which peptides were attached and which were then probed with purified vWf in order to identify sequences of D-amino acids which potentially inhibit the GPIb-vWf interaction;

Figure 19 shows the confirmatory structural results of 3D computer modeling of the interaction between a D-peptide and vWf;

Figure 20 shows schematically how surface plasmon resonance in a Biacore machine can be used to validate that the peptides can act as receptors/binding partners;

Figure 21 shows a Langmuir binding analysis used to determine the KD of the binding interaction between the peptide and fibrinogen;

Figure 22 illustrates a) a spacefill model of the vWf-GPIb complex. (vWf=blue; GPIb= red). b) Computed interaction interface of the vWf-PGIb complex;

Figure 23 illustrates the composition of the interaction interface of the GPIb-vWf complex: a) vWf: K549, W550, S562, Y565, E596, K599, Y600, P603, Q604, 1605, R632; b) GPIb: V9, A10, K152, F199, E225, D235, V236, K237, M239, T240;

Figure 24 shows hydrophobic patches on the subunits of the complex GPIb-vWf: (a) vWf : K549, W550, S562, H563, Y565, R571,I580, E596, K599, Y600, P603, Q604, 1605, P606, S607, R611, E613, R632; (b) GPIb: D21, T23, P27, D28, K31, L42,Y44, M52, P53, T55, E66, P77,V78, Q88, F109, R121, K137, T145, N157, E172, E181, S194, R218, D252, K253, K258, P260, K262;

Figure 25 depicts Molecular Dynamics simulations for the 4 target D-peptides. To obtain the most energetically stable conformations for the peptides in solution a series of minimizations and MD simulations were carried out. All these computations were performed using the force fields in AMBER-6 (Ponder J.A. Case DA. (2003) Force fields for protein simulations. Adv. Prot. Chem. 66:27-85) and CHARM (Richichi A. Percheron 1. (2002) CHARM: A catalog of high angular resolution measurements. A&A 386:492-503);

Figure 26 illustrates backbone models for the peptides under study. Starting conformation (left), conformations after MD simulation (red) and point minimization (blue) for (a) D-pep1, (b) D-pep2, (c) D-pep3 and (d) D-pep4;

Figure 27 shows hydrogen bond network (vWf intramolecular and vWf-GPIb intermolecular) and hydrophobic interactions at the interface between vWf-GPIb. The vWf-GPIb intermolecular bonds are marked with a circle around the donor amino acid number;

Figure 28 depicts the MD simulation process for the vWf-peptide complexes obtained by the docking experiment:

Figure 29 shows MIAX derived complex of vWf - D-pep1: a) the relaxation process using molecular dynamics of the best decoy output by MIAX for this interaction. Initial and final configuration for the complex and the position of D-pep1 on the surface of vWf (teal blue) before and after (red) the molecular dynamics simulation; b) Space-fill model for the complex vWf - D-pep1;

Figure 30 illustrates MIAX derived complex of vWf - D-pep2: a) the relaxation process using molecular dynamics of the best decoy output by MIAX for this interaction. Initial and final configuration for the complex and the position of D-pep2 on the surface of vWf (teal blue) before and after (red) the molecular dynamics simulation; b) Space-fill model for the complex vWf - D-pep2;

Figure 31 shows MIAX derived complex of vWf - D-pep3: a) the relaxation process using molecular dynamics

of the best decoy output by MIAX for this interaction. Initial and final configuration for the complex and the position of D-pep3 on the surface of vWf (teal blue) before and after (red) the molecular dynamics simulation; b) Space-fill model for the complex vWf - D-pep3;

Figure 32 illustrates MIAX derived complex of vWf - D-pep4: a) the relaxation process using molecular dynamics of the best decoy output by MIAX for this interaction. Initial and final configuration for the complex and the position of D-pep4 on the surface of vWf (teal blue) before and after (red) the molecular dynamics simulation; b) Space-fill model for the complex vWf - D-pep4;

Figure 33 illustrates the results of flow cytometric detection of platelet interaction with crosslinked hydrogel FITC labeled liposomes, with and without RGD-peptide. Peptide-substituted liposomes show greater attachment to platelets than hydrogel liposomes without the peptide;

Figure 34 shows a standard curve for the emission of the D-Pep3 peptide's Trp residue in a mixture with 0.8 mM lipid (at the same concentration as the test samples); and the stability of the washed peptide-hydrogel-liposomes (P-HL);

Figure 35 illustrates the stability of the covalent attachment of the D-Pep3 peptide to hydrogel liposomes after storage for 24 hours and one month;

Figure 36 depicts D-Pep3 peptide-hydrogel-liposomes capture of vWf from plasma cryoglobulin fraction;

Figure 37 illustrates the interaction of D-Pep3 peptide-hydrogel-liposomes with platelets in plasma; a) platelets/CD42-PE/anti vWf; b) liposomes-control/CD42-PE/anti vWf; c) liposomes-peptide/CD42-PE/anti vWf; d) liposomes-control/platelets/CD42-PE/anti vWf; and e) liposomes-peptide/platelets/CD42-PE/anti vWf;

Figure 38 illustrates a schematic representation of an example of a peptide-hydrogel liposome with high P-HL substitution; and

Figure 39 illustrates a schematic representation of an example of a peptide-hydrogel liposome with low P-HL substitution.

## DETAILED DESCRIPTION OF THE PREFERRED EMBODIMENT

As platelets are routinely in short supply, it would be highly desirable to develop an artificial platelet (also referred to herein as platelet substitute). The artificial platelet would need to be able to interact specifically with platelets and/or the (sub)endothelium, and provide the adhesive functions required for the formation of a platelet plug.

The invention described in the foregoing provides a synthetic platelet-like structure, or artificial platelet, capable of binding to either real (natural) platelets or other artificial (synthetic platelets), comprising a peptide ligand coupled to the surface of a surface cross-linked lipidic particle, such as a liposome.

The invention further provides a new class of antithrombotic molecule, comprising a peptide ligand coupled to a surface cross-linked lipidic particle, such as a liposome, at low density (e.g. a quasi-monovalent interaction) enabling the peptides to function as platelet-inhibitors.

The synthetic platelet and antithrombotic molecule are obtained by combining: (i) a surface cross-linked lipidic particle as a carrier molecule with (ii) a peptide ligand as a receptor molecule. In an embodiment, the carrier molecule comprises a liposome with a biocompatible hydrogel coating which stabilizes the individual liposome, reduces uptake of the liposome by blood cells *in vitro,* and can be chemically modified to add receptor-like functions. This hydrogel-liposome (HL) is a preferred example of the carrier molecule, i.e. the "cell", of the platelet substitute. In this exemplary embodiment, the receptor molecule preferably comprises one or more synthetic peptides (P) to provide specific receptor functions to the carrier molecule, i.e., so as to mimic GPIb and GPIIbIIIa functions. Combined, the peptide-hydrogel liposomes (P-HL) bind adhesive proteins such as fibrinogen (Fib) or von Willebrand factor (vWf), as do platelet receptors, and therefore mimic platelet function.

Artificial platelets in accordance with the present invention can be used either in addition to a standard platelet concentrate prepared from donations to reduce the number of units in a transfusion and consequent donor exposure, or as a free-standing transfusion product to treat acute bleeding.

Artificial platelets in accordance with the present invention can recapitulate the adhesion interactions of a natural platelet. In a damaged vessel wall, for instance, platelets adhere to the subendothelium through an interaction with von Willebrand factor (vWf), which forms a bridge between subendothelial collagen and the platelet receptor glycoprotein GPIb/IX/V (GPIb). This reversible adhesion allows platelets to roll over the damaged area, slow down and become activated. This then leads to the conformational activation of the platelet GPIIbIIIa receptor, fibrinogen binding and finally to platelet aggregation. Each interaction - collagen-VWF, VWF-GPIb and GPIIbIIIa-fibrinogen - plays a role in primary haemostasis.

In order to create a material that is able to substitute for platelets hemostatically, the inventors have developed peptides that mimic those functions which enable platelets to interact in the circulation with each other, with other blood cells and with the (sub)endothelium. These peptides particularly act as a receptor analog for adhesion through von Willebrand factor (vWf). These peptides can be used alone, in combination with each other, or in combination with other

peptides or receptor molecules.

In a preferred embodiment, such peptides are synthesized using D-amino acids in order to resist protelolytic degradation. In alternate embodiments these peptides may be synthesized using L-amino acids.

These peptides may be modified either at their N-terminus or C-terminus by adding one or more amino acids, or other molecules, as a tag. Such a tag may be used, for instance, to facilitate attachment of the peptides to the surface of the carrier molecule, or to incorporate a marker or other detectable moiety.

In an embodiment, the receptor molecule is coupled to the carrier molecule via covalent linkage.

At high levels of surface derivatization with receptor molecules, the P-HL molecules can bind to platelets *via* vWf and participate in platelet thrombus formation. Alternatively, at a very low level of surface derivatization, the P-HL molecules can also bind to platelets *via* vWf, but can physically block platelet thrombus formation (Figures 38 and 39).

In an embodiment, a ratio of less than 100 peptides : 1 liposome will produce an antithrombotic effect. In a preferred embodiment a receptor molecule of the present invention will, when coupled to a carrier molecule of the present invention, produce an antithrombotic effect when prepared with the carrier in a 10 peptides : 1 liposome ratio.

In a further embodiment, greater than 100 peptides conjugated to a carrier molecule (e.g. >100 peptides : 1 liposome) will a desirable effect for a platelet substitute in accordance with the invention. In a preferred embodiment, a platelet substitute of the invention will comprise a 1000 peptides : 1 liposome ratio or greater.

The present invention accordingly provides a synthetic platelet substitute that interacts with a recipient's own platelets to enhance the formation of a platelet plug and arrest acute bleeding. Additionally, as a secondary function the present invention can be to block platelet-platelet and platelet-endothelium interactions by preventing ligand-mediated bridging, thereby acting as an anti-thrombotic agent.

**(I) CARRIER MOLECULE:**

Disclosed in the following is an exemplary embodiment of a carrier molecule system for use in accordance with the present invention, in which individual liposomes are modified to carry a surface hydrogel layer. The hydrogel is polymerized onto the liposome surface and significantly reduces the liposomes' propensity for fusion and non-specific interaction with blood cells. At the same time, the liposomes remain as individual units that are not entrapped in a hydrogel matrix, but are generally free to circulate. As both liposomes and hydrogels are eventually biodegradable, these liposomes are particularly suitable. Furthermore, as the fusibility/ blood cell interaction of these liposomes is greatly reduced, they are suitable for being specifically targeted by biologically relevant molecules that can be attached to the exterior hydrogel layer. Consequently, such hydrogel-carrying liposomes constitute a material that can be used for site-specific delivery and/or controlled release of a drug or other biologically relevant molecules.

<u>Liposome Preparation</u>

The phospholipids, obtained from Avanti Polar Lipids (Alabaster, AL), were the following: 1 ,2 dipalmitoyl-sn-glycero-3-phosphoethanolamine (DPPE), 1,2 dipalmitoyl-sn-glycero 3-phosphocholine (DPPC) and L-$\alpha$-phosphatidyl-N-(Fluorescein) from egg (EPC-FL), while cholesterol (CHOL) was purchased from Sigma-Aldrich (Oakville, ON, Canada). The liposomes used in this study had the following lipid molar ratios: DPPE/DPPC/CHOL 20/50/30; DPPE/DPPC/CHOL 30/40/30 and DPPE/DPPC/CHOL 40/30/30. The lipids were hydrated in buffer containing 280 mM sucrose and 20 mM $NaHCO_3$ (pH 7.4), with or without 100 $\mu$M 5-carboxyfluorescein (CF) purchased from Molecular Probes (Eugene, OR, USA). Some liposomes contained DPPE/DPPC/CHOL/EPC-fluorescein 30/39.7/30/0.3 (molar ratio) and these were hydrated with the same buffer but without the CF marker. The lipids were resuspended in the appropriate buffer by vortexing, then the suspensions were subjected to 5 freeze-thaw cycles using liquid nitrogen, warming to -50 °C and vigorous agitation (Reinish et al., 1988, Thromb. & Haemostas. 60:518-523). The suspensions were maintained at -50 °C and extruded 5 - 10 times through 2 layers of polycarbonate membranes with 400 nm diameter pores (Costar Nuclepore Toronto, ON, Canada), under nitrogen pressure (100-500 lb/in$^2$) using an extruder (Lipex Biomembranes, Vancouver, BC). The resulting liposomes were washed twice with carbonate/bicarbonate buffer, pH 8 (95 mM $NaHCO_3$, 5 mM $Na_2CO_3$ and 70 mM NaCl) and centrifuged at 49,000 x g in an Optima TLX Ultracentrifuge (Beckman-Coulter, Mississauga, ON, Canada) to prepare them for the coupling reaction at constant pH, between 7 and 9. The lipid concentration of the liposome suspension was calculated based on a phosphate assay (Fiske et al., 1935, J. Biol. Chem. 66:375-389).

In order to determine the lipid formulation that would maximize PEG derivatization and the relative amount of PEG that becomes coupled to the liposome, three different DPPE concentrations were incorporated into the starting lipid mix to yield 20, 30 or 40 mol-% DPPE. As mentioned above, each of these formulations was subjected to three PEGylation cycles. Data in Figure 1 indicate that the total amount of PEG bound to the liposomes increases with the amount of DPPE present in the liposomes' lipid composition. This is not unexpected, as the available surface amine groups increase and the N-hydroxysuccinimide-ester (NHS) coupling reaction is designed to bind to these primary amine groups. There was only a small increase of the amount of PEG coupled between 30 mol-% and 40 mol-% DPPE. 30%

DPPE was thus used as the base formulation for the rest of this study.

Surface Hydrogel Formation

*PEGylation*: 2 - 3 mL of CF-liposome or EPC-liposome suspensions (20 - 30 mM lipid) in carbonate/bicarbonate buffer were added to dry Acryl-$PEG_{3400}$-NHS [Shearwater/NEKTAR, Huntsville, AL] at molar ratios ranging from 1:1 lipid:PEG to 4:1 lipid:PEG (in some cases the Acryl-$PEG_{3400}$-NHS powder was dissolved first in carbonate/bicarbonate buffer and then mixed with the liposomes). After de-gassing with nitrogen for 1 min, followed by 4 hours of incubation and shaking, the liposome/PEG mixture was pelleted for 25 min at 49,000 x g. The free PEG was removed with the supernatant and the pellet was resuspended in fresh buffer (the same volume as removed). The newly PEGylated liposomes were then remixed with the dry Acryl-$PEG_{3400}$-NHS, using the same procedure, for two more cycles in order to couple more Acryl-$PEG_{3400}$-NHS to the liposomes' surface. After the third coupling step, the liposomes were washed twice in a bicarbonate buffer containing 150 mM NaCl, 20 mM $NaHCO_3$ (pH 7.4), and the lipid concentration of the final mixture was determined by the phosphate assay.

The same protocol was done in parallel for unlabeled liposomes (no CF inside, no EPC-FL) to be used as controls. In that case, before each PEGylation step, aliquots (2 x 100 μL) of liposomes were sampled from the bulk liposome batch and added in duplicate, to a homogenous dry mixture of Acryl-$PEG_{3400}$-NHS / Fluorescein-$PEG_{5000}$-NHS, 98/2 molar ratio [Shearwater/NEKTAR, Huntsville, AL].

The samples with Fluorescein-$PEG_{5000}$-NHS were used to quantify (by ratio) the amount of Acryl-$PEG_{3400}$ that was bound to the liposomes at each step. To reduce the potential for self-quenching by fluorescein (FL), only 2 mol-% fluorescent PEG was used in the mixture. For binding calculations it was assumed that all liposomes coupled under the same conditions were PEGylated at the same rate, resulting in a similar number of PEG molecules attached to the vesicle.

The concentration of FL in the coupled liposome-PEG-FL-2% was detected by fluorimetry on a microplate fluorometer (Spectra Max GeminiXS, Molecular Devices, Sunnyvale, CA) by measuring the emission at 518 nm, (excitation 492 nm) and using a standard curve.

*Cross-linking*: In order to crosslink the liposome-coupled PEG-Acryl, a free monomer that could bridge the acrylate end of the PEG-acrylate was needed. Three different lengths of Diacryl-PEG (700, 3400 and 6000 MW) obtained from SunBio (Anyang City, South Korea) were tested at a range of concentrations, and optimal results were obtained with 1mM $PEG_{6000}$-diacryl. The cross-linking reaction was done in bicarbonate buffer using 2 mM (lipid) PEG-liposomes, under UV (Yang et al., 1995, J. Am. Chem. Soc. 117:4843-4850) light at 254 nm (UV Strataliker Crosslinker 1800, Stratagena, LA Jolla, CA) and room temperature (RT), for 100 min using ammonium persulfate as the initiator. The cross-linking reaction was also conducted at room temperature and with natural light but it was found, as by others (Yang et al., 1995, *supra),* that the acrylate-end groups polymerize better under UV light. The cross-linked liposomes were washed twice in bicarbonate buffer and the lipid concentration was measured by the phosphate assay.

Liposome Characterization

*Demonstration of coupling*: The presence of Acryl-PEG on the liposome surface was confirmed by thin layer chromatography (TLC). TLC was done on MKC18 Silica, 2.5 x 7.5 Whatman plates (Fisher Scientific, Ottawa, ON, Canada) using a solvent mixture containing chloroform/methanol/water, 40/27/2 (by volume) to develop the spots which were visualized by iodine vapour staining.

TLC analysis confirmed the presence of cross-linked PEG on the surface of the liposomes, as the cross-linked material does not migrate with the solvent flow and remains at the origin (Bonte et al., 1987, Biochim. Biophys. Acta. 900:1-9). The TLC analysis further showed that uncoupled lipids move with a retention factor, (Rf) of about 0.64 - 0.72 while the coupled PEG-DPPE moved closer to the solvent front (Table 1a), and the native PEG-$diacryl_{6000}$ (not UV treated) remained at the solvent front (Figure 2)

Table 1a

| Rf Values | | | | | | | |
|---|---|---|---|---|---|---|---|
| Sample / Lane | 0 | A1 | A2 | A3 | B4 | B5 | B6 |
| PEG-$Diacryl_{6000}$ | 0.97 | | | | | | |
| Lipids | | 0.72 | 0.72 | 0.71 | 0.70 | 0.66 | 0.64 |
| PEG-DPPE | | | | | 0.89 | | |
| PEG-DPPE & Unreacted PEG-$Diacryl_{6000}$. | | | | | | 0.99 | 1.00 |

(continued)

| Rf Values | | | | | | | |
|---|---|---|---|---|---|---|---|
| Sample / Lane | 0 | A1 | A2 | A3 | B4 | B5 | B6 |
| DPPE-PEG crosslinked | | | | | | 0.03 | 0.04 |

Figure 2 also shows clear differences among the colour intensities of the spots relative to the amount of diacryl-$PEG_{6000}$ used to crosslink the liposomes: 0.5 mM (Figure 2, B5) and 1 mM (Figure 2, B6). TLC was also used to analyse liposomes that had received three PEGylation cycles using 3 different levels of PEG and were subsequently cross-linked using 1 mM diacryl-$PEG_{6000}$.

Figure 3 shows the increasing colour intensities of the spots that correspond to cross-linked PEG that remains at the origin. Conversely, analysing the colour intensity of the PEGylated phospholipid spot from these liposomes shows that the intensity of the DPPE-PEG in cross-linked liposomes is less than in the unmodified liposomes, for the same cycle, because some of the DPPE-PEG is retained at the origin with the cross-linked material.

*Liposome size*: Evidence of surface polymer derivatization comes from measurements of the liposomes' mean diameter using quasi-elastic light scattering (Nicomp Submicron Particle Sizer System, Model 370, Santa Barbara, CA, USA). These studies indicate that the liposomes' effective hydrodynamic size increased from -130 nm to -230 nm when PEG was coupled to the liposomes. This apparently large increase is more likely related to the initial variation of the liposomes' size as indicated by the wide SD, (also apparent on AFM, vide infra) than the incremental size increase created by the PEG addition. Cross-linking of the acrylate end groups did not cause any further size increases (Figure 4).

*Demonstration of cross-linking:*

*(i) Lipophilic fluorophore uptake*: CF-labelled liposomes (200 μL, 1 mM) were incubated for 5 min at RT with 3 μL of a 0.82 mM solution containing the lipophilic marker octadecyl rhodamine B chloride (R18) in ethanol (Molecular Probes, Eugene, OR, USA). After the incubation, the liposomes were diluted up to 2 mL in an aqueous buffer, and analysed by flow cytometry (Beckman Coulter Exel-MCL, Hialeah, FLA). The green liposome bitmap was analysed for red (R18) fluorescence.

By coupling PEG to the liposomes and cross-linking their surface PEG, a network or hydrogel was built around the lipid bilayer that was expected to increase the liposomes' resistance to lipophilic molecules. Figure 5 shows that if a liposome's surface is covered by a strongly hydrophilic layer formed by linear PEG molecules, a lipophilic fluorophore, such as R18, has reduced access to the phospholipid bilayer, resulting in lower red fluorescence. This access is further reduced when the PEG is cross-linked to form a hydrogel.

*(ii) Triton™ X-100 resistance*: Liposomes containing head-group labelled phospholipids EPC-FL (0.33 mM final lipid concentration) were mixed with a range of Triton™ X-100 (Sigma-Aldrich, Oakville, ON, Canada) concentrations (final concentration between 0 % and 1.5 % by volume), incubated for 2 h at room temperature, then centrifuged for 45 min at 21000 x g. The supernatant was analyzed by phosphate assay to quantify the amount of lipid released by the detergent. The amount of EPC-FL released from the liposomes was quantitated by fluorimetry.

Figures 6 and 7 show that PEGylating liposomes, then generating a hydrogel by crosslinking PEG acrylate ends, resulted in increased liposomal stabilility. When liposomes containing headgroup-labelled lipids were mixed with Triton™ X-100 detergent, increasingly more lipid was solubilized from the untreated liposomes, followed by PEGylated, and cross-linked liposomes (Figure 6). Assessment of the release of fluorescent lipids from the three liposome groups paralleled these (Figure 7).

*(iii) Cryogenic responses:* The CF-labelled liposome suspensions were subjected to a controlled-rate freezing and thawing protocol to -40 °C (McGann et al., 1976, Cryobiology 13:261-268). Briefly, 100 μL samples in glass tubes were maintained at 0 °C for 5 minutes in an ice bath, and then placed into a -5 °C alcohol bath (MC880A1, FTS Systems Inc.) for 5 minutes. Extracellular ice formation was induced by touching the outside of the samples with liquid-nitrogen-chilled forceps before the samples were cooled to -40 °C at -1 °C/min. Samples were removed at 0, -5, -10, -15, -20, -30, and -40 °C, and rapidly thawed in a circulating 37 °C water bath. The recovered liposomes were analyzed by flow cytometry using a uniform 20 sec. acquisition time and two-colour analysis of the liposome bitmap.

PEGylation and further modification by PEG cross-linking altered the liposomes' cryogenic responses (Figure 8). In this case, liposomes encapsulating CF were subjected to controlled-rate freezing to sub-zero temperatures and rapid thawing, followed by flow cytometric analysis. The total number of liposomes that remained fluorescent remained highest for the cross-linked liposomes (~75 %) with decreasing temperature, as compared to PEG-liposomes (~60 %), or unmodified liposomes (~50 %). The total number of cross-linked liposomes also remained more stable in terms of size: the slopes of the lines relating final freezing temperature to liposome size were 0.07 for unmodified liposomes, 0.015 for PEGylated liposomes, and 0.0005 for cross-linked liposomes, indicating that unmodified liposomes swelled

during thawing, while the PEGylated and cross-linked liposomes resisted swelling the more they were modified (data not shown).

*(iv) Liposome morphology*: Liposomes were visualized by atomic force microscopy (AFM) using a VEECO Digital Instruments (Santa Barbara CA, USA) BIOScope and silicon nitride probes in tapping mode under ambient conditions. Samples were prepared by depositing 10 μL droplets onto freshly cleaved mica, then rapidly dehydrating under vacuum (133 mbar, 30 min). The final lipid concentration was 0.5 mM. Phase images were collected at a scanning rate of 2.5 Hz. Electron microscopy (TEM) was done on a Philips/FEI Tecnai F30 H-7600 electron microscope using negatively stained samples with 2 % uranyl acetate 1 % trehalose (wt/vol) solution.

Both of these methods (AFM and TEM) confirmed that the liposomes remained discrete and that their size distributions were similar to that measured by the Nicomp Particle Sizer. Images 1 - 4 of Figure 9 show that unmodified liposomes have a tendency to collapse during dehydration and staining, which is a well recognized problem in the preparation of liposomes for TEM analysis (Olson et al., 1979, Biochim. Biophys. Acta. 557:9-23). Images 5-8 are PEGylated liposomes that have a tendency to trap the stain within the PEG layer, giving a darker outline and a "halo effect." Images 9-12 show the hydrogel-liposomes that trap the stain in the surface hydrogel resulting in a "soccer-ball" pattern. These results also indicate that surface-cross-linked liposomes remain stable and resist collapse during dehydration.

AFM is one of the newest techniques employed to image solid lipid nanoparticles (zur Muhlen, A. et al., 1996, Pharm. Res. 13:1411-1416), cells (Radmacher et al., 1992, Science, 257:1900-1905) and liposomes (Anabousi et al., 2005, European Journal of Pharmaceutics and Biopharmaceutics 60:295-303; Ruozi et al., 2005, European Journal of Pharmaceutical Sciences 25:81-89). In "tapping mode, the AFM surface topological images are obtained by gently tapping the surface with an oscillating probe tip. This tool provides visual information, at a nanoscale level, about the size, shape and the surface of the liposomes. However the "halo" and "soccer ball" patterns were not visible due to the samples being unstained. The AFM images also show that PEG crosslinking and the resultant surface hydrogel formation does not lead to liposome fusion, but leave the liposomes as distinct, individual entities (Figure 10: 3). At high magnification, unmodified liposomes appear smooth (Figure 10: 4) while PEGylated liposomes (Figure 10: 5) appear to have a halo. The hydrogel liposomes (Figure 10: 6) appear to be associated with an extra layer of material spreading from the dried liposome.

Liposome Interaction With Blood Cells

*Blood cells*: Blood samples were obtained from consenting donors as sanctioned by the Research Ethics Boards of both the University of British Columbia and Canadian Blood Services. Blood was drawn into EDTA anticoagulant and used without dilution. Alternately, the various cell types were purified by standard laboratory methods using differential centrifugation (Constantinescu et al., 2003, Artificial Cells, Blood Substitutes and Biotechnology 31:394-424). Platelet rich plasma (PRP) was obtained by centrifugation of 5 mL of citrate anticoagulated blood at 200 x g for 15 min (Beckman Coulter GS-6R centrifuge, Hialeah, FLA).

*Interactions*: A range of volumes (0 - 50 μL, containing 1 mM lipid) of internally-labelled CF liposomes (unmodified; PEGylated; and PEGylated-cross-linked) were incubated for 2 hours at room temperature with 5 μL PRP (~100 x 109/L platelets) in 55 μL. Five μL of a specific anti-platelet surface antibody, CD42b (anti-glycoprotein IBIX, coupled to phycoerythrin (PE), Beckman Coulter) was added in order to distinguish the platelets from some liposomes that have the same apparent size on the flow cytometer's bitmap. The liposome/plateletlantibody mix was incubated for a further hour at room temperature.

The interaction of red cells (RBC) from whole blood (6 μL) with CF-liposomes (0 - 100 μL. 1 mM lipid) in bicarbonate buffer (200 μL final volume) was also analysed. After a 2.5 h incubation at room temperature, the samples were diluted with 0.8 mL bicarbonate buffer and analyzed by flow cytometry.

Figure 11 shows that, as previously described, platelets take up unmodified liposomes (Constantinescu et al., 2003, *supra*; Mordon et al., 2001, Microvascular Research 63:315-325). This uptake is much greater than the uptake of cross-linked liposomes and it is dose-dependent.

Figure 12 shows that this is also true of liposome uptake by red blood cells: modified CF-liposomes are taken up to a much lesser extent than unmodified liposomes. Similar results were obtained with liposomes that contained head-group labelled phospholipids (EPC-FL) rather than the encapsulated CF as the fluorescent indicator [data not shown].

Discussion:

The foregoing experiments demonstrate that it is possible to modify the surface of a lipidic particle, in the present example by creating a hydrogel layer on the surface of a liposome, such that the lipidic particles remain as discrete units and yet acquire new characteristics provided by the surface layer.

In the aforementioned example, the first step to establishing a hydrogel on the liposome surface was to add a PEG layer (Figure 13). A single PEG addition step can be used, although it was observed that a number of low-concentration addition cycles loaded more PEG onto the liposomes and subsequently gave more cross-linked material than a single high concentration step (Figures 1 & 3). As the PEG to be cross-linked was tethered to the liposome via the amino group of a DPPE, leaving only one reactive end free, the effective surface distribution/concentration of PEG also contributed to the hydrogel's formation.

Due to steric/repulsion and solution effects (van Oss, 2003, J. Mol. Recognit. 16: 177-190; Lal et al., 2004, Eur. Phys. J. E15:217-223), the fraction of added PEG that became attached onto the liposome surface decreased with each PEGylation cycle, although only a small proportion of the total available DPPE became substituted (Figure 1). Increasing the mol-% of the liposomes' DPPE to more than 30 mol-% did not appreciably increase the amount of attached PEG due to mutual exclusion (van Oss, 2003, J. Mol. Recognit. 16: 177-190) by the highly mobile polymer chains (Amsden, 1998, Macromolecules 31:8382-8395; Garbuzenko et al., 2005, Chemistry and Physics of Lipids 135:117-129).

Choosing Diacryl-PEG lengths that resulted in surface gel rather than bulk gel formation was conducted by testing macro monomers of a range of molecular weights. In general, the shorter length Diacryl-PEG chains (e.g. 700 MW) were more difficult to work with in that higher concentrations (about 15 - 25 mM) were required for optimal cross-linking, but at slightly higher concentrations (>25 mM) often resulted in bulk gelation. The optimal concentration range was somewhat wider for Diacryl-PEG 3400 MW. The 6000 MW was easiest to handle, with an optimal concentration range extending as low as 0.5 mM (Figure 2 & Figure 13).

PEGylation increased the effective hydrodynamic diameter of the liposomes compared to those that remained unmodified. However, dynamic light scattering did not show a further size increase after cross-linking (Figure 4). This was also supported by both TEM and AFM analysis (Figures 9 & 10). It was noted that the unmodified sucrose-filled, and therefore more dense, liposomes slowly settled out from the solution, but the PEGylated and cross-linked liposomes remained suspended in the buffer, due to their more hydrophilic surface and the PEG's and the hydrogel's ability to bond to water molecules (Lal et al., 2004, Eur. Phys. J. E15:217-223) while repulsing each other (van Oss et al., 2003, *supra*). Such complex and flexible interactions with the water phase (van Oss 2003, *supra*; Lal et al., 2004, *supra)* may increase the apparent, rather than the calculated actual size of the liposomes (Garbuzenko et al., 2005, Chemistry and Physics of Lipids 135:117-129).

The lipophilic fluorophore R18 was used to investigate the establishment of a hydrophilic surface layer on the liposomes. To externally label cells or liposomes, R18 is dissolved in ethanol to carry it through the water phase and into the phospholipid bilayer (Ohki et al., 1998, Biochemistry 37:7496-7503). This caused rapid dye partitioning into exposed phospholipid bilayers (Melikyan et al., 1996, Biophys. J. 71:2680-2691): cells and untreated liposomes took up the dye almost immediately, while PEGylated liposomes took up the fluorophore more slowly. The cross-linked hydrogel was the slowest to take up R18 because the crosslinking restricted R18's diffusional access to the phospholipid bilayer (Figure 5). Diffusion of even relatively small molecules across hydrogels can be restricted by mesh size (Behravesh et al., 2003, Biomaterials, 24:4365-4374). In an end-linked structure, such as the one formed on the liposome surface, the molecular weight between cross-links is the total Diacryl-PEG molecular weight. The relatively short (6000 MW) crosslinking PEG chain lengths, on the ends of the 3400 MW lipid-tethered PEG, define a relatively small mesh size of the order of 1.5 - 3.0 nm (15-30 A; Stringer et al., 1996, J. Controlled Release 42:195-202; Cruise et al., 1998, Biomaterials 19:1287-1294). The hydrated R18 (732 MW, -0.55 nm = -5.5A) is of the order of magnitude (Baba et al., 2004, J. Chromatography A, 1040:45-51) that can be restricted and its diffusion slowed by such a mesh size (Cruise et al., 1998, *supra).* As well, the relative hydrophobicity of the molecule would alter the ease with which it permeates the channels of moving water among areas of PEG-bound water (Baba et al., 2004, J. Chromatography A, 1040:45-51) of the PEGylated liposome or the fully hydrated hydrogel.

Initially, a similar logic applies to the detergent-based solubilization of the liposomes with Triton™ X-100 (Figures 6, 7). Access of the amphipathic detergent molecules (625 MW) to the phospholipid bilayer would be only slightly faster than the movement of R18, assuming that hydrodynamic diameter is the predominant factor proscribing diffusion. However, in this case, the liposomes were exposed to a range of detergent concentrations. As the detergent solubilized the membrane's constituent phospholipid molecules and removed them from the bilayer, there was consequent formation of incrementally increasing detergent-lipid mixed micelles in the supernatant (Goni et al., 1986, Eur. J. Biochem. 160: 659-665). At the critical micellar concentration (CMC) of Triton™ X-100 (0.015 %; 0.2 x 10-3 at 25 °C), the amount of solubilized lipid in the liposomes' supernatant decreased because the detergent-phospholipid mixed micelles were removed by the centrifugation step that removed the liposomes. Overall, more lipid was solubilized from untreated liposomes than from polymer-coated ones. The effects of the detergent were seen at higher detergent concentrations for liposomes with the cross-linked hydrogel, which may be a function of more phospholipid having to be solubilized to create sufficiently large gaps in the lipid-anchored hydrogel and to allow the mixed micelles to escape to the supernatant. The solubilization of fluorescent EPC-FL also shows a similar biphasic curve for untreated liposomes where the saddle point corresponds to the detergent's CMC. PEG and hydrogel-carrying liposomes show incremental increases of lipid-associated fluorescence in the supernatant that reflects not only solubilization of the lipid into a mixed micelle, but also

its diffusion out of the hydrated PEG or remaining hydrogel.

The freezing responses of untreated and surface-modified liposomes are perhaps the most interesting. Liposomes are osmotically active vesicles, so like cells, they shrink and swell in response to osmolality changes in their environment (Meryman, 1971, Cryobiology 8:489-500). As the degree of cellular shrinkage has been associated with the extent of freezing damage (Meryman, 1971, Cryobiology 8:489-500), PEGylation, and especially cross-linking, may stabilize liposomes to freeze-thaw by mechanically limiting the degree of shrinkage/expansion that the vesicle can undergo in response to osmotic fluctuations. The hydrogel may also limit the rate of the movement of water across the membrane, as a consequence of the cross-link mesh size and polymer-bound water. This in turn, limits the change of liposome volume that will occur due to the increasing extra-liposomal solute concentration during freezing that would cause the liposomes to shrink. Subsequent to membrane damage by freeze-thaw, membrane breaks would allow the escape of the entrapped CF. However, the PEG, and more so the hydrogel, would either support membrane resealing, or limit the diffusibility of the CF from liposomal aqueous core.

Evidence for the retention of materials in the hydrogel also comes from the TEM images (Figure 9). The uranyl acetate stain used for visualizing liposomes in an electron beam is trapped in the hydrogel layer and produces localized electron dense material that shows up as black spots on the liposome surface. Both these and the AFM images (Figure 10) confirm that the liposomes remain as distinct, regular-sized particles that retain their spherical, cell-like shape and that the cross-linking process does not cause undue fusion or over-all hydrogel formation.

In addition to inhibiting the entry of disruptive molecules and the movement of water, the hydrogel can also prevent lipidic particle fusion with cell membranes. Fusion is thought to take place when membrane proteins have been excluded from the contact region and the phospholipid bilayers form close contacts through local dehydration which is then followed by transient destabilization of the apposed membranes (Bangham et al., 1967, Chemistry and Physics of Lipids 1:225-246; Arnold et al., 1983, Biochim. Biophys. Acta 728:121-128). The PEG molecules' movement is limited due to their mutual repulsion (van Oss, 2003, *supra*; Lal et al., 2004, *supra*) and the hydrogel restricts phospholipid re-ordering by limiting the movement of the hydrogel-tethered phospholipids in the plane of the membrane. The membrane dehydrating tendency of the PEG (Arnold et al., 1983, *supra*) is limited by its attachment to the liposome and to other PEG molecules by cross-linking. Consequently, the coated lipidic particles have a lower tendency to fuse with cell membranes. In the aforementioned example, it is shown that surface modified liposomes fuse with red cells and platelets only to a limited extent (Figures 11,12). At the same lipid:cell ratio, cross-linked liposomes are taken up 3- to 4-fold less than unmodified liposomes, depending on the cell type.

### (II) RECEPTOR MOLECULE:

In general, and as will be elaborated below, the receptor molecules used in accordance with the present invention comprise peptides which mimic the shape and function of natural platelet receptors and ligands, thus providing synthetic binding sites. These receptor molecules are attached to the carrier molecules, such as the hydrogel liposomes (HL) described above, to act as synthetic platelets by providing binding sites for binding to other (natural or synthetic) platelets or to the (sub)endothelium. When bound to the carrier molecule at very low stoichiometric ratios (see above), the receptor molecules can alternately act as anti-thrombotics by inhibiting platelet-platelet and/or platelet-endothelium interactions.

Referring to Figure 14a-c, and as illustrated in Figure 14c, a peptide-based material can be used as a 'mimotope' to mimic the form/shape (and thus the function) of a receptor. In one embodiment, the mimotope receptor (receptor mimic) can bind to a ligand to inhibit binding of the ligand to a natural receptor. In another embodiment, the mimotope receptor can be a peptide-based material that mimics an adhesion receptor or integrin on the surface of a platelet-like carrier such as a liposome, preferably a cross-linked liposome.

In the context of platelets, an *integrin, integrin receptor* or (simply) *receptor* shall be used synonymously in the present specification to mean a molecule, such as a peptide or protein, on the surface of the platelet or carrier that selectively binds a specific molecule known as a *ligand.*

As illustrated in Figure 15a, a peptide-based material can be used as a receptor mimetic to bind to the ligand like a receptor, thus inhibiting receptor-ligand interactions. As shown in Figure 15a, the mimotope receptor can be a "free" (unattached) peptide that has a shape/topology like that of a natural receptor so that it binds "preemptively" to ligands, thus preventing the ligands from binding to their natural receptors. These unattached, "free" receptor mimics thus act as inhibitors or blockers of the natural receptor-ligand interactions. In one embodiment, these mimotope receptors can be made of peptides that mimic the adhesion receptors or integrins of platelets. In the context of platelets, therefore, these unattached, "free" peptides would have an antithrombotic effect by binding to ligands and/or other factors, thus inhibiting normal platelet-platelet or platelet-endothelium adhesion.

As noted above, the mimotope receptor shown in Figure 15a can be a peptide that mimics an integrin of a platelet. In a preferred embodiment, the peptide mimic is shaped to bind to a ligand such as one of the attachment sites of a von Willebrand factor (vWf) protein. In a vWF monomer (which is a -2050 amino acid protein), a number of specific

domains are known to have specific functions. The A1 domain, for example, binds to the platelet GPIB receptor. The C1 domain binds to platelet integrin $\alpha_{IIb}\beta_3$ when activated. Therefore, in this example, the mimotope receptor will preferably be a peptide that mimics the shape and structure of the binding site of platelet GPIb-receptor by binding preemptively to the A1 domain of the vWf monomer. Similarly, and again by way of example only, the mimotope receptor could be a peptide that mimics the shape and structure of the binding site of platelet integrin $\alpha_{IIb\beta3}$.

The mimotope receptor shown in Figure 15a can also be used to inhibit platelet-(sub)endothelium interaction by binding to the corresponding natural ligand that normally promotes adhesion of platelets to the vascular endothelial cells such as, for example, von Willebrand factor. As is known in the art, circulating platelets do not adhere to normal (sub)endothelium because platelet adhesion requires endothelial cell secretion of von Willebrand factor, which is found in the vessel wall and in plasma. The vWf protein binds during platelet adhesion to a glycoprotein receptor of the platelet surface membrane (glycoprotein Ib). Thus, in this example, platelet-(sub)endothelium interaction can be inhibited by a mimotope receptor (peptide mimic) that binds preemptively to one of the active sites of the vWf protein to thus obstruct subsequent binding to that particular site on the vWf protein.

As illustrated in Figure 15b, a peptide-based material can also be attached to a carrier molecule at low coupling ratios for providing monovalent or quasi-monovalent inhibitory functions. This mimotope is thus a monovalent receptor mimic which, whether attached to a carrier or not, can bind to a corresponding ligand, thus inhibiting receptor-ligand interactions. By mimicking a receptor, this mimotope provides a specific, quasi-monovalent inhibitory function that can be used, for example, as an inhibitor of platelet-platelet and platelet-(sub)endothelium interactions. This mimotope can thus be used as an antithrombotic.

As illustrated in Figure 15c, a peptide-based material can be coupled to a carrier molecule at high coupling ratios to provide specific, multivalent attachment possibilities, i.e. the synthetic receptor can simultaneously bind a plurality of ligands. In this case, the mimotope mimics a multivalent receptor and thus can form the basis of a synthetic platelet substitute.

As is known in the art, *platelets* (or "thrombocytes") are anuclear and discoid spherules ("flattened ellipsoids") that measure approximately 1.3-3.0 microns in diameter. Platelets adhere to each other via adhesion receptors or integrins that bind their specific ligands, which in turn facilitate adhesion to the endothelial cells of blood vessel walls. Platelets form haemostatic plugs with fibrin, a clotting protein derived from fibrinogen.

A synthetic platelet thus includes a carrier molecule, such as the hydrogel-liposome described above, that is manufactured to emulate some of the key physical characteristics of platelets (approximate size and shape, and resistance to liposome-cell fusion). The synthetic platelet also includes at least one receptor mimic attached to the carrier (i.e. the outer surface of the liposome). The receptor mimic includes a peptide that mimics a shape and size of a binding site of a natural receptor on a natural platelet. Preferably, the cross-linked liposome (or other equivalent carrier molecule) includes a plurality of peptides attached to its outer surface, each one functioning as a receptor mimic to thus provide a "multivalent" synthetic platelet with multiple binding sites. In other words, each of the peptides is a mimotope that mimics a natural adhesion receptor or integrin found on a natural platelet.

As shown in Figure 16a, a peptide-based material comprising D-amino acids can be used to bind an integrin receptor to thus inhibit its ligand-binding function. Although some L-peptides (levorotatory peptides) are known in the art, D-peptides (dextrorotary peptides) are preferred because they resist proteolytic degradation.

As shown in Figure 16b, a peptide-based material can be attached to a carrier molecule (e.g. a liposome, vesicle or other body) at a low coupling ratio for binding to the receptor, thus mimicking a ligand and thus providing a specific, quasi-monovalent inhibition function. For example, the monovalent ligand mimic interferes with ligand-receptor interaction and thus can serve as an antithrombotic in the case of platelet-platelet interactions or platelet-endothelium interactions. The peptide attached to the carrier can be levorotary (L) or dextrorotary (D). Attachment to the carrier molecule would resist excretion through the kidneys. In other words, the carrier (preferably a PEG, polyglycidol, or cross-linked liposome) provides circulatory resistance and physical blocking or obstruction of the binding site(s).

A peptide-based material in accordance with one of the foregoing embodiments would have great utility in the context of an artificial platelet substitute or as an antithrombotic drug.

A peptide-based antithrombotic drug will resist proteolytic degradation (proteolysis) when made of D-amino acids, which form peptide bonds that natural enzymes cannot break down. Furthermore, a peptide drug where the peptide is attached to a large carrier structure would resist excretion through the kidneys.

Mimotope Peptide Design

The von Willebrand factor (vWf) amino acid sequence and available literature were used to select the potential vWf binding site for the integrin, glycoprotein Ib (GPIb). As is known in the art, *von Willebrand factor* (vWf) is a large multimeric blood glycoprotein present in blood plasma that plays a significant role in platelet thrombus formation. The vWf is produced in the Weibel-Palade bodies of the endothelium, in megakaryocytes (stored in $\alpha$-granules of platelets), and in subendothethial connective tissue. The primary function of von Willebrand factor is binding to other proteins, such

as Factor VIII, binding to collagen, binding to platelet GPIb, and binding to other platelet receptors when activated, e.g. by thrombin.

The vWf amino acid sequence was used to generate 10-mer L-amino acid overlapping peptides, shifted by two (2), according to the following pattern:

```
ACDFGHIKWER;
   DFGHIKWERAL;
     GHIKWERALND; etc.
```

These peptides were synthesized and remained attached on the cellulose membrane. The membranes were probed by purified GPIb which was detected by anti-GPIb coupled to horseradish peroxidase (HRP). A number of positive spots were found whose sequences were derived from their positions on the membrane.

The sequences were analyzed *in silico* by (a) finding their positions in a 3D model of the parent protein (see Figure 17) and then (b) relating that position to the potential vWf-GPIb interactive site. This suggested that the peptides colored black and brown (identified in Figure 17 as "+ve peptides") were in the interactive region and thus, as free peptides, could serve as competitive inhibitors of the interaction.

A similar study was conducted using overlapping peptides of the GPIb molecule, but the positive peptides identified by colours (in Figure 17) contributed relatively little to the interactive site.

This series of experiments identified a number of native sequences of L-amino acids with potential inhibitory activity for the GPIb-vWf interaction.

Random D-amino acid peptides (15 mer) were synthesized and probed with vWf to detect random sequences capable of binding vWf. Figure 18 shows the membranes from which four positive sequences were derived.

To determine whether these peptides were complementary to the binding surface defined by the GPIb molecule, they were analyzed *in silico* by (a) comparing them to known sequences in PDB.A. Fasta search provided homologues/ decoys of known structure, (b) then the structures were docked onto the vWf molecule to check for 3D fit. Figure 19 shows the confirmatory structural results of this analysis for one of the three functional peptides identified (D-PEP3; SEQ ID NO: 3).

Thus, the structural analysis by computer confirms the physical findings that random D-amino acid peptides that are structurally complementary (in this case to vWf) are also those that can be demonstrated experimentally to bind *in vitro*.

To confirm that synthesized peptides can act as receptors/binding partners, not just as inhibitors, real-time binding was demonstrated by surface plasmon resonance in a Biacore machine. In this case, peptides known to interfere with fibrinogen-GPIIbIIIa interaction were synthesized, and coupled to the end of a long (3400MW) PEG molecule whose other end was attached to biotin, as illustrated schematically in Figure 20. (As is known in the art, *fibrinogen* is a soluble protein in the blood plasma essential for clotting of blood which the enzyme thrombin converts into the insoluble protein fibrin.) As shown schematically in Figure 20, the biotin molecule was used to tether down the peptide-PEG onto a streptavidin-modified Biacore chip. This allowed the GPIIbIIa mimicking peptide to be hanging off the free end of the PEG.

By allowing free fibrinogen to flow past the peptide, the binding kinetics (i.e., the "on/off rate") between fibrinogen and the peptides were measured. Then, the fibrinogen was released from the peptide. Using several fibrinogen concentrations, it was possible to measure the KD of the binding interaction between the peptide and the fibrinogen. The Langmuir binding analysis is shown in Figure 21.

This showed that a peptide can generate binding kinetics/affinities similar to that of the parent protein and thus confirms the concept that the peptides can act as the desired synthetic receptor molecules.

A synthetic receptor bestows a number of significant advantages. First, since the receptor is synthetic, it does not have to be extracted, or made out of living material, purified, cleaned, etc. Second, it can be made (designed) to carry out any *receptor* function as long as the three dimensional shape of the receptor is mimicked. Third, the future production of synthetic cells (or cell-replacing materials) would require synthetic receptor functionality and thus a synthetic receptor would be a very significant first step in creating synthetic cells or synthetic platelets.

Potential uses of the synthetic receptor are numerous. As mentioned above, the synthetic receptor can be used on a platelet substitute (i.e. a synthetic or artificial platelet). Furthermore, the synthetic receptor can be used to offer a specific binding capacity for isolating and analyzing ligand molecules without the need for monoclonal antibodies. These synthetic receptors could thus replace monoclonal antibodies in assay systems currently relying on monoclonal antibody technology. This would thus potentially eliminate the need for culturing and maintaining specific antibody-producing clones. Moreover, the synthetic receptors can be tailored to obtain defined kinetics and binding affinities. The synthetic receptors can also be synthesized using D-amino acids, thereby preventing proteolysis.

Analysis of the GPIb-vWf Binding Surface

Four peptides were selected based on their ability to bind vWf: The following study describes the analysis of these peptides to define structural characteristics that would allow them and similar peptides to target the interface between vWf and GPIb. The intention was to generate peptides with high affinity for vWf. Computer modeling of affinity was used to reduce the number of candidate peptide structures and to define the initial peptides.

Commercial software for computational methodologies that allow these types of evaluations was not available; therefore, the study was carried out using a suite of programs developed in the inventors' laboratories. Central to this collection of computational procedures is the MIAX paradigm (Macromolecular Interaction Assessment computer system) which enables the prediction of the most probable configuration of protein-protein, protein-peptide, and other bio-macromolecular complexes.

Complexes output by MIAX are tested for stability using molecular dynamics (MD) methods. The results show that three out of the selected four peptides bind to regions in the interface of interaction between vWf and GPIb. Stability of the vWf-binding peptides is high since MD simulations performed for several pico-seconds hardly distort the complex output by MIAX. Furthermore a hydrophobic complementarity as well as the network of hydrogen bonds can clearly be mapped among the interacting units in the three cases of high affinity peptides. These analyses and several others discussed in the following methodology section unveil the most important forces at the atomic level that contribute to the binding of the peptides to vWf, and reinforce the postulated complex configurations.

*Methodology*

Given a target (protein), designing a drug to interact with it is intrinsically as difficult as predicting structural function. The approach is then to provide thousands if not millions of compounds that can be screened for their potential activity as drugs. This type of design, usually conceptualized as the design of an enzyme active site inhibitor, requires the substrate chemical structure as a starting point without further structural reference to or knowledge of the protein (enzyme). In contrast to this type of design, one can target the inhibition of protein-protein interactions. This is a technique experimentally realizable but is especially suitable for computer design when the structure of the complex structure is available, and thus it is more appropriate for the task undertaken in this study. Targeting protein-protein interactions, even knowing the 3D structure of the individual proteins requires identification of the key amino acids involved in the protein-protein interaction (PPI). Experimentally, this is done by point mutation experiments. Recent advances in crystallographic data analysis that allow the determination of protein complex structures make it possible to design inhibitors to proteins using bioinformatic approaches by targeting the interaction sites between the subunits composing the complexes. This approach can be applied to the complex made up of GPIb integrin and von Willebrand factor (vWf) which can be found in the Protein Data Bank (PDB) with the code PDB:1SQ0.

MIAX was applied to the analysis of the GPIb-vWf system and the characteristics of the interaction interface in the resulting complex were determined. A methodology to evaluate the interaction of the selected peptides with vWf that consists of six steps performed recursively for each of the peptides. These steps are described in detail in the following.

*A) Characterization of the interaction interface of the GPIb-vWf protein complex:*

Characterization of the interaction interface for the complex structure was performed by computing the decrement in surface area of the subunits at complex formation. SASA (solvent accessible surface area) was computed with a water molecule radius of 1.4 A. Differences in SASA for the amino acids enabled their identification as those involved or not involved in the interaction interface. Furthermore, computing distances among atoms belonging to different units in the respective interaction units allowed the inference of particular interactions between the units such as hydrogen bonds, electrostatic interactions or hydrophobic interactions, which can be compared with reported interactions or with those in the entries of interaction databases.

*B) Physicochemical characteristics of the interaction interfaces:*

Physicochemical characteristics of the interacting subunits (interacting proteins and peptides) are computed by means of the SOM-MIAX module in MIAX. Here, the main physicochemical characteristic computed for the GPIb and vWf is the relative hydrophobocity of regions on the proteins' surfaces. The calculation was carried out by using the molecular hydrophobic potential introduced by Brasseur (Brasseur R. (1991) Differentiation of lipid-associating helices by use of 3-dimensional molecular hydrophobicty potential, J. Biol. Chem. 266-24:16120-16127) and a learning algorithm that incorporates the self organized maps of Kohonen (Kohonen T. (1990) The Self-Organizing Map. Proceedings IEEE 78:1464-80). Finally, an image processing process was applied to define the limits of the hydrophobic patches on the surfaces of the interacting units.

*C) Generation of peptide sequences:*

Random peptide arrays of 1120 peptides made of D-amino acids were synthesized on a cellulose membrane using an AutoSpot ASP 222 peptide synthesizer (ABiMED, Langenfeld, Germany). The resulting replicate libraries of 15-mer sequences were probed for vWf binding function by exposing the membranes to purified vWf (a gift of Dr. F. A. Ofosu, McMaster University, Hamilton ON, Canada) and blocking with milk, then identifying positive spots with a polyclonal goat anti-human vWf IgG coupled to horseradish peroxidase (Cedarlane, Canada). Immunochemical detection was done using the chemiluminescent substrates from the Amersham Pharmacia ECL kit; and the resulting spots were recorded on photographic film. Negative controls consisted of probing the membranes with the antibodies only, but without prior exposure of the membrane to purified vWf.

*D) Modeling the 3D structures of the designed peptides:*

The three dimensional structures (3D) of peptides can be determined by *ab initio* calculations such as the system GAX (Del Carpio CA. (1996) A parallel genetic algorithm for polypeptide three dimensional structure prediction. A transputer implementation. J. Chem. Inf. and Comp. Sci. 36:258-269). Here a rather robust methodology was adopted to build the 3D structures of the peptides designed to bind vWf. This consisted of scanning the Brookhaven PDB for segments of high similarity to the sequences of the selected peptides. A FASTA search was performed in order to obtain those highly similar sequences and their structures were used as the initial conformations for the peptides. The 3D structures underwent a change from the L conformation to the D conformation and a series of minimizations and Molecular Dynamics simulations were performed to obtain the most energetically stable conformations for the peptides in solution. All these computations were performed using the force fields in AMBER-6 (Ponder JA. Case DA. (2003) Force fields for protein simulations. Adv. Prot. Chem. 66:27-85) and CHARM (Richichi A. Percheron I. (2002) CHARM: A catalog of high angular resolution measurements. A&A 386:492-503).

*E) Docking of the peptides to a receptor using MIAX:*

With the 3D structures of the interacting molecular entities, computation of the complex structure that they may form when they interact was done using the docking module of MIAX (Del Carpio CA. Qiang P. Ichiishi E. Koyama M. Kubo M. Endou A. Takaba H. Miyamoto A. (2006) Robotic path planning and protein complex modeling considering low frequency intra-molecular loop and domain motions. Genome Informatics 17:270-278). MIAX is endowed with three types of modules for docking macromolecules. The first is a rigid body docking module that is appropriate to discover interaction pathways when the structure of the resulting complex is known *a priori.* The second is the "soft docking" module, that docks two units of which the structures are known only in the isolated state. This being the present case, this module was applied first to dock the peptides to vWf. The third module in MIAX is constituted by the flexible docking of units, in which there is a rigorous analysis of the conformation of the side chains of interface amino acids. MIAX performs the docking taking into account the geometry of the molecules as well as the interaction energy of the system.

Geometric characteristics of the interacting subunits are considered by a discretization process of the molecular bodies and performing a grid point complementarity analysis of the subunits and their fit into 3D space. The interaction energies are computed by the following expression:

$$\Delta G^{AB(s)} = E_{hy} + E_{elec} + E_{hb} + E_{tor} + E_{desol} \qquad (1)$$

where $\Delta G^{AB(s)}$ is the change in free energy at complex formation in solution, and the terms in the right hand stand for the hydrophobic energy ($E_{hy}$), electrostatic interaction ($E_{elec}$), hydrogen bonding ($E_{hb}$), torsional energy ($E_{tor}$) and the energy of desolvation ($E_{desolv}$). Each of these terms is described in detail elsewhere (Del Carpio CA. Ichiishi E. Yoshimori A. Yoshikawa T. (2002) MIAX: A new paradigm to model bio-molecular interaction and complex formation in condensed phases. Proteins: Structure, Function and Genetics 48:696-732).

*F) Molecular dynamics simulation of the complexes to compute complex stability:*

The stability of the complexes obtained by the MIAX docking process was tested by means of molecular dynamic simulations using the AMBER-6 force field (Ponder JA. Case DA. (2003) Force fields for protein simulations. Adv. Prot. Chem. 66:27-85). The simulation is performed in vacuum and for 50 ps for each of the complexes. The objective of this simulation besides testing the stability of the complex obtained by the docking experiment is to detect any major change

in the conformation of the subunits, in particular changes in the interaction interface that may lead to improved accommodation of the ligand (peptide molecule) in the receptor.

*G) Characterization of peptide-vWf interaction interfaces and validation of the selected peptides:*

The characterization of the interaction interfaces of the decoys (peptide-vWf) output by MIAX followed by the molecular dynamics experiment is carried out in a similar way as in the case of the characterization of the interaction interface of the complex GPIb and vWf. The decrement of SASA of atoms constituting the peptides and vWf leads to the map of the interface in terms of the interacting atoms. The visualization of the interface and the identification of the main interactions such as hydrogen bonding and hydrophobic interactions are displayed using the LIGPLOT system (Wallace AC Laskowski RA. Thomton J M. (1995) LIGPLOT: A program to generate schematic diagrams of protein-ligand interactions. Prot. Eng. 8:127-134).

*Results*

The described methodology is applied to the set of peptides selected experimentally by binding to purified vWf. Since the desired peptides should be oriented to inhibit the interaction between GPIb and vWf, the first step is the characterization of this interface.

*Characterization of the interaction interface between vWf and GPIb:*

Figure 22 (a and b) shows the complex and the interaction interface for the complex GPIb-vWf, as recorded in PDB with the entry 1SQ0. Applying the SASA methodology to both units, using a water radius of 1.4 A, the result is shown in Figure 23 where the interaction surfaces are mapped on each of the subunits constituting the complex GPIb-vWf (Figure 23a: vWf, Figure 23b: GPIb). For an amino acid to be part of the interface, at least one of its constituting atoms is in contact with another atom of the interacting partner.

*Physicochemical characteristics of the interaction interfaces:*

One of the most important properties driving proteins to interact with each other is the hydrophobicity of their surfaces. This physicochemical characteristic of the protein surface is usually expressed in terms of the number of hydrophobic amino acids present in particular regions of the molecular surface. Here, a series of calculations were performed in order to obtain these regions, using the SOM module in MIAX. The learning steps were set to 6000, and the filtering coefficient was set to 5 (Del Carpio CA. Ichiishi E. Yoshimori A. Yoshikawa T. (2002) MIAX: A new paradigm to model bio-molecular interaction and complex formation in condensed phases. Proteins: Structure, Function and Genetics 48:696-732). The results are shown graphically in Figure 24 together with the list of the amino acids composing the main hydrophobic region, for each of the components of the complex of GPIb and vWf.

A careful inspection of the list of amino acids of the hydrophobic patch on vWf *(K549, W550*, *S562*, H563, *Y565*, R571, I580, *E596*, *K599*, *Y600*, *P603*, *Q604*, *I605*, P606, S607, R611, E613, *R632*) with those involved in the interaction with *GPIb: K549, W550, S562*, *Y565*, *E596*, *K599, Y600, P603, Q604, I605, R632* (Figure 24) shows that all of the computed interactive amino acids are present in the hydrophobic patch (concordances in italics). Furthermore, experimental studies by Shimizu *et al.* (Shimizu A. Matsushita T. Kondo T. Inden Y. Kojima T. Saito H. Hirai M. (2004) Identification of the amino residues of the platelet glycoprotein Ib (GPIb) essential for the von Willebrand Factor binding by clustered charged-to alanine scanning mutagenesis. Journal of Biol. Chem., 279-16:16285-16294) as well as those of Hauert *et al.* (Hauert J. Femandez-Carneado J. Michielin O. Mathieu S. Grell D. Schapira M. Spertini O. Mutter M. Tuchscherer G. Kovacsovics T. (2004) A template-assembled synthetic protein surface mimetic of the von Willebrand factor A1 domain inhibits botrocetin-induced platelet aggregation. Chembiochem 5:856-64) have established the importance of several of these amino acids by mutation assays that led to inhibition of the protein interaction between GPIb and vWf. They focus especially on amino acids R571, E613, K599 through P611 and R632, coinciding to a high degree with the computed results obtained here.

*Selection of peptides that interact with vWf:*

Peptides on random 15-mer peptide arrays that were built of D-amino acids were selected by their binding of vWf. Four sequences were identified: D-pep1 - VSRQNGKQYWAIKEG (SEQ ID NO: 1); D-pep2 - WQNEGTHVLSRCYEC (SEQ ID NO: 2); D-pep3 - RSARMQVCWNAFKNR (SEQ ID NO: 3); and D-pep4 - DSCPRDWDNNFLFFE (SEQ ID NO: 4). By definition, their binding to vWf identified them. However, where on vWf molecule they attached, and whether that binding site was at the vWf-GPIb interface, and thus whether they could potentially inhibit the vWf-GPIb interaction

remained to be determined. The identification of each vWf - peptide binding interface constitutes the results that follow.

*Modeling the 3D structures of the selected D-peptides:*

Three dimensional structures for the experimentally selected peptides are modeled according to the methodology described above. Results for the four peptides of the present study are summarized in Table I.

TABLE I. Characteristics of the D-Peptide Conformation Modeling Process

| Peptide | D-pep1 | D-pep2 | D-pep3 | D-pep4 |
|---|---|---|---|---|
| Sequence | VSRQNGKQ YWAIKEG | WQNEGTHV LSRCYEC | RSARMQVC WNAFKNR | DSCPRDWD NNFLFFE |
| FASTA output of most similar sequence | I50-G64 of PDB: 1XSX | D141-C155 of PDB: 1M8Y | R13-K27 of PDB: 1W81 | P31-L45 of PDB: 1A88 |
| Energy of D-peptide after conformation change and MD (kcal/mol) | 595.81 | 140.19 | 55.49 | 141.49 |
| Energy of D-peptide after minimization (kcal/mol) | -777.22 | -397.87 | -954.67 | -985.43 |

Figure 25 shows the MD simulation process for each one, while sketches of the structures as ribbon models are shown in Figure 26.

In Table I the sequence of each peptide is shown together with the most similar sequence derived by a FASTA search from PDB. The backbone of such a peptide was used as the starting backbone structure for each peptide before molecular dynamics simulation. Table I also summarizes the energies of the D-peptides after undergoing the conformation shift and the MD simulation process until energy convergence is achieved The table also shows energies after minimization of the MD derived peptide structures, this procedure is performed in order to obtain the most realistic conformation for each peptide in solution.

*Docking peptides to vWf using MIAX:*

After modeling the 3D structure of the four target D-peptides, the next step was to dock the peptides to the target receptor, which in this case was vWf, using MIAX (*vide supra*). The complexes obtained by MIAX were submitted to further MD simulation and energy minimization to relax the structure. Since the purpose is to block the protein-protein interaction between vWf and GPIb, we performed a further analysis of the interface of the GPIb-vWf complex. This additional analysis consisted of computing the entire network of hydrogen bonds and hydrophobic interactions that bind these two proteins. The computation was carried out using HYPLUS (Xu D. Tsai CJ. Nussinov R. (1997) Hydrogen bonds and salt bridges across protein-protein interfaces Protein Engineering 10: 999-1012) which outputs the quantitative characteristics of the hydrogen bonds and LIGPLOT (Wallace AC Laskowski RA. Thomton J M. (1995) LIGPLOT: A program to generate schematic diagrams of protein-ligand interactions. Prot. Eng. 8:127-134) for their visualization. This additional computation was aimed at enabling a comparison of the interfaces of the original complex and the peptide-vWf complexes obtained by docking (*vide infra*). Table II shows the inter-unit hydrogen bonds computed using the HYPLUS system, while Figure 27 illustrates the network of intra molecular hydrogen bonds of vWf and the hydrogen bonds at the interface between the vWf and the GPIb. The latter set of bonds is marked with a circle around the donor amino acid number.

Table II summarizes the characteristics of the hydrogen bonds at the interface. The main characteristics shown are the polypeptide chains (A for vWf and B for GPIb), the number of the amino acids involved in the hydrogen bond as donor and acceptor, and the PDB names of the donor and acceptor atoms. Additionally, the Donor - Acceptor distance (D-A), the hydrogen acceptor (H-A), and the respective angles are also illustrated in Table II.

TABLE II. Characteristics of the intermolecular hydrogen bonds of the vWf-GPIb complex

| DONOR | | ACCEPTOR | | | DHA[a] | | Dist. H-A | Angles | |
|---|---|---|---|---|---|---|---|---|---|
| Amino Acid | Atom | Amino Acid | Atom | Dist D-A | dist | angle | | H-A-AA | D-A-AA |
| [a]A0549[b] LYS[c] | NZ[d] | B0005-GLU | OE1 | 3.32 | 11.79 | 170 | 2.33 | 99.8 | 100.2 |
| A0562-SER | N | B0239-MET | O | 2.91 | 5.39 | 160 | 1.95 | 146.8 | 150.2 |
| B0239-MET | N | *A0562-SER | O | 3.01 | 5.39 | 148 | 2.11 | 145.3 | 154.9 |
| A0564-ALA | N | B0237-LYS | O | 3.21 | 5.29 | 167 | 2.22 | 128.5 | 126.5 |
| B0237-LYS | N | A0564-ALA | O | 3.04 | 5.29 | 153 | 2.12 | 134.6 | 142.8 |
| A0571-ARG | NE | B0018-ASP | OD2 | 2.91 | 9.38 | 166 | 1.93 | 138 | 134.9 |
| *A0571-ARG | NH2 | B0039-SER | OG | 2.87 | 10.86 | 109 | 2.39 | 130.2 | 136.5 |
| B0228-TYR | OH | *A0596-GLU | OE1 | 2.91 | 11.22 | 171 | 1.92 | 103.6 | 102.6 |
| *A0599-LYS | NZ | B0198-PRO | O | 3.13 | 8.6 | 157 | 2.19 | 123.5 | 123.7 |
| A0599-LYS | NZ | B0228-TYR | OH | 2.86 | 12.57 | 159 | 1.9 | 115.7 | 116.7 |
| B0152-LYS | NZ | A0603-PHE | O | 3.01 | 9.7 | 157 | 2.06 | 127.9 | 133.8 |
| *A0604-GLN | NE2 | B0176-THR | OG1 | 2.85 | 8.54 | 164 | 1.87 | 147.2 | 145.4 |
| *A0632-ARG | NH2 | B0225-GLU | OE1 | 2.52 | 11.09 | 119 | 1.88 | 120.9 | 112.2 |

a) Subunit: A = vWf, B = GPIb
b) Amino acid number within the subunit
c) Amino acid name
d) Atom name
e) DHA (Donor, Hydrogen, Acceptor)
* Homolog hydrogen bonds, found in the vWf-GPIb complex and in the vWf-peptide complexes below.

*Peptide docking results:*

Each docking experiment was performed in two stages. The first was the soft docking (Del Carpio CA. Rajjack SA, Koyama M, Kubo M, Ichiishi E, Miyamoto A. (2005) A graph theoretical approach for analysis of protein flexibility change at protein complex formation. Genome Informatics 16:148-160), and the second consisted of performing molecular dynamics on each complex (vWf-peptide) to relax the structure and to evaluate the most important features of the complex output by MIAX, as mentioned before. Figure 28 illustrates the MD simulation for each of the complexes obtained by the docking experiment.

Table III shows the energies of the complexes after the energy minimization procedure. Binding energy (BE) calculated as:

$$BE = E(complex) - [E(vWf) + E(D\text{-}peptide)] \qquad (2)$$

was computed for each complex to evaluate the stability of the derived species.

Table III. Binding energies (BE) for the vWf - D peptide complexes

| | vWf - D-pep1 | vWf - D-pep2 | vWf - D-pep3 | vWf - D-pep4 |
|---|---|---|---|---|
| Energy vWf (kcal/mol) | -3350.00 | -3350.00 | -3350.00 | -3350 |
| Energy D-pep (kcal/mol) | -777.22 | -397.87 | -954.67 | -985.43 |
| Energy Complex (kcal/mol) | -4870.12 | -4220.2 | -5110.20 | -5460.31 |
| BE (kcal/mol) | -742.90 | -472.33 | -805.53 | -1124.88 |

A final evaluation of the complex output by the computational process described here was performed to characterize the complex in terms of the network of hydrogen bonds at the interaction interface as well as the hydrophobic interactions identified by means of the MIAX, HYPLUS and LIGPLOT software programs.

The soft docking module of MIAX has the characteristic of optimizing the contacts among receptor and ligand atoms that may attract each other by electrostatic and London forces, and outputs a list of candidate conformations for the complex (decoys). MIAX does not *a priori* require the specification of the binding site, however information on the interaction interface of any of the interacting subunits is valuable at the final ranking stage. The ranking of the decoys is then performed according to the scoring function that takes into account the energy of the complex, the geometric complementarity of the receptor and ligand as well as the *a priori* knowledge of 'hot spots' (which in this case are the hydrophobic patches on the surfaces of the receptor). Here we analyzed decoys that have been ranked high, and we performed an analysis of the forces that may lead to vWf - D-peptide complex formation. We have mainly studied these aspects from the number of hydrogen bonds formed in the interface, and the stability of the complex expressed in terms of the binding energy (Equation 2) resulting from the energy to which the MD run converges after a certain number of simulation steps and a further energy minimization process. This evaluation has been extended to compare the plausible hydrogen bonds in the interface of the predicted complexes with those in the experimental vWf-GPIb complex.

*Complex of vWf - D-pep1:*

For the first complex obtained by docking D-pep1 with vWf factor (vWf - D-pep1) Figure 29 and Table IV summarize the characteristics of this complex. Table IV summarizes the characteristics of the inter-molecular hydrogen bonds for this complex. Hydrogen bonds sharing homology with those of the original complex vWf-GPIb are marked with an asterisk.

TABLE IV. Characteristics of the intermolecular hydrogen bonds for the vWf - D-pep1 complex.

| DONOR | | ACCEPTOR | | | DHA | | | Angles | |
|---|---|---|---|---|---|---|---|---|---|
| Amino Acid | Ato m | Amino Acid | Ato m | Dist D-A | dist | ang le | Dis tH-A | H-A-AA | D-A-AA |
| 0004-GLN | NE2 | *A0562-SER | O | 2.9 6 | 7 | 120 | 2.3 4 | 120.7 | 137.4 |
| B0001-VAL | N | A0563-HIS | NE2 | 3.1 7 | 5.57 | 152 | 2.2 4 | 103.1 | 92.6 |
| B0002-SER | OG | A0565-TYR | OH | 3.2 9 | 7.14 | 172 | 2.3 1 | 92.2 | 93.4 |
| *A0571-ARG | NH1 | B0014-GLU | OE1 | 2.8 | 11.36 | 118 | 2.1 8 | 135.8 | 151.4 |
| B0011-ALA | N | *A0604-GLN | O | 2.9 9 | 4.47 | 129 | 2.2 2 | 114.7 | 120 |
| B0007-LYS | NZ | *A0604-GLN | OE1 | 3.0 8 | 8.25 | 155 | 2.1 6 | 108.4 | 107.4 |
| A0607-SER | N | B0015-GLY | OXT | 2.9 2 | 4.47 | 172 | 1.9 3 | 117.6 | 114.8 |
| A0608-LYS | NZ | B0014-GLU | O | 3.1 1 | 7.94 | 121 | 2.4 | 7 150.7 | 146.6 |
| A0616-ARG | NH1 | B0015-GLY | O | 3.0 1 | 11-.31 | 145 | 2.1 | 128.8 | 129.8 |

(Symbols as described for Table I; Chain A = vWf , B = D-pep1)

Fig. 29 illustrates the position of the ligand peptide D-pep1 in the complex output as number one by MIAX. The interaction can be quantified by the number of hydrogen bonds formed in the interaction interface, which is shown in Table IV, where the amino acids holding the donor and acceptor atoms are listed together with the distances and angles of each hydrogen bond. Amino acids belonging to vWf are represented by chain A while amino acids of the ligands are those belonging to chain B in the table. Additionally, asterisks point to homolog hydrogen bonds observed in the wild type complex of vWf - GPIb. It is evident that vWf amino acids ARG571, SER562, GLN604, SER607, HIS563 and TYR565, play a critical role in the formation of this complex, although ARG571, SER607 and HIS563 are not directly involved in the vWf - GPIb interface as computed. The binding energy of the vWf - D-pep1 complex is -742.9 kcal/mol (Table III).

*Complex of vWf - D-pep2:*

For the second complex (vWf - D-pep2) Figure 30 and table V summarize the characteristics of the complex

obtained by docking D-pep2 with vWf factor. Table V summarizes the characteristics of the inter-molecular hydrogen bonds for this complex. Hydrogen bonds sharing homology with those of the original wild type complex vWf-GPIb are marked with an asterisk.

TABLE V. Characteristics of the intermolecular hydrogen bonds for the vWf - D-pep2 complex

| DONOR | | ACCEPTOR | | Dist D-A | DHA | | Dist H-A | Angles | |
|---|---|---|---|---|---|---|---|---|---|
| Amino Acid | Atom | Amino Acid | Atom | | dist | angle | | H-A-AA | D-A-AA |
| B0012-CYS | N | *A0562-SER | O | 3.45 | 6.4 | 172.0 | 2.44 | 166.9 | 166.9 |
| B0013-TYR | OH | A0599-LYS | O | 2.96 | 10.0 | 142.2 | 2.15 | 109.0 | 106.9 |
| *A0599-LYS | NZ | B0008-VAL | O | 2.75 | 10.5 | 125.7 | 2.00 | 138.2 | 154.1 |
| *A0599-LYS | NZ | B0009-LEU | O | 3.31 | 8.83 | 135.5 | 2.48 | 111.0 | 123.8 |
| A0629-ARG | NE | B0004-GLU | OE1 | 2.97 | 5.74 | 155.8 | 2.02 | 94.2 | 94.6 |
| A0632-ARG | NE | B0002-GLN | O | 3.00 | 6.93 | 159.5 | 2.03 | 140.7 | 144.5 |
| *A0632-ARG | NH2 | B0002-GLN | O | 3.25 | 6.93 | 145.6 | 2.39 | 162.0 | 169.1 |
| *A0632-ARG | NH2 | B0003-ASN | OD1 | 3.28 | 6.4 | 142.3 | 2.44 | 146.3 | 156.0 |
| A0633-ASN | ND2 | B0004-GLU | O | 3.18 | 7.75 | 141.3 | 2.34 | 100.5 | 111.8 |

(Symbols as described for Table I; Chain A = vWf, B = D-pep2).

Figure 30 illustrates the position of the ligand peptide D-pep2 in the complex output as number one by MIAX. The interaction can be quantified by the number of hydrogen bonds formed in the interaction interface, which is shown in Table V, where the amino acids holding the donor and acceptor atoms are listed together with the distances and angles of each hydrogen bond. Amino acids belonging to vWf are represented by chain A while amino acids of the ligands are those belonging to chain B in the table. Additionally, asterisks point to homolog hydrogen bonds observed in the wild type complex vWf-GPIb. It is evident that in the case of vWf - D-pep2 complex the amino acids of vWf ARG562, ARG599, ARG629, ARG632 and ASN633, play a critical role in the formation of the complex of which ASN633 and ARG629 were not in the computed vWf - GPIb interface (Figure 23). The binding energy of the vWf - D-pep2 complex is 472.33 kcal/mol (Table III).

*Complex of vWf - D-pep3:*

For the third complex (vWf - D-pep3) Figure 31 and table VI summarize the characteristics of the complex obtained by docking D-pep3 with vWf factor. Table VI summarizes the characteristics of the inter-molecular hydrogen bonds for this complex. Hydrogen bonds sharing homology with those of the original wild type complex vWf-GPIb are marked with an asterisk

TABLE VI. Characteristics of intermolecular hydrogen bonds for the vWf - D-pep3 complex.

| DONOR | | ACCEPTOR | | Dist D-A | DHA | | Dist H-A | Angles | |
|---|---|---|---|---|---|---|---|---|---|
| Amino Acid | Atom | Amino Acid | Atom | | Dist | angle | | H-A-AA | D-A-AA |
| B0001-ARG | NE | A0560-ASP | OD1 | 2.83 | 7.62 | 138.9 | 1.97 | 115.9 | 106.2 |
| B0008-CYS | SG | A0563-HIS | NE2 | 3.38 | 5.57 | 127.6 | 2.42 | 122.8 | 105.3 |

(Symbols as described for Table I; Chain A = vWf , B = D-pep3)

The interaction can be quantified by the number of hydrogen bonds formed in the interaction interface, which is shown in Table VI, where the amino acids holding the donor and acceptor atoms are listed together with the distances and angles of each hydrogen bond. Amino acids belonging to vWf are represented by chain A while amino acids of the ligands are those belonging to chain B in the table. Additionally, asterisks point to homolog hydrogen bonds observed in the wild type complex vWf - GPIb. It is evident that in the case of the vWf - D-pep3 complex the amino acids A560 A563, play a critical role in the formation of the complex. Although neither of these amino acids is directly involved in

the computed vWf - GPIb interface, the peptide sequence should have inhibitory activity as it binds to amino acids that are next to those involved in the interface. The binding energy of the vWf - D-pep3 complex is - 805.53 kcal/mol (Table III).

*Complex of vWf - D-pep4:*

For the fourth complex (vWf - D-pep4) Figure 32 and Table VII summarize the characteristics of the complex obtained by docking D-pep4 with vWf factor. Table VII summarizes the characteristics of the inter-molecular hydrogen bonds for this complex. Hydrogen bonds sharing homology with those of the original wild type complex vWf-GPIb are marked with an asterisk.

TABLE VII. Characteristics of the intermolecular hydrogen bonds for complex of D-pep4 - vWf.

| DONOR | | ACCEPTOR | | | DHA | | | Angles | |
|---|---|---|---|---|---|---|---|---|---|
| Amino Acid | Atom | Amino Acid | Atom | Dist D-A | dist | angle | Dist. H-A | H-A-AA | D-A-AA |
| B0005-ARG | NH2 | *A0596-GLU | OE2 | 2.77 | 11.87 | 157.6 | 1.77 | 135.1 | 135.9 |
| A0600-TYR | OH | B0015-GLU | OXT | 2.66 | 9.7 | 158 | 1.73 | 157.6 | 150.3 |
| A0629-ARG | NH2 | B0008-ASP | OD1 | 3.03 | 8.12 | 143.7 | 2.15 | 134.5 | 123.5 |
| A0637-TYR | OH | B0001-ASP | OD2 | 2.96 | 10.86 | 155.3 | 2.08 | 94.8 | 100.4 |
| B0005- | NH1 | A0637- | OH | 3.46 | 13.45 | 173 | 2.44 | 123.8 | 122.8 |
| ARG | | TYR | | | | | | | |

(Symbols as described for Table I; Chain A = vWf, B = D-pep4)

Figure 32 illustrates the position of the ligand peptide D-pep4 in the complex output as number one by MIAX. The interaction can be quantified by the number of hydrogen bonds formed in the interaction interface, which is shown in Table VII, where the amino acids holding the donor and acceptor atoms are listed together with the distances and angles of each hydrogen bond. Amino acids belonging to vWf are represented by chain A while amino acids of the ligands are those belonging to chain B in the table. Additionally, asterisks point to homolog hydrogen bonds observed in the wild type complex vWf - GPIb. It is evident that in the case of the vWf - D-pep4 complex that the amino acids GLU 596, ARG629 and TYR637 play a critical role in the formation of the complex, and of them GLU596 is also involved in the originally computed vWf - GPlb binding interface. The binding energy of the vWf - D-pep4 complex is - 1124.53 kcal/mol (Table III).

*Conclusions:*

A computational study was performed to confirm peptide-protein interaction among experimentally selected peptides and vWf. The peptides that bind to vWf are intended to inhibit or mimic the protein-protein interaction between vWf and GPIb therefore their binding locations are of paramount importance. Four peptides were selected experimentally from among 1120 on a random peptide array by identifying them on the basis of their ability to bind to vWf. Prior to the computational study, the location of peptides' binding site on vWf was unknown and therefore their potential to interfere with or mimic the vWf-GPIb interaction remained to be determined. The peptides' evaluation as potential mimotope receptors and/or inhibitors of the protein-protein interaction between GPIb and vWf consisted of using bioinformatics systems to design the three dimensional structures of the peptides and to describe their potential spatial relationships with vWf. Three dimensional structures for the peptides were modeled using homology studies, to get an initial conformation for the D-peptides, and molecular dynamics and energy minimization processes were used to obtain the optimal 3D structures for each peptide. The optimal structures were docked to their prospective binding partner, vWf, by means of the flexible docking module of MIAX. Since MIAX outputs a large number of decoys (>4000) ranked by geometrical and energy instances (geometrical complementarity and interaction energy), only the best decoys were selected for each of the four studies corresponding to the four peptides initially selected. These complexes were further relaxed by MD simulations.

Interfaces of the final vWf - D-peptide complexes were then evaluated for hydrogen bonding networks and hydrophobic interactions. Binding energy results show that D-pep4 binds to the vWf molecule with the highest affinity, followed by D-pep3, then D-pep1 and finally D-pep2. However, D-pep2 binds to vWf and is able to realize far more hydrogen bonds than the other three peptides. Many of the hydrogen bonds realized by docking D-pep2 to vWf share homology to the hydrogen bonds found in the original protein-protein complex (vWf - GPIb). These bonds are highlighted

with asterisks in Table II. The number of similar hydrogen bonds that D-pep2 is able to make with vWf in the best decoy output by MIAX is 5 while D-pep1 is able to make only 4 bonds, D-pep4 one and D-pep3 none. Stabilities of the complexes output by MIAX, signaled by the MD simulation, show that vWf - D-pep4 is marginally the most stable, followed by vWf - D-pep3, then vWf - D-pep1, with the most unstable being again vWf - D-pep2.

In conclusion D-pep4 may interact with the highest affinity and interaction energy to vWf followed by D-pep3 and D-pep1, while D-pep2 is the lowest ranked. Thus the D-pep4 peptide would be the most likely molecule to mimic and/or interfere with the formation of the GPIb-vWf complex and would constitute a preferred mimotope receptor and/or inhibitory peptide. It should be understood, however, that the D-pep1, D-pep2 and D-pep3 peptides were shown to bind vWf and therefore also constitite mimotope receptors and/or inhibitory peptides that may be used in accordance with the present invention either alone or in combination with the above-described carrier molecule to provide an artificial platelet and/or antithrombotic molecule.

*Mimotope receptors*

The following peptides represent exemplary embodiments of mimotope receptors, or receptor molecules for use in accordance with the invention (upper case= L-peptide; lower case D-peptide):

*Peptides that replace GPIb (and therefore bind vWf or inhibit GPIb-vWf interaction):*

Source - a random d-peptide array was probed with vWf and developed with anti-vWf-FITC. "Forward" D-amino acid peptides were tested, although L-amino acid versions of the peptides as well as the reverse (retro) of both L- and D- sequences will be also encompassed within the scope of these receptor molecules.

D-pep1: vsrqngkqywaikeg L-pep1: VSRQNGKQYWAIKEG (SEQ ID NO:1)
D-pep2: wqnegthvlsrcyec L-pep2: WQNEGTHVLSRCYEC (SEQ ID NO:2)
D-pep3: rsarmqvcwnafknr L-pep3: RSARMQVCWNAFKNR (SEQ ID NO:3)
D-pep4: dscprdwdnnflffe L-pep4: DSCPRDWDNNFLFFE (SEQ ID NO:4)

DR-pep1: gekiawyqkgnqrsv LR-pep1: GEKIAWYQKGNQRSV (SEQ ID NO:5)
DR-pep2: ceycrslvhtgenqw LR-pep2: CEYCRSLVTHGENQW (SEQ ID NO:6)
DR-pep3: rnkfanwcvqmrasr LR-pep3: RNKFANWCVQMRASR (SEQ ID NO:7)
DR-pep4: efflfnndwdrpcsd LR-pep4: EFFLFNNDWDRPCSD (SEQ ID NO:8)

Based on the theoretically calculated binding site described above, a general GPIb analogue sequence for binding vWf has also been calculated and the consensus sequence is as follows:

**VA $(X)_3$K $(X)_2$F $(X)_2$EDVK (X) MT**

where x represents any uncharged amino acid.

Embodiments of a peptide or mimotope receptor molecule having the above consensus sequence can be prepared using D or L amino acids, and can further be prepared in either the forward or reverse orientation as follows, whereby the peptides are shown from left to right in the N-terminal - C-terminal direction:

D-ideal: vaxxxkxxfxxedvkxmt L-ideal: VAXXXKXXFXXEDVKXM (SEQ ID NO:9)
DR-ideal: tmxkvdexxfxxkxxxav LR-ideal: TMXKVDEXXFXXKXXAV (SEQ ID NO:10)

Further embodiments of a peptide or mimotope receptor molecule are prepared by synthesizing overlapping peptides of the vWf amino acid sequence. Peptides of the following sequences can be prepared using D or L amino acids, and can further be prepared in either the forward or reverse orientation whereby the peptides are shown from left to right in the N-terminal - C-terminal direction:

| | | |
|---|---|---|
| D-brown: shayiglkdr | L-brown: SHAYIGLKDR | (SEQ ID NO:11) |
| D-black: evlkytlfqi | L-black: EVLKYTLFQI | (SEQ ID NO:12) |
| DR-brown: rdklgiyahs | LR-brown: RD KLGIYAHS | (SEQ ID NO:13) |
| DR-black: igfltyklve | LR-black: IQFLTYKLVE | (SEQ ID NO:14) |

Further embodiments of a peptide or mimotope receptor molecule have been obtained by probing d-peptide arrays with fibrinogen (developed with anti-fibrinogen-FITC), whereby the following four (4) strongly binding peptides were identified. These peptides mimic GPIIbIIIa, the fibrinogen receptor on peptides. Peptides of such sequences can

be prepared using D or L amino acids, and can further be prepared in either the forward or reverse orientation The peptides are shown from left to right in the N-terminal - C-terminal direction:

D-fib-a: smtsmcyligapkyk L-fib-a: SMTSMCYLIGAPKYK (SEQ ID NO:15)
D-fib-b: kyqcyapahpsyvny L-fib-b: KYQCYAPAHPSYVNY (SEQ ID NO:16)
D-fib-c: fkwswewqgqeayyd L-fib-c: FKWSWEWQGQEAYYD (SEQ ID NO:17)
D-fib-d: friyyvyttsqqdsc L-fib-d: FRIYYVYTTSQQDSC (SEQ ID NO:18)

DR-fib-a: kykpagilycmstms LR-fib-a: KYKPAGILYCMSTMS (SEQ ID NO:19)
DR-fib-b: ynvysphapaycqyk LR-fib-b: YNVYSPHAPAYCQYK (SEQ ID NO:20)
DR-fib-c: dyyaeqgqwewswkf LR-fib-c: DYYAEQGQWEWSWKF (SEQ ID NO:21)
DR-fib-d: csdqqsttyvyyirf LR-fib-d: CSDQQSTTYVYYIRF (SEQ ID NO:22)

If not already present within their sequence, each of the above described peptides can be modified to include a Cys residue to facilitate attachment, e.g. via a Michael Addition reaction (discussed in further detail below), to the carrier molecule. In a preferred embodiment the Cys residue is located at either the C- or N-terminal end of the peptide sequence. In addition, and if necessary or advantageous to facilitate greater access to the binding region of the peptide, a poly-Gly or similar linker sequence can be added as follows:

$$C\text{-}(G)_n\text{-}PEP;$$

or

$$PEP\text{-}(G)_n\text{-}C.$$

wherein n is preferably 0-5.

In yet further embodiments, any of the above sequences, modified or otherwise, can be further modified to insert one or more spectrophotometrically traceable amino acids within the sequence, e.g. Phe, Trp or Tyr residues. In a preferred embodiment the spectrophotometrically traceable amino acid comprises one or more Trp residues inserted at the C- or N-terminal end of the peptide, or within a poly-Gly tag inserted into the peptide sequence. Addition of such a spectroscopically active amino acid allows for easy fluorescence absorption/emission detection of the peptide.

In still further embodiments, the sequences of the above-identified molecules can be modified by preparing peptide analogs, e.g. through conservative replacement of the amino acid moieties, having 90% sequence identity, preferably a 95% sequence identity.

**(III) PLATELET SUBSTITUTE/ANTITHROMBOTIC:**

Combination of the carrier molecule (I) and receptor molecule (II) described above can be effected by means of a covalent linkage to provide a synthetic platelet substitute and/or antithrombotic in accordance with the present invention.

In an embodiment, the covalent linkage may be formed by means of a conjugate addition reaction (Michael-type addition) between the amine or thiol groups of a peptide receptor molecule (e.g. mimotope receptor) and free acrylate ends of a hydrogel-coated carrier molecule (e.g. hydrogel-liposome), as illustrated in the following reaction scheme

(Scheme I):

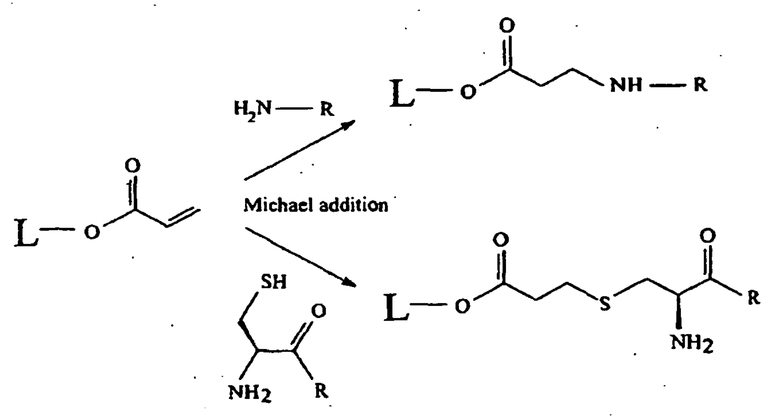


Scheme I

**L - hydrogel liposomes**
**R - peptide**

The top reaction shows additions to primary amines, and the bottom to cysteine via a thiol group, and other additions are possible (e.g. to amides).

*Michael Addition reaction:*

The addition reaction is derived from Hubbell et al. (United States Patent 6,958,212 October 25, 2005) and Mather B.D. et al. ("Michael addition reactions in macromolecular design for emerging technologies", Prog. Polym. Sci. 31 (2006) 487-531.). The reaction was carried out at pH 7.4 - 8.0, 30°C, for 20h using an exemplary peptide, D-Pep3 (RSARMQVCWNAFKNR) which has a net charge of +4 and a molecular weight (MW) of 1867.

In brief, 1.7 mg of cys-containing peptide (D-pep3) was dissolved in 0.6 mL of saline (154 mM NaCl). 170 uL of the resulting 2.8 mg/mL peptide solution was then mixed with 0.93 mL of washed hydrogel-liposome (HL)-5.4 mM lipid in Hepes buffer-1 (50 mM Hepes, 100mM NaCl, pH 8) - to give a final volume of 1.1 mL solution whereby D-Pep3 concentration is 0.43 mg/mL, lipid concentration is 4.5 mM and pH is 8. A control sample was also prepared using the same final concentration of HL in Hepes buffer-1, pH 8 without D-Pep3 peptide. The mixtures were incubated for 20 hours at 30 °C with shaking. The samples were centrifuged at 15,000 x g for 15 min. The supernatant was removed and the pellets were resuspended in 1.5 mL of Hepes buffer-2 (10 mM Hepes, 150 mM NaCl, pH 7.4). This washing step was repeated two (2) more times. The resulting D-Pep3-liposomes and controls were stored at 4°C under nitrogen.

The samples were analyzed for phosphate content in order to determinate the lipid concentration and also for tryptophan emission to measure the concentration of the attached peptide.

*Confirmation of HL-bound peptide surface availability:*

As illustrated in Figure 33, validation of the covalent linkage of the P-HL was undertaken using a peptide motif known to bind platelets, i.e., RGD. Using flow cytometry platelet interaction with FITC-labelled HL, with or without attached RGD peptides (CGGGGG-RGDW), was measured as follows.

Platelets were prepared from anticoagulated whole blood by slow centrifugation (15 min at 150 x g) and retention of the platelet-rich plasma (PRP) supernatant. Platelet counts were adjusted to $400*10^6$/mL. 2.5 μL platelets were mixed with increasing amounts, 0, 5, 10, 15, 20, 30, 40, 50, 75 μL of a 1 mM suspension of HL or P-HL. The HL or P-HL were made containing 1% FITC head-group-labelled phospholipidlipid incorporated in the liposome [Egg-PE-Fluorescein/DPPE/DPPC/Choles: 1/30/39/30 mol%]. The HL or P-HL were suspended in bicarbonate buffer pH 7.4 (20 mM $NaHCO_3$, 150 mM NaCl). All volumes were normalized to 100 μL The mixtures were co-incubated at room temperature, for 2 hrs, with slow agitation. The platelet population was analysed by flow cytometry for green fluorescence (FITC) carried by the liposomes.10,000 events were counted.

As shown, the RGD-peptide substituted hydrogel liposomes show greater attachment to platelets than hydrogel liposomes without the peptide.

*Stability of the covalent linkage:*

As illustrated in Figures 34 and 35, the covalent attachment of a D-Pep3 peptide to HL by means of the linkage

reaction described above is stable for a duration in excess of one month. Further, the trp-dependent fluorescence remains with the liposome and does not leach out after washing (Figure 34), which would have been the case if the peptides had merely permeated the liposomes' hydrogel. In brief: fluorescence emission at 348 nm (excitation 280 nm) of the single tryptophan of peptide D-Pep3 was used to analyze the stability of the peptide on the liposomes' surface. Fluorescence emission standard curves of trp-associated fluorescence were prepared using a range of concentrations from 1 μM to 20 μM free peptide D-Pep3 in the presence of 1 mM $PEG_{2000}$ or HL liposomes (Figure 34).

HL and $PEG_{2000}$ liposomes were reacted with D-Pep3 peptide (as described according to Scheme I) then washed and adjusted to a concentration of 1mM lipid. The fluorescence emission was measured. The samples were stored under nitrogen, in the dark, at 4°C for one month. The liposomes were again washed, resuspended to the same lipid concentration and the fluorescence emission was measured as before (Figure 35). The attachment was stable for >1 months in hypothermic liquid storage.

*Capture of vWf from Plasma Cryoglobulin Fraction*

As shown in Figure 36, D-amino acid mimotope receptors attached to hydrogel liposomes are able to bind their specific ligand. Aliquots of 30 μL of HL or D-Pep3-HL liposomes were mixed with 5 μL human cryoprecipitate diluted 1/100 in Hepes Buffered Saline, (10mM Hepes, 154 mM NaCl, pH 7.4.) The samples were diluted in buffer to achieve final lipid concentrations of 0.1, 0.25, 0.5, 0.75 and 1 mM, and incubated for 1 hour at 37°C. Anti-vWf-FITC antibodies were added (final dilution 1/128) and incubated for 40 minutes at RT. Samples were diluted with 350 μL of formal-saline and analyzed by flow cytometry.

*P-HL interaction with platelets in plasma*

Mimotope receptor-hydrogel-liposomes can interact with platelets (Figure 37a to Figure 37e) and form aggregates when sheared together, while liposomes without the peptide (HL) can not. In brief, platelet-rich plasma (PRP) was prepared as described and mixed 1:1 with Acetate Citrate Dextrose buffer, pH 6, and the platelets were pelleted by centrifugation for 15 min at 514 x g. The platelets were resuspended in 1 ml of 10 mM Hepes Buffered Saline, counted and adjusted to a concentration of $400*10^6$/mL. 40 μL HL or D-Pep3-HL liposomes, at 0.75 mM lipid, (lipid /peptide ratio 1/72) were mixed with 5 μL washed platelets and incubated for one hour at room temperature. Red anti-Cd42-PE antibodies (7 μL) to detect platelets and 5 μL green anti-vWf-FITC antibodies at a 1/160 dilution were added to the tubes and incubated one more time for 30 min. The samples were diluted to 1 mL with formolsaline solution and analysed by flow cytometry. Singlet platelets (platelet gate), singlet liposomes and co-aggregates (P1 gate) were scored.

The post-stained aggregates were positive for CD42 (GPIb) and vWf. All the relevant controls were done, and the critical double stained examples (with anti-CD42-PE and anti-vWf-FITC) were as follows:

Figure 37a: platelets alone, double stained with anti-CD42-PE and anti-vWF-FITC
Figure 37b: HL (control liposomes), double stained
Figure 37c: P-HL (D3-peptide hydrogel liposomes), double stained
Figure 37d: HL + platelets, (double stained)
Figure 37e: P-HL + platelets, (double stained)

As is clearly evident from the large population that appears in the P1 gate only with the peptide-coupled liposomes (Figure 37e), the peptide-hydrogel-liposomes interact with the platelets to form aggregates. This type of experiment constitutes formal evidence that such synthetic constructs can be used as a platelet substitute.

*Tolerance and bleeding control by P-HL in mice*

Through careful observation during administration of the D-Pep3-HL, it was found that that such liposomes are well-tolerated by mice in the short-term, and reduced bleeding.

Two mice were injected with D-Pep3-HL via tail vein and were observed to be undisturbed by the treament. Three hours later, prior to sacrifice, a blood sample was taken from each injected mouse and the control mouse. The tail-vein of the control mouse was pricked and -50 uL of blood was retrieved. The tail vein of each of the D-Pep3-HL test mice was pricked in the same manner but a blood sample large enough to test could not be obtained. A sample still could not be obtained by cutting off the tip of the tail. This observed significant reduction in bleeding validates the use of the platelet substitute of the present invention for blood clotting and bleeding control.

It will be understood that numerous modifications to the present invention will appear to those skilled in the art. Accordingly, the above description and accompanying drawings should be taken as illustrative of the invention and not in a limiting sense. It will further be understood that it is intended to cover any variations, uses, or adaptations of the

invention following, in general, the principles of the invention and including such departures from the present disclosure as come within known or customary practice within the art to which the invention pertains and as may be applied to the essential features herein before set forth, and as follows in the scope of the appended claims.

## Claims

1. A synthetic platelet substitute that interacts with platelets and the (sub)endothelium, comprising:

   a. a carrier molecule comprising lipidic particles with a cross-linked surface mesh, the lipidic particles comprising: an inner lipidic particle of pharmaceutically acceptable particle-forming lipids; hydrophilic polymer chains linked to the surface of the lipidic particle, the hydrophilic polymer chains being straight chain non-toxic and comprising a crosslinkable end group at free ends thereof; and cross-linker groups linking the end groups of the hydrophilic polymer chains to form the cross-linked surface mesh; and
   b. at least one receptor molecule attached to the surface of the carrier molecule, wherein the receptor molecule is a peptide selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO: 16, SEQ ID NO:17, SEQ ID NO:18, SEQ ID NO:19, SEQ ID NO:20, SEQ ID NO:21, SEQ ID NO:22, analogs thereof having 90% sequence identity, and modified peptides thereof having an insertion of a Cys residue and/or a spectrophotometrically traceable amino acid and/or a poly-Gly tag consisting of 1 to 5 Gly residues.

2. The synthetic platelet substitute of claim 1 wherein the peptide is synthesized using D-amino acids or L-amino acids.

3. The synthetic platelet substitute of claim I wherein the at least one receptor molecule is covalently attached to the carrier molecule between an amine group of the receptor molecule and free acrylate ends of a hydrogel-coated carrier molecule.

4. The synthetic platelet substitute of claim I wherein a plurality of receptor molecules are attached to the surface of the carrier molecule.

5. The synthetic platelet substitute of claim 1, wherein the inner lipidic particles comprise liposomes, the liposomes comprising 1,2 dipalmitoyl-sn-glycero-3-phosphoethanolamine (DPPE), 1,2 dipalmitoyl-sn-glycero-3-phosphocholine (DPPC) and cholesterol (CHOL).

6. The synthetic platelet substitute of claim 1, wherein the hydrophilic polymer chains comprise polyethylene glycol with an acrylate end group.

7. The synthetic platelet substitute of claim 1, wherein the cross-linker groups comprise polyethylene glycol diacrylate.

8. An antithrombotic composition that interacts with platelets and the (sub)endothelium, comprising: a peptide selected from the group consisting of SEQ ID NO:1, SEQ ID NO:2, SEQ ID NO:3, SEQ ID NO:4, SEQ ID NO:5, SEQ ID NO: 6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, analogs thereof having 90% sequence identity, modified peptides thereof having an insertion of a Cys residue and/or a spectrophotometrically traceable amino acid and/or a poly-Gly tag consisting of 1. to 5 Gly residues, and combinations thereof, wherein the peptide is attached by means of a covalent linkage to a carrier molecule, and wherein the carrier molecule comprises lipidic particles with a cross-linked surface mesh, the lipidic particles comprising: an inner lipidic particle of pharmaceutically acceptable particle-forming lipids; hydrophilic polymer chains linked to the surface of the lipidic particle, the hydrophilic polymer chains being straight chain non-toxic and comprising a crosslinkable end group at free ends thereof; and cross-linker groups linking the end groups of the hydrophilic polymer chains to form the cross-linked surface mesh.

9. The antithrombotic composition of claim 8, wherein the peptide is synthesized using D-amino acids or L-amino acids.

10. The antithrombotic composition of claim 8, wherein the peptide is covalently attached to the carrier molecule at an amine group of the receptor molecule and free acrylate ends of a hydrogel-coated carrier molecule.

11. The antithrombotic composition of claim 8, wherein the inner lipidic particles comprise liposomes, vesicles, micelles, or combinations thereof.

12. The antithrombotic composition of claim 11, wherein the liposomes comprise a molar ratio of about 40:30:30, respectively, of 1,2 dipalmitoyl-sn-glycero-3-phosphoethanolamine, 1,2 dipalmitoyl-sn-glycero-3-phosphocholine, and

cholesterol.

**13.** The antithrombotic composition of claim 8, wherein the hydrophilic polymer chains comprise polyethylene glycol with an acrylate end group.

**14.** The antithrombotic composition of claim 8, wherein the cross-linker groups comprise polyethylene glycol diacrylate.

**15.** A method for preparing a synthetic platelet substitute that interacts with platelets and the (sub)endothelium, said synthetic platelet substitute comprising a receptor molecule and a carrier molecule, said method comprising:

a. preparing a carrier molecule comprising lipidic particles with a cross-linked surface mesh by

i. preparing lipidic particles comprising pharmaceutically acceptable lipids,
ii. binding hydrophilic polymer chains to the surface of the lipidic particles wherein the hydrophilic polymer chains are straight chain non-toxic polymers, and
iii. cross-linking the hydrophilic polymer chains to form the cross-linked surface mesh wherein said cross-linking comprises cross-linking free ends of the hydrophilic polymer claims with a cross-linker; and

b. attaching at least one receptor molecule to the surface of the carrier molecule, wherein the receptor molecule is a peptide selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, analogs thereof having 90% sequence identity, and modified peptides thereof having an insertion of a Cys residue and/or a spectrophotometrically traceable amino acid and/or a poly-Gly tag consisting of I to 5 Gly residues.

**16.** The method of claim 15 wherein the peptide is synthesized using D-amino acids or L-amino acids.

**17.** The method of claim 15 wherein the at least one receptor molecule is attached by means of a conjugate addition reaction between an amine group of the receptor molecule and free acrylate ends of a hydrogel-coated carrier molecule.

**18.** The method of claim 15 wherein a plurality of receptor molecules are attached to the surface of the carrier molecule.

**19.** The method according to claim 15, wherein the lipidic particles in step (i) comprise liposomes, the liposomes being prepared using 1,2 dipalmitoyl-sn-glycero-3-phosphoethanolamine (DPPE), 1,2 dipalmitoyl-sn-glycero-3-phosphocholine (DPPC) and cholesterol (CHOL).

**20.** The method according to claim 15, wherein the hydrophilic polymer chains in step (ii) comprise polyethylene glycol with an acrylate end group.

**21.** The method according to claim 15, wherein the cross-linker comprises polyethylene glycol diacrylate.

## Patentansprüche

**1.** Ein synthetischer Blutplättchenersatz, der mit Blutplättchen und dem (Sub)Endothel interagiert, umfassend:

(a) ein Trägermolekül, umfassend Lipidteiichen mit einem vernetzten Oberflächennetz, wobei die Lipidteilchen umfassen: ein inneres Lipidteilchen aus pharmazeutisch akzeptablen teilchenbildenden Lipiden; hydrophile Polymerketten, die an die Oberfläche des Lipidteilchens gebunden sind, wobei die hydrophilen Polymerketten geradkettige nicht-toxische Polymere sind, die an ihren freien Enden eine vernetzbare Endgruppe umfassen; und Vernetzungsgruppen, die die Endgruppen der hydrophilen Polymerketten verbinden, um das vernetzte Oberflächennetz zu bilden; und
(b) mindestens ein Rezeptormolekül, das an die Oberfläche des Trägermoleküls gebunden ist, wobei das Rezeptormolekül ein Peptid ist, das aus der Gruppe bestehend aus SEQ ID NO:1, SEQ ID NO:2, SEQ ID NO: 3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:

17, SEQ ID NO:18, SEQ ID NO:19, SEQ ID NO:20, SEQ ID NO:21, SEQ ID NO:22, Analogen davon, die 90% Sequenzidentität aufweisen, und modifizierten Peptiden davon mit einer Insertion eines Cys-Restes und/oder einer spektrophotometrisch nachweisbaren Aminosäure und/oder eines poly-Gly-Anhängsels, das aus 1 bis 5 Gly-Resten besteht, ausgewählt ist.

2. Synthetischer Blutplättchenersatz nach Anspruch 1, wobei das Peptid unter Verwendung von D-Aminosäuren oder L-Aminosäuren synthetisiert wird.

3. Synthetischer Blutplättchenersatz nach Anspruch 1, wobei das mindestens eine Rezeptormolekül an das Trägermolekül über eine kovalente Bindung zwischen einer Aminogruppe des Rezeptormoleküls und freien Acrylatenden eines Hydrogel-beschichteten Trägermoleküls gebunden ist.

4. Synthetischer Blutplättchenersatz nach Anspruch 1, wobei mehrere Rezeptormoleküle an die Oberfläche des Trägermoleküls gebunden sind.

5. Synthetischer Blutplättchenersatz nach Anspruch 1, wobei die inneren Lipidteilchen Liposomen umfassen, wobei die Liposomen 1,2 Dipalmitoyl-sn-glycero-3-phosphoethanolamin (DPPE), 1,2 Dipalmitoyl-sn-glycero-3-phosphocholin (DPPC) und Cholesterin (CHOL) umfassen.

6. Synthetischer Blutplättchenersatz nach Anspruch 1, wobei die hydrophilen Polymerketten Polyethylenglykol mit einer Acrylat-Endgruppe umfassen.

7. Synthetischer Blutplättchenersatz nach Anspruch 1, wobei die Vernetzungsgruppen Polyethylenglykoldiacrylat umfassen.

8. Eine antithrombotische Zusammensetzung, die mit Blutplättchen und dem (Sub)Endothel interagiert, umfassend: ein Peptid, das aus der Gruppe bestehend aus SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, Analogen davon, die 90% Sequenzidentität aufweisen, modifizierten Peptiden davon mit einer Insertion eines Cys-Restes und/oder einer spektrophotometrisch nachweisbaren Aminosäure und/oder eines poly-Gly-Anhängsels, das aus 1 bis 5 Gly-Resten besteht, und Kombinationen davon, ausgewählt ist, wobei das Peptid mittels einer kovalenten Bindung an ein Trägermolekül gebunden ist, und wobei das Trägermolekül Lipidteilchen mit einem vernetzten Oberflächennetz umfasst, wobei die Lipidteilchen umfassen: ein inneres Lipidteilchen aus pharmazeutisch akzeptablen teilchenbildenden Lipiden; hydrophile Polymerketten, die an die Oberfläche des Lipidteilchens gebunden sind, wobei die hydrophilen Polymerketten geradkettige nicht-toxische Polymere sind, die an ihren freien Enden eine vernetzbare Endgruppe umfassen; und Vernetzungsgruppen, die die Endgruppen der hydrophilen Polymerketten verbinden, um das vernetzte Oberflächennetz zu bilden.

9. Antithrombotische Zusammensetzung nach Anspruch 8, wobei das Peptid unter Verwendung von D-Aminosäuren oder L-Aminosäuren synthetisiert wird.

10. Antithrombotische Zusammensetzung nach Anspruch 8, wobei das Peptid an das Trägermolekül über eine kovalente Bindung an einer Aminogruppe des Rezeptormoleküls und freien Acrylatenden eines Hydrogel-beschichteten Trägermoleküls gebunden ist.

11. Antithrombotische Zusammensetzung nach Anspruch 8, wobei die inneren Lipidteilchen Liposomen, Vesikel, Mizellen oder Kombinationen davon umfassen.

12. Antithrombotische Zusammensetzung nach Anspruch 11, wobei die Liposomen 1,2 Dipalmitoyl-sn-glycero-3-phosphoethanolamin, 1,2 Dipalmitoyl-sn-glycero-3-phosphocholin, beziehungsweise Cholesterin in einem molaren Verhältnis von etwa 40:30:30 umfassen.

13. Antithrombotische Zusammensetzung nach Anspruch 8, wobei die hydrophilen Polymerketten Polyethylenglykol mit einer Acrylat-Endgruppe umfassen.

14. Antithrombotische Zusammensetzung nach Anspruch 8, wobei die Vernetzungsgruppen Polyethylenglykoldiacrylat umfassen.

**15.** Ein Verfahren zur Herstellung eines synthetischen Blutplättchenersatzes, der mit Blutplättchen und dem (Sub) Endothel interagiert, wobei der synthetischer Blutplättchenersatz ein Rezeptormolekül und ein Trägermolekül umfasst, wobei das Verfahren umfasst:

(a) Herstellung eines Trägermoleküls, umfassend Lipidteilchen mit einem vernetzten Oberflächennetz, durch

(i) Bereitstellen von Lipidteilchen, die pharmazeutisch akzeptable Lipide umfassen,
(ii) Binden von hydrophilen Polymerketten an die Oberfläche der Lipidteilchen, wobei die hydrophilen Polymerketten geradkettige nicht-toxische Polymere sind, und
(iii) Vernetzen der hydrophilen Polymerketten, um das vernetzte Oberflächennetz zu bilden, wobei das Vernetzen die Vernetzung von freien Enden der hydrophilen Polymerketten mit einem Vernetzungsmittel umfasst; und

(b) Bindung mindestens eines Rezeptormoleküls an die Oberfläche des Trägermoleküls, wobei das Rezeptormolekül ein Peptid ist, das aus der Gruppe bestehend aus SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, Analogen davon, die 90% Sequenzidentität aufweisen, und modifizierten Peptiden davon mit einer Insertion eines Cys-Restes und/oder einer spektrophotometrisch nachweisbaren Aminosäure und/oder eines poly-Gly-Anhängsels, das aus 1 bis 5 Gly-Resten besteht, ausgewählt ist.

**16.** Verfahren nach Anspruch 15, wobei das Peptid unter Verwendung von D-Aminosäuren oder L-Aminosäuren synthetisiert wird.

**17.** Verfahren nach Anspruch 15, wobei das mindestens eine Rezeptormolekül mittels einer Konjugat-Additionsreaktion zwischen einer Aminogruppe des Rezeptormoleküls und freien Acrylatenden eines Hydrogel-beschichteten Trägermoleküls gebunden ist.

**18.** Verfahren nach Anspruch 15, wobei mehrere Rezeptormoleküle an die Oberfläche des Trägermoleküls gebunden sind.

**19.** Verfahren nach Anspruch 15, wobei die Lipidteilchen in Schritt (i) Liposomen umfassen, wobei die Liposomen unter Verwendung von 1,2 Dipalmitoyl-sn-glycero-3-phosphoethanolamin (DPPE), 1,2 Dipalmitoyl-sn-glycero-3-phosphocholin (DPPC) und Cholesterin (CHOL) hergestellt werden.

**20.** Verfahren nach Anspruch 15, wobei die hydrophilen Polymerketten in Schritt (ii) Polyethylenglykol mit einer Acrylat-Endgruppe umfassen.

**21.** Verfahren nach Anspruch 15, wobei das Vernetzungsmittel Polyethylenglykoldiacrylat umfasst.

## Revendications

**1.** Produit de substitution synthétique de plaquettes, qui interagit avec des plaquettes et le sous-endothélium, comprenant :

a. une molécule porteuse comprenant des particules lipidiques ayant un réseau de surface réticulé, les particules lipidiques comprenant : une particule lipidique interne constituée de lipides de formation de particule acceptables du point de vue pharmaceutique ; des chaînes polymères hydrophiles liées à la surface de la particule lipidique, les chaînes polymères hydrophiles étant des chaînes linéaires non toxiques et comprenant un groupe de terminaison pouvant être réticulé au niveau d'extrémités libres ; et des groupes de réticulation reliant les groupes d'extrémité des chaînes polymères hydrophiles pour former le réseau de surface réticulé ; et
b. au moins une molécule réceptrice fixée à la surface de la molécule porteuse, la molécule réceptrice étant un peptide sélectionné dans le groupe comprenant SEQ ID NO:1, SEQ ID NO:2, SEQ ID NO:3, SEQ ID NO: 4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO: 18, SEQ ID NO:19, SEQ ID NO:20, SEQ ID NO:21, SEQ ID NO:22, des éléments analogues à ceux-ci ayant

une identité de séquence à 90 %, et des peptides modifiés de ceux-ci comportant l'insertion d'un résidu Cys et/ou un acide aminé traçable par spectrophotométrie et/ou une étiquette poly-Gly constituée de 1 à 5 résidus Gly.

**2.** Produit de substitution synthétique de plaquettes selon la revendication 1, dans lequel le peptide est synthétisé en utilisant des acides aminés D ou des acides aminés L.

**3.** Produit de substitution synthétique de plaquettes selon la revendication 1, dans lequel ladite au moins une molécule réceptrice est fixée de façon covalente à une molécule porteuse entre un groupe amine de la molécule réceptrice et des extrémités acrylates libres d'une molécule porteuse revêtue d'hydrogel.

**4.** Produit de substitution synthétique de plaquettes selon la revendication 1, dans lequel plusieurs molécules réceptrices sont fixées à la surface de la molécule porteuse.

**5.** Produit de substitution synthétique de plaquettes selon la revendication 1, dans lequel les particules lipidiques intérieures comprennent des liposomes, les liposomes comprenant du 1,2-dipalmitoyl-sn-glycéro-3-phosphoéthanolamine (DPPE), du 1,2-dipalmitoyl-sn-glycéro-3-phosphocholine (DPPC) et du cholestérol (CHOL).

**6.** Produit de substitution synthétique de plaquettes selon la revendication 1, dans lequel les chaînes polymères hydrophiles comprennent du polyéthylène glycol avec un groupe d'extrémité acrylate.

**7.** Produit de substitution synthétique de plaquettes selon la revendication 1, dans lequel les groupes de réticulation comprennent du diacrylate de polyéthylène glycol.

**8.** Composition anti-thrombose qui interagit avec des plaquettes et le sous-endothélium, comprenant un peptide sélectionné dans le groupe comprenant SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, des éléments analogues à ceux-ci ayant une identité de séquence à 90 %, et des peptides modifiés de ceux-ci comportant l'insertion d'un résidu Cys et/ou un acide aminé traçable par spectrophotométrie et/ou une étiquette poly-Gly constituée de 1 à 5 résidus Gly, et leurs combinaisons, dans laquelle le peptide est fixé au moyen d'une liaison covalente à une molécule porteuse, et dans laquelle la molécule porteuse comprend des particules lipidiques ayant un réseau de surface réticulé, les particules lipidiques comprenant : une particule lipidique intérieure de lipides de formation de particule acceptables du point de vue pharmaceutique ; des chaînes polymères hydrophiles liées à la surface de la particule lipidique, les chaînes polymères hydrophiles étant des chaînes linéaires non toxiques et comprenant un groupe d'extrémité pouvant être réticulé au niveau d'extrémités libres ; et des groupes de réticulation reliant les groupes d'extrémités des chaînes polymères hydrophiles pour former le réseau de surface réticulé.

**9.** Composition anti-thrombose selon la revendication 8, dans laquelle le peptide est synthétisé en utilisant des acides aminés D ou des acides aminés L.

**10.** Composition anti-thrombose selon la revendication 8, dans laquelle le peptide est fixé de façon covalente à la molécule porteuse au niveau d'un groupe amine de la molécule réceptrice et d'extrémités acrylates libres d'une molécule porteuse revêtue d'hydrogel.

**11.** Composition anti-thrombose selon la revendication 8, dans laquelle les particules lipidiques intérieures comprennent des liposomes, des vésicules, des micelles, ou des combinaisons de ceux-ci.

**12.** Composition anti-thrombose selon la revendication 11, dans laquelle les liposomes ont un rapport molaire d'environ 40:30:30, respectivement, de 1,2-dipalmitoyl-sn-glycéro-3-phos-phoéthanolamine, de 1,2-dipalmitoyl-sn-glycéro-3-phosphocholine et de cholestérol.

**13.** Composition anti-thrombose selon la revendication 8, dans laquelle les chaînes polymères hydrophiles comprennent du polyéthylène glycol ayant un groupe d'extrémité acrylate.

**14.** Composition anti-thrombose selon la revendication 8, dans laquelle les groupes de réticulation comprennent du diacrylate de polyéthylène glycol.

**15.** Procédé de préparation d'un produit de substitution synthétique de plaquettes qui interagit avec des plaquettes et le sous-endothélium, le produit de substitution synthétique de plaquettes comprenant une molécule réceptrice et une molécule porteuse, le procédé comprenant les étapes suivantes :

a. préparer une molécule porteuse comprenant des particules lipidiques ayant un réseau de surface réticulé

i. en préparant des particules lipidiques comprenant des lipides acceptables du point de vue pharmaceutique,
ii. en liant des chaînes polymères hydrophiles à la surface des particules lipidiques, les chaînes polymères hydrophiles étant des polymères non toxiques à chaînes linéaires, et
iii. en réticulant les chaînes polymères hydrophiles pour former le réseau de surface réticulé, la réticulation comprenant la réticulation d'extrémités libres des chaînes polymères hydrophiles à l'aide d'un agent de réticulation ; et

b. fixer au moins une molécule réceptrice à la surface de la molécule porteuse, la molécule réceptrice étant un peptide sélectionné dans le groupe comprenant SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, des éléments analogues à ceux-ci ayant une identité de séquence à 90 %, et des peptides modifiés de ceux-ci comportant l'insertion d'un résidu Cys et/ou un acide aminé traçable par spectrophotométrie et/ou une étiquette poly-Gly constituée de 1 à 5 résidus Gly.

**16.** Procédé selon la revendication 15, dans lequel le peptide est synthétisé en utilisant des acides aminés D ou des acides aminés L.

**17.** Procédé selon la revendication 15, dans lequel ladite au moins une molécule réceptrice est fixée au moyen d'une réaction d'addition conjuguée entre un groupe amine de la molécule réceptrice et des extrémités acrylates libres d'une molécule porteuse revêtue d'hydrogel.

**18.** Procédé selon la revendication 15, dans lequel la pluralité de molécules réceptrices est fixée à la surface de la molécule porteuse.

**19.** Procédé selon la revendication 15, dans lequel les particules lipidiques de l'étape (i) comprennent des liposomes, les liposomes étant préparés en utilisant du 1,2-dipalmitoyl-sn-glycéro-3-phosphoéthanolamine (DPPE), du 1,2-dipalmitoyl-sn-glycéro-3-phosphocholine (DPPC) et du cholestérol (CHOL).

**20.** Procédé selon la revendication 15, dans lequel les chaînes polymères hydrophiles de l'étape (ii) comprennent du polyéthylène glycol avec un groupe d'extrémité acrylate.

**21.** Procédé selon la revendication 15, dans lequel l'agent de réticulation comprend du diacrylate de polyéthylène glycol.

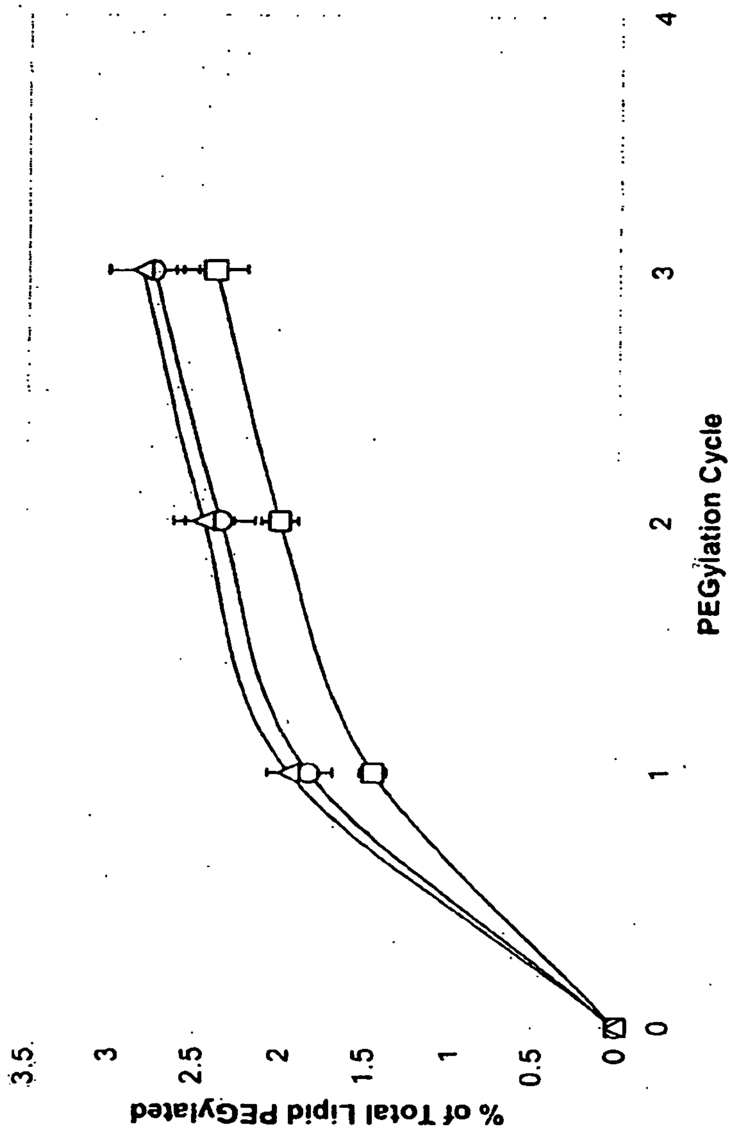
% of Total Lipid PEGylated
0
0.5
1
1.5
2
2.5
3
3.5
PEGylation Cycle
0
1
2
3
4

Figure 1

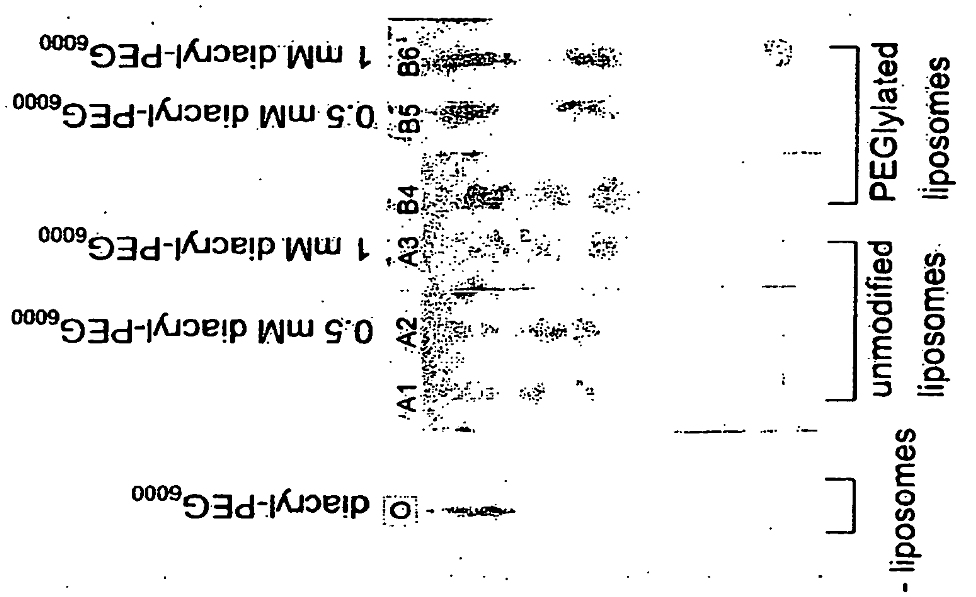

1 mM diacryl-PEG6000
0.5 mM diacryl-PEG6000
1 mM diacryl-PEG6000
0.5 mM diacryl-PEG6000
diacryl-PEG6000
O
A1
A2
A3
B4
B5
B6
- liposomes
unmodified liposomes
PEGylated liposomes


Figure 2

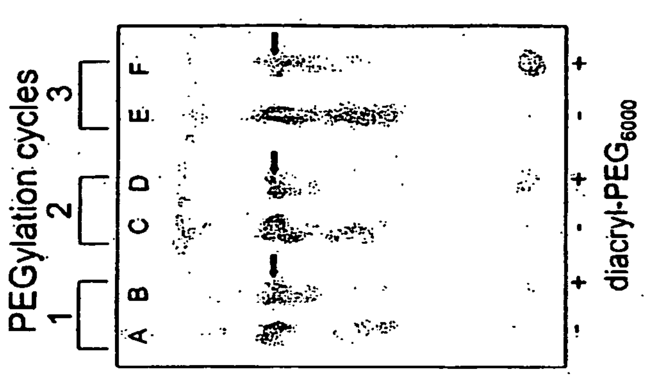


Figure 3

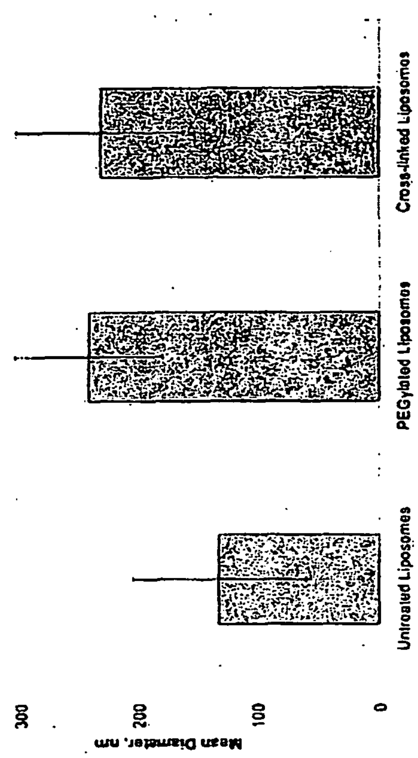


Figure 4

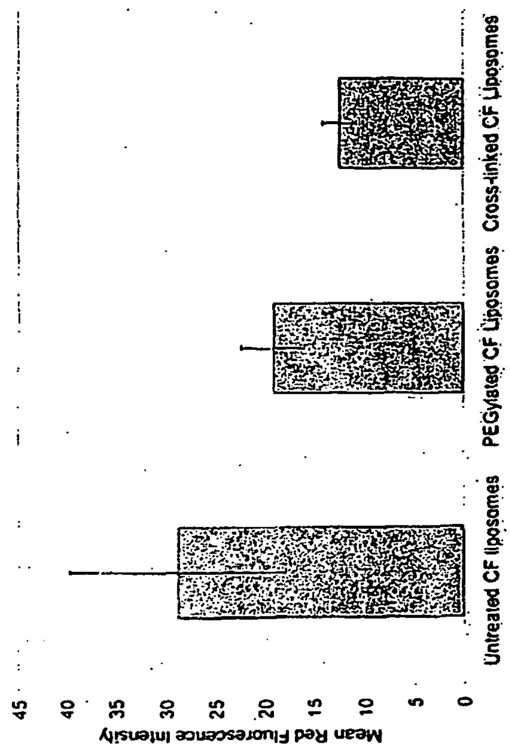


Figure 5

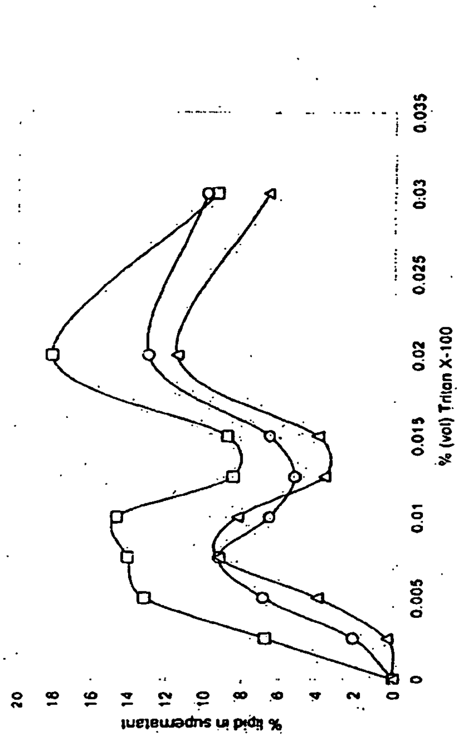


Figure 6

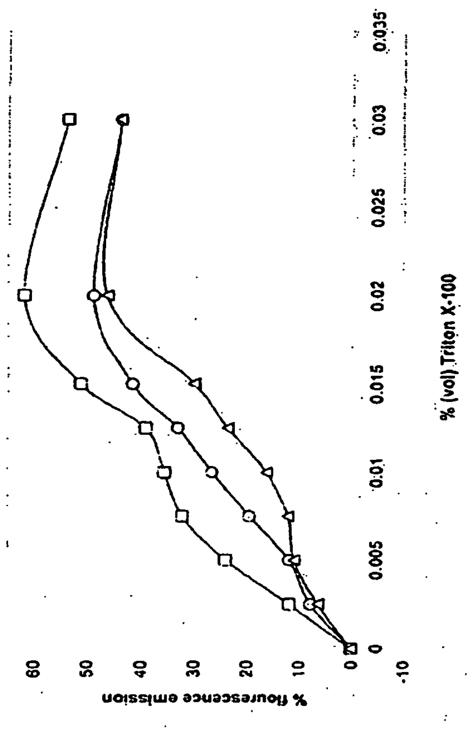


Figure 7

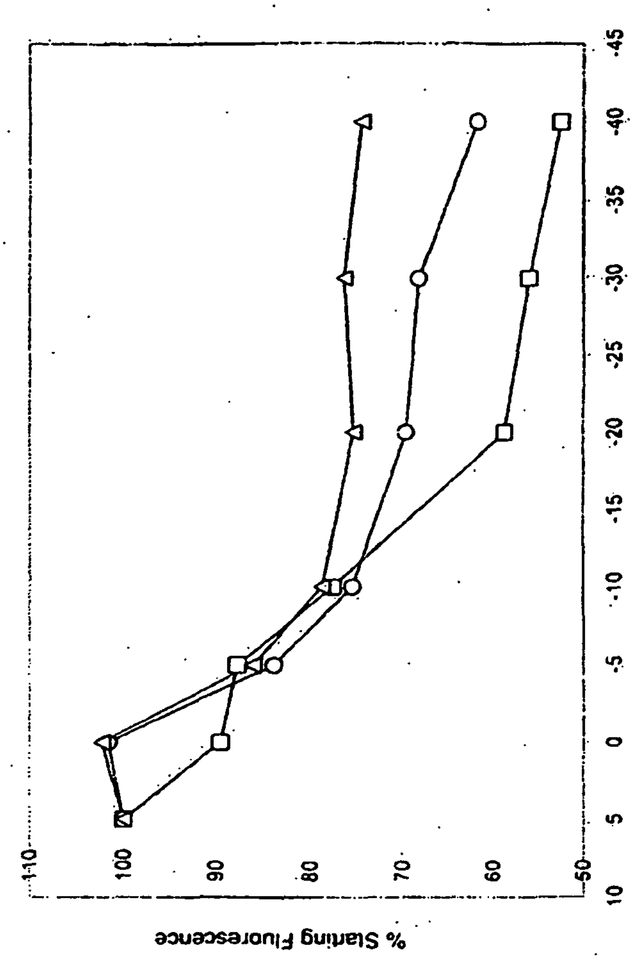
% Starting Fluorescence
110
100
90
80
70
60
50
10
5
0
-5
-10
-15
-20
-25
-30
-35
-40
-45
Temperature, °C

Figure 8

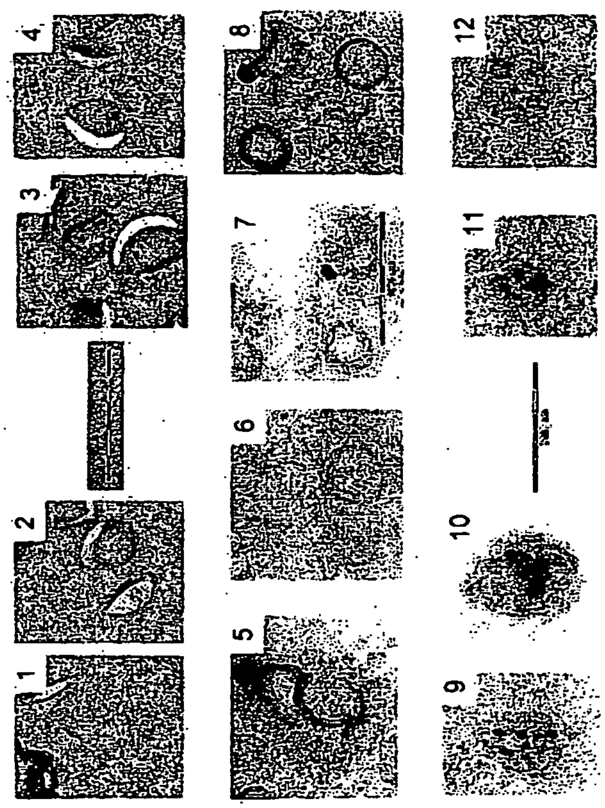


Figure 9

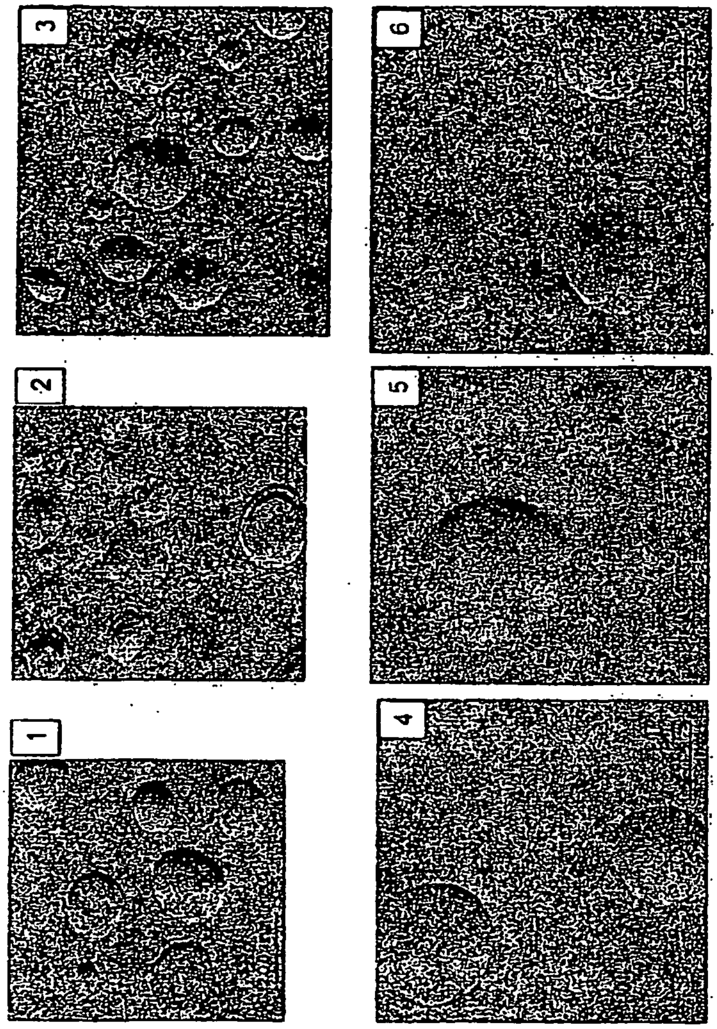


Figure 10

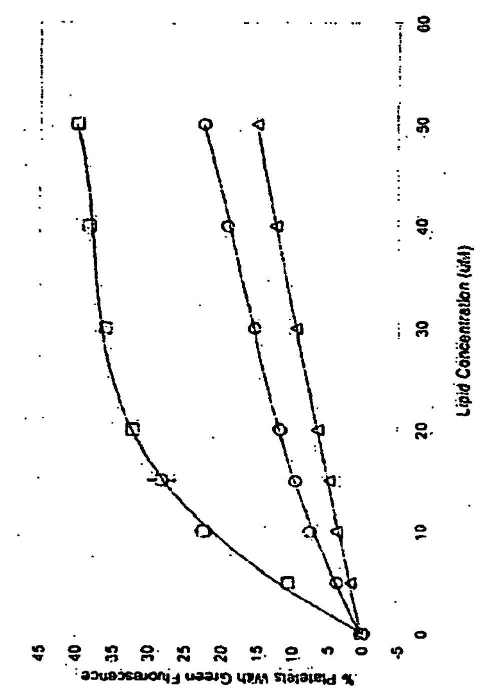


Figure 11

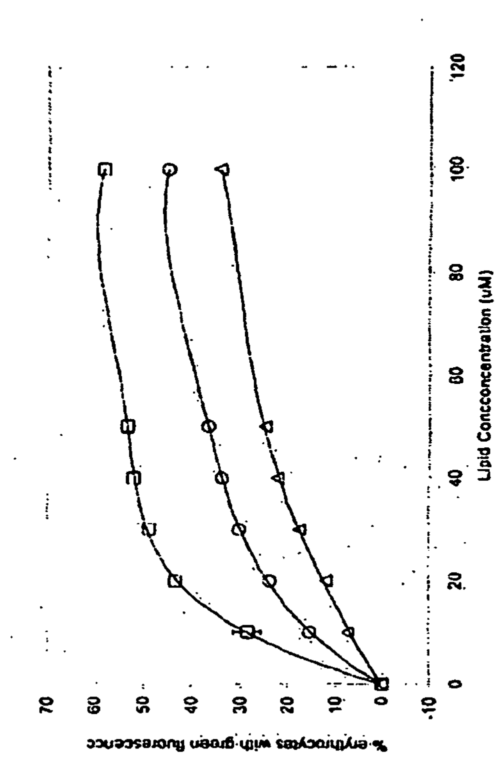


Figure 12

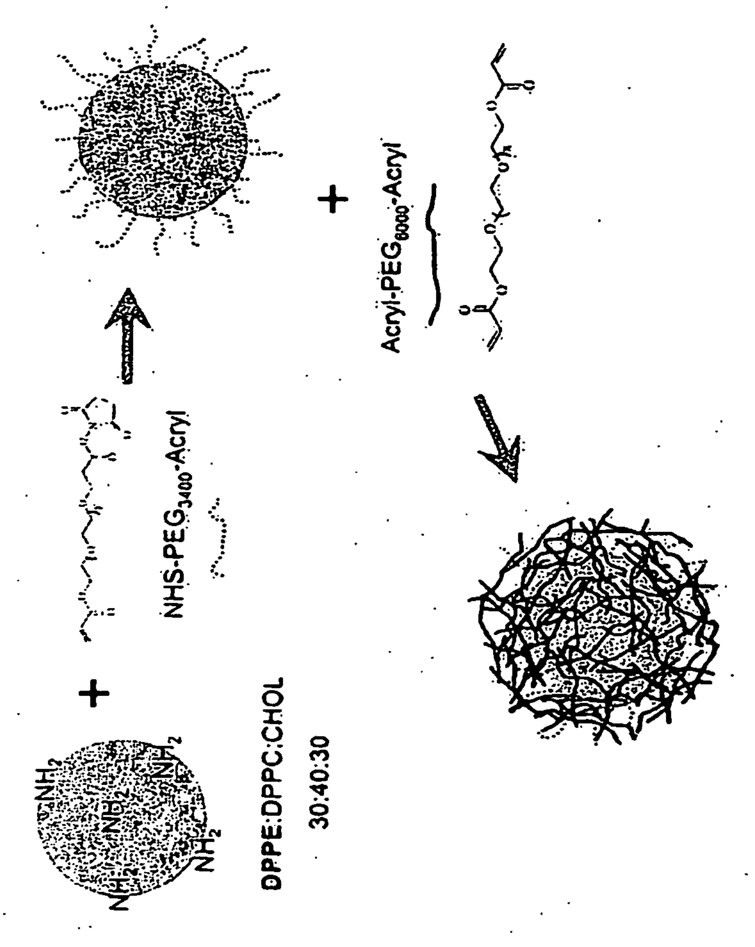
DPPE:DPPC:CHOL
30:40:30
NHS-PEG$_{3400}$-Acryl
Acryl-PEG$_{6000}$-Acryl

Figure 13

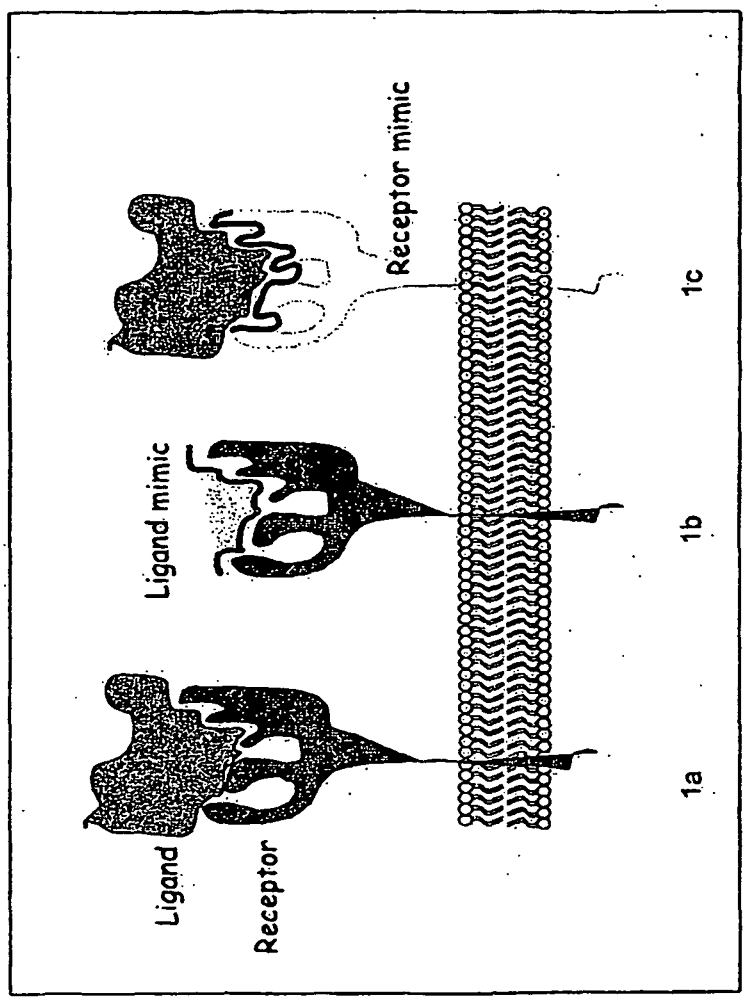


Figure 14a-14c

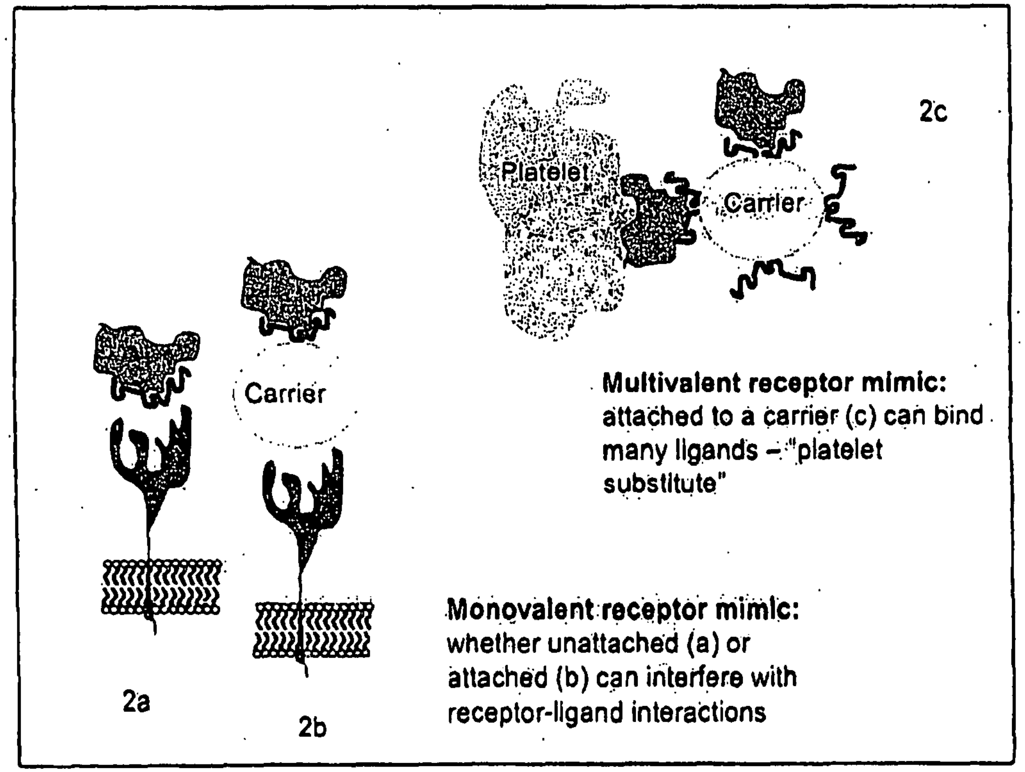


Figure 15a-15c

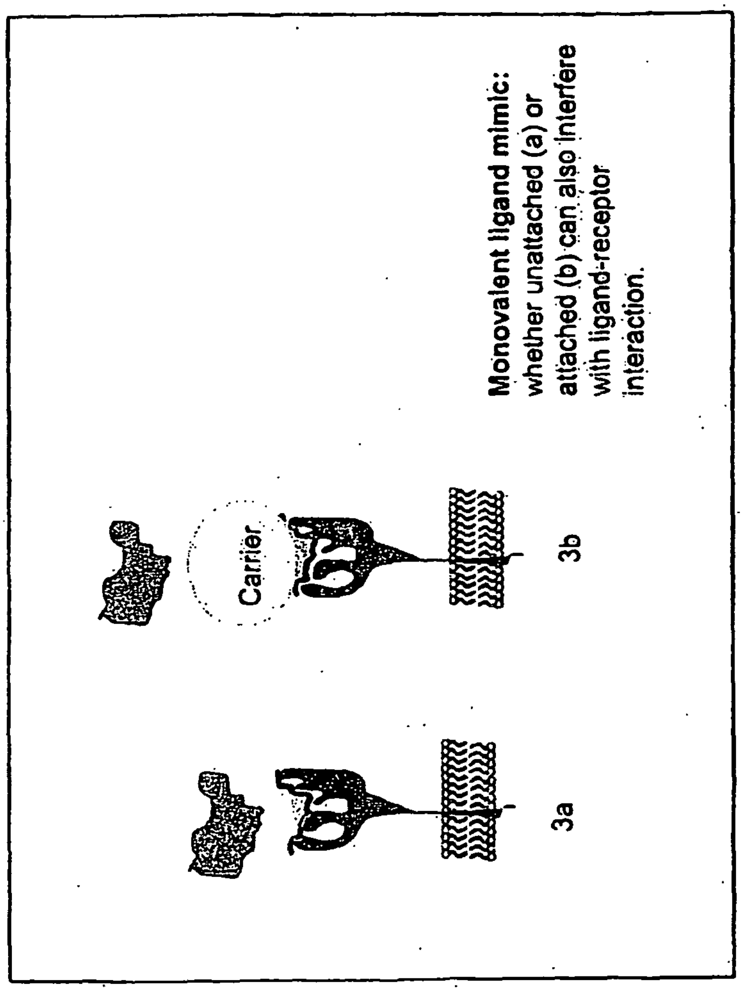


Figure 16a-16b

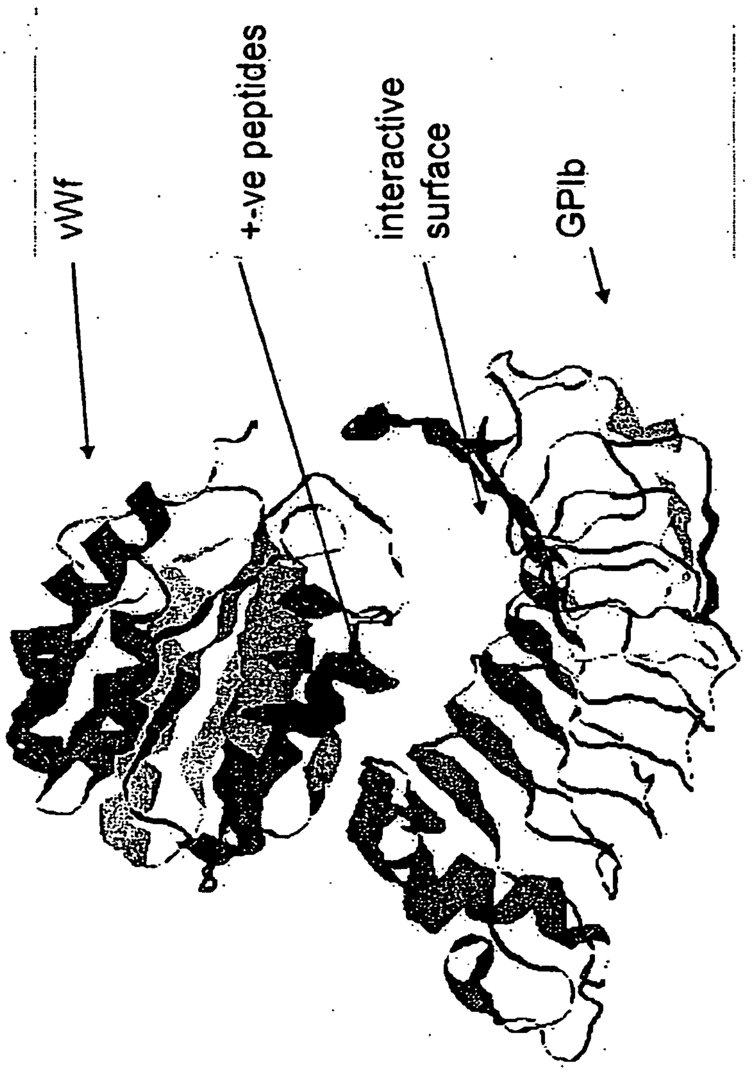


Figure 17

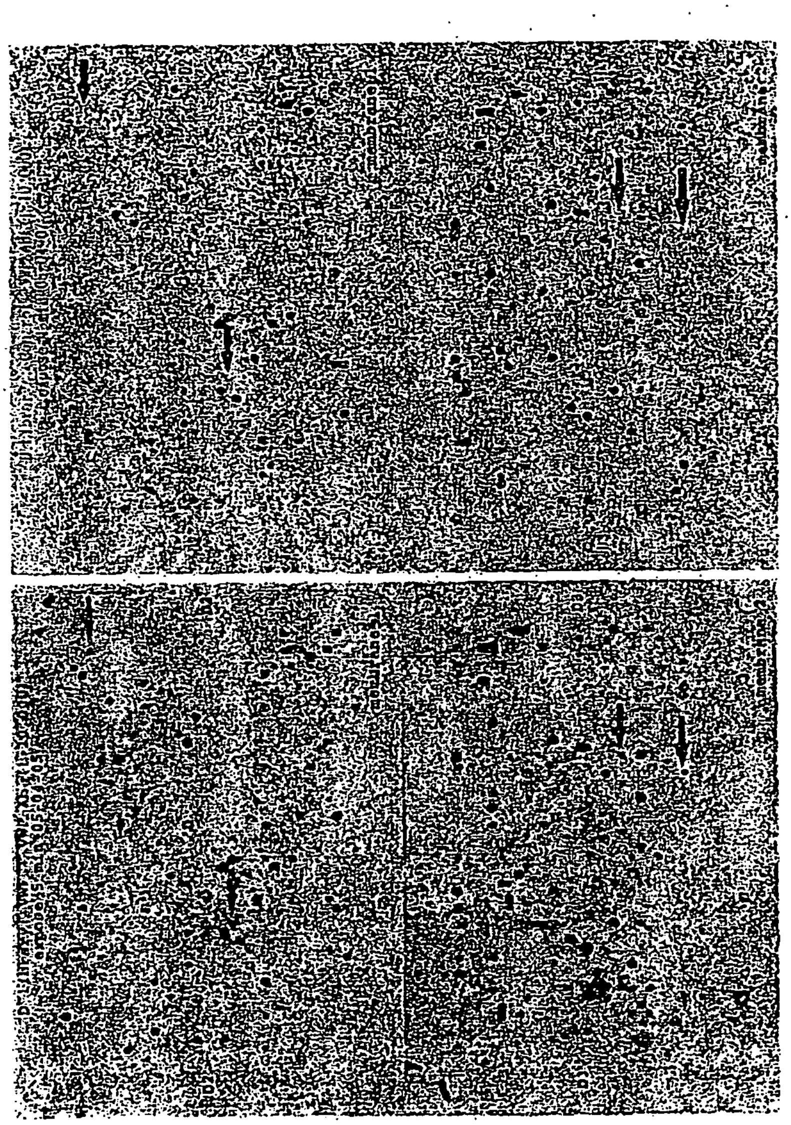

Figure 18

**Interaction between D-peptide 3 with 1vWf**

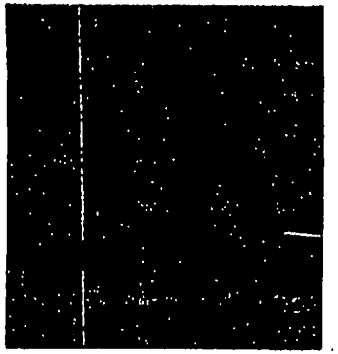

*Energy: 4328.53 Kcal/mol*

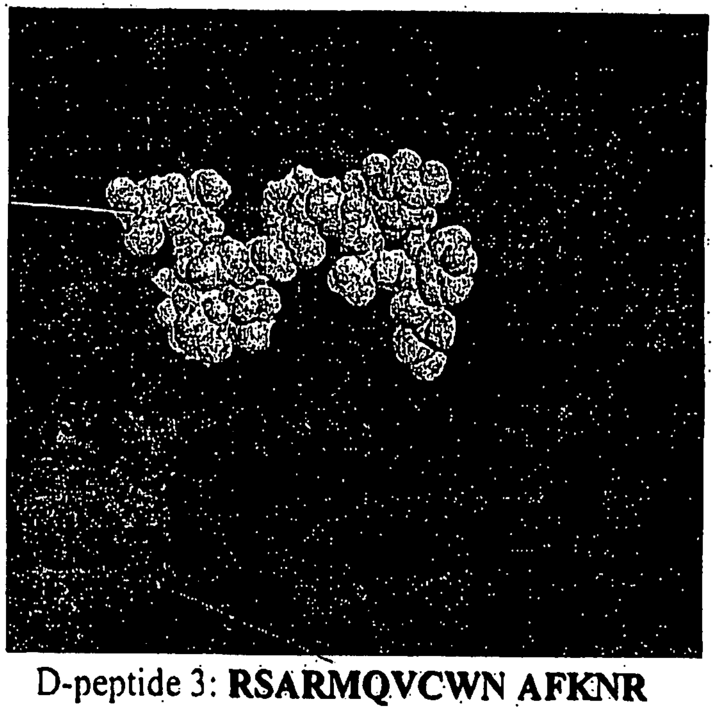

D-peptide 3: **RSARMQVCWN AFKNR**

Figure 19

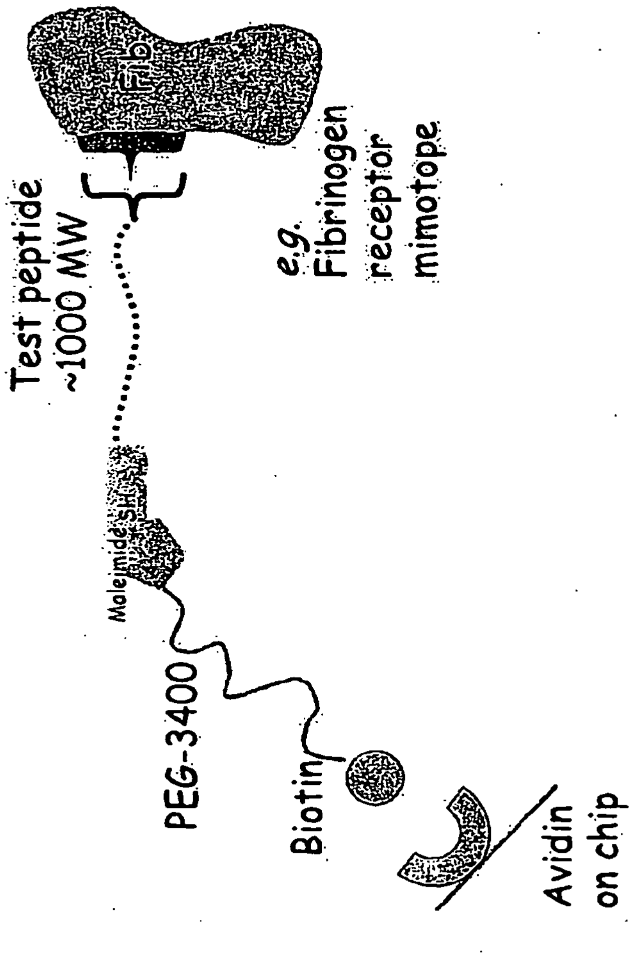
Test peptide
~1000 MW
Fib
e.g.
Fibrinogen
receptor
mimotope
Maleimide
SH
PEG-3400
Biotin
Avidin
on chip

Figure 20

# Langmuir binding analysis

Peptide 1: Fibrinogen Dose-response

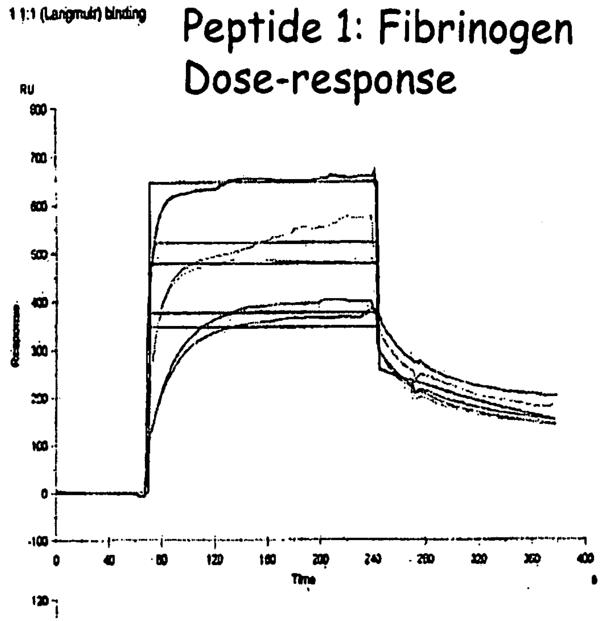


KD (M)1.25e-5

Peptide 2: Fibrinogen Dose-response

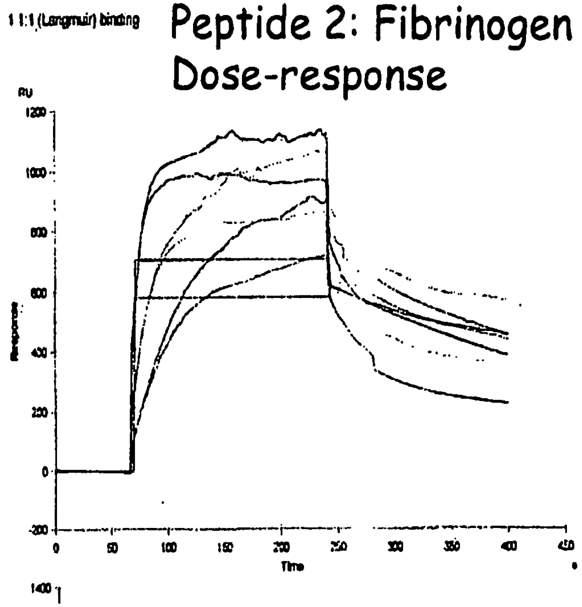


KD (M)3.34e-7

GPIIbIIIa mimotopes

GPIIbIIIa -Fibrinogen = KD (M) 1.03e-8

**Figure 21**

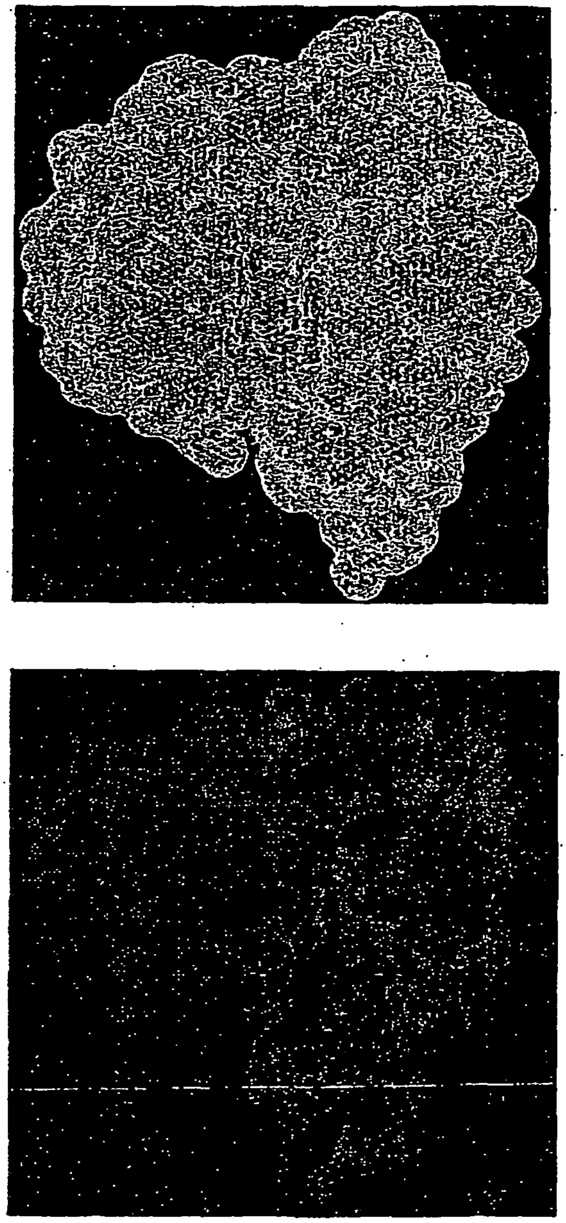

b)

a)

Figure 22a-22b

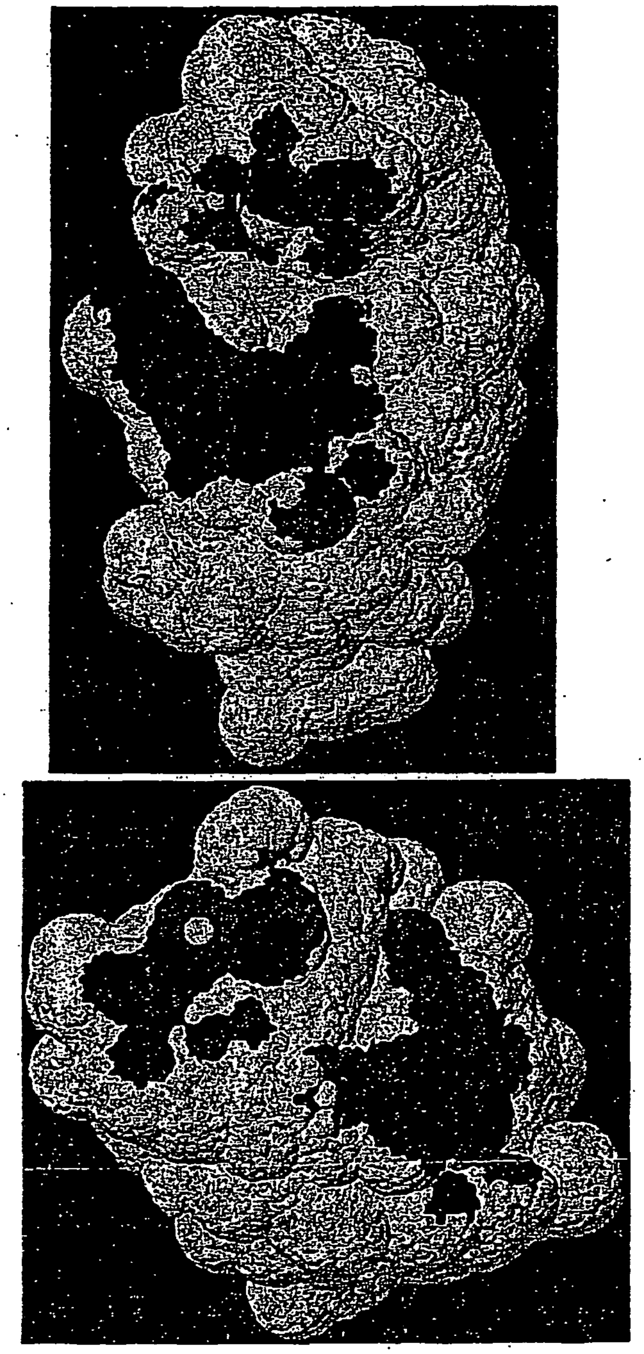

b)

a)

Figure 23a-23b

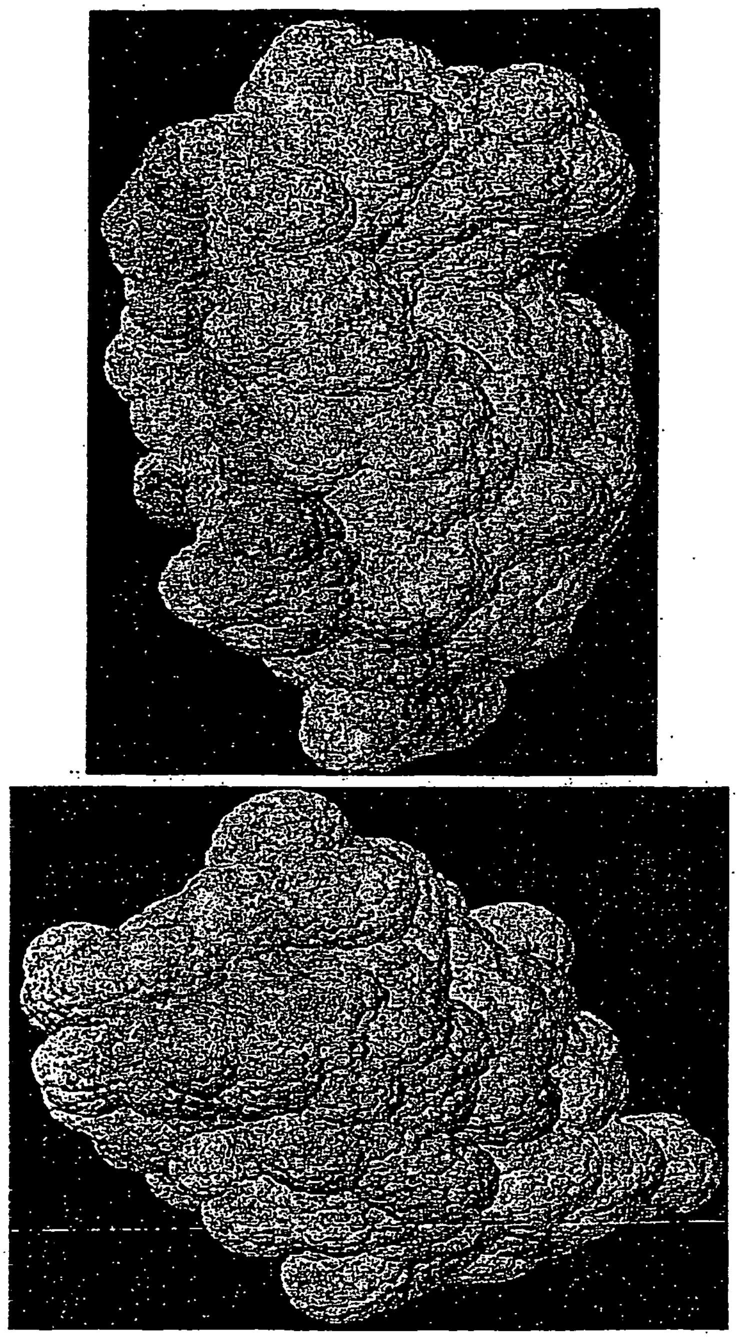

a)

b)

Figure 24a-24b

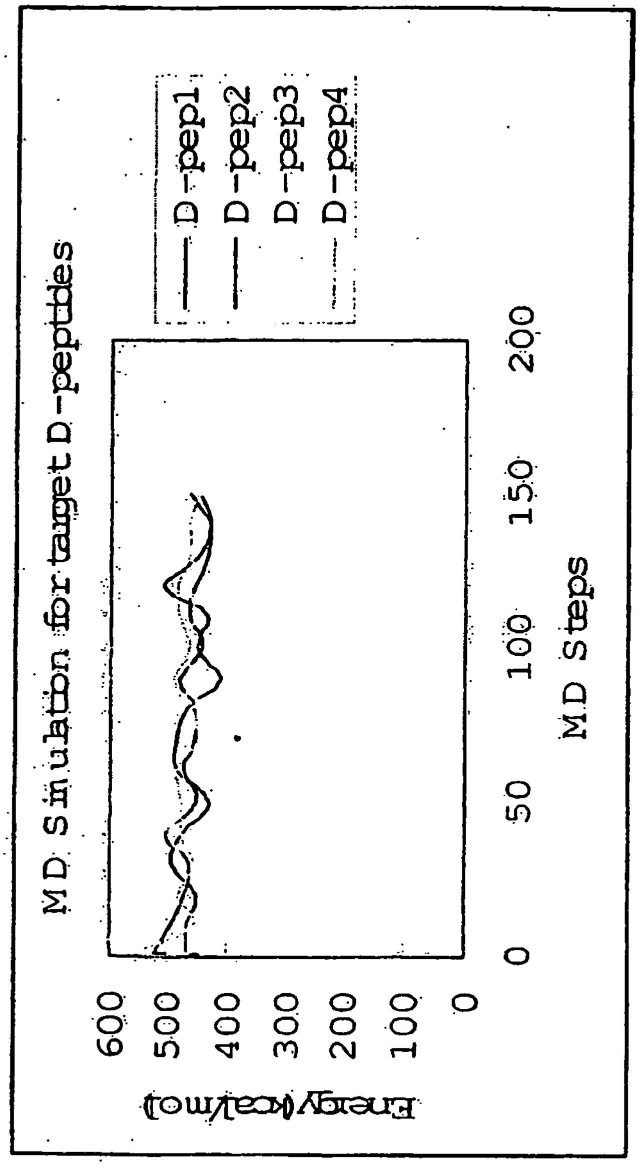
MD Simulation for target D-peptides
Energy (kcal/mol)
600
500
400
300
200
100
0
0
50
100
150
200
MD Steps
D-pep1
D-pep2
D-pep3
D-pep4

Figure 25

Figure 26 a

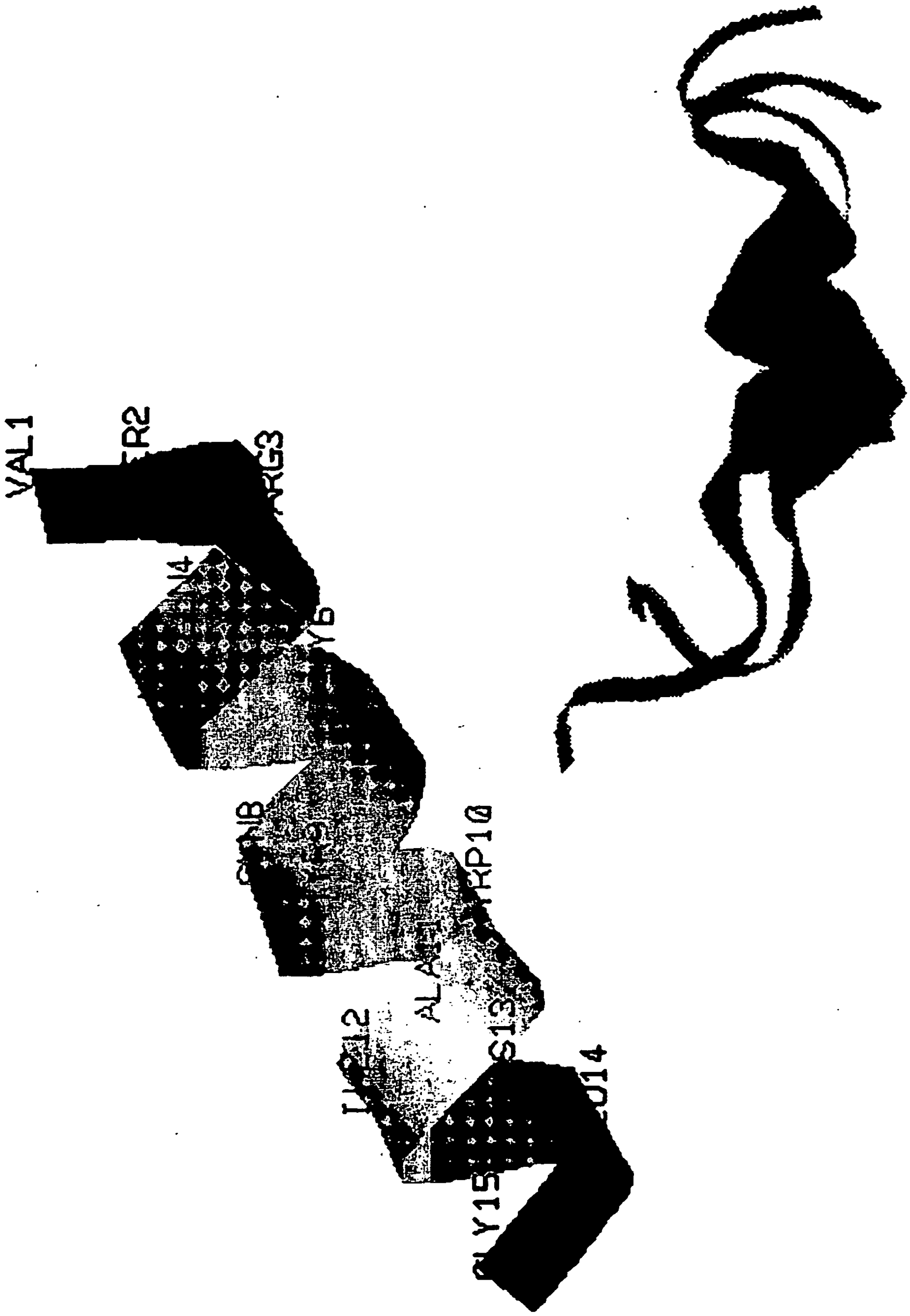

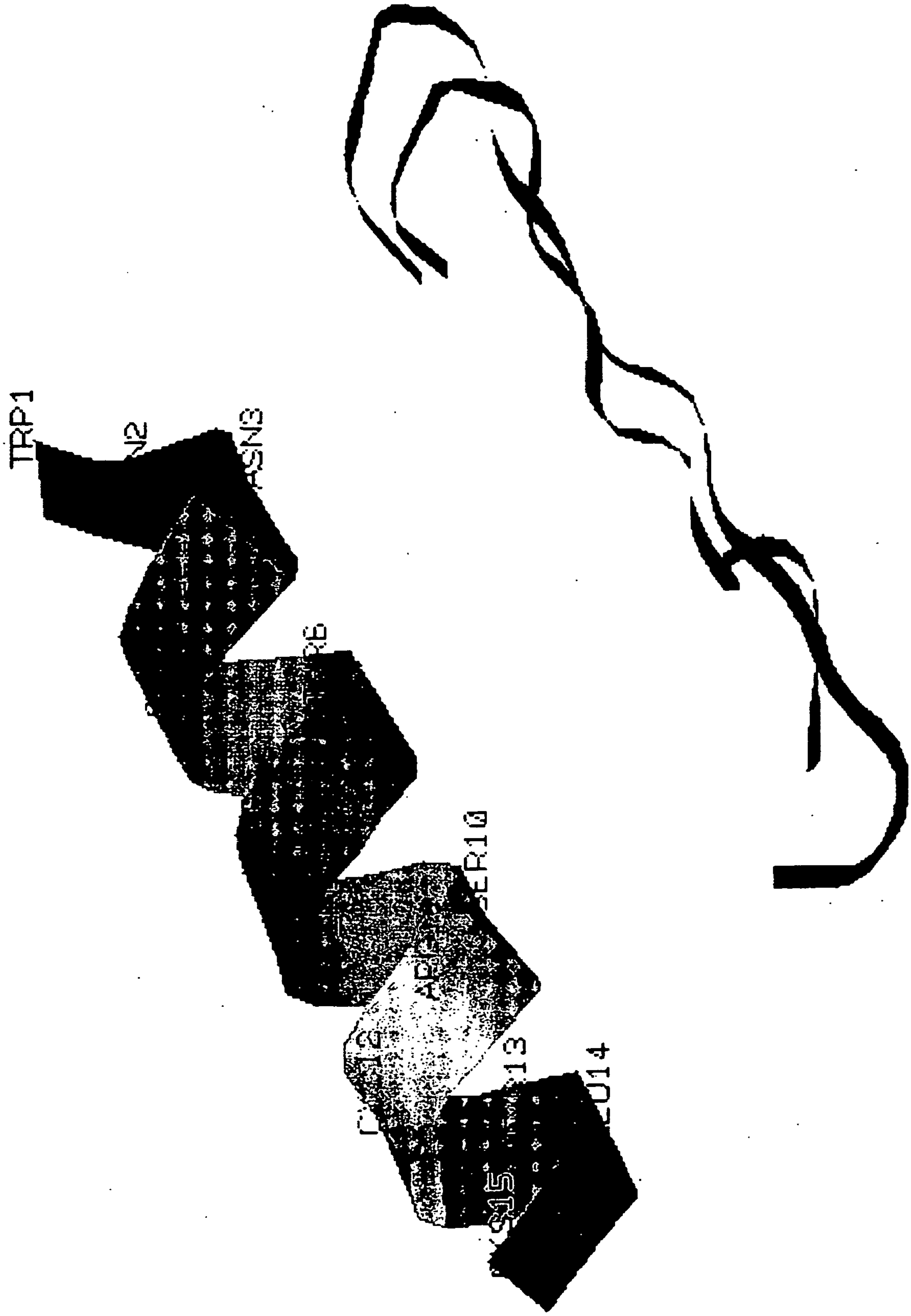


Figure 26 b

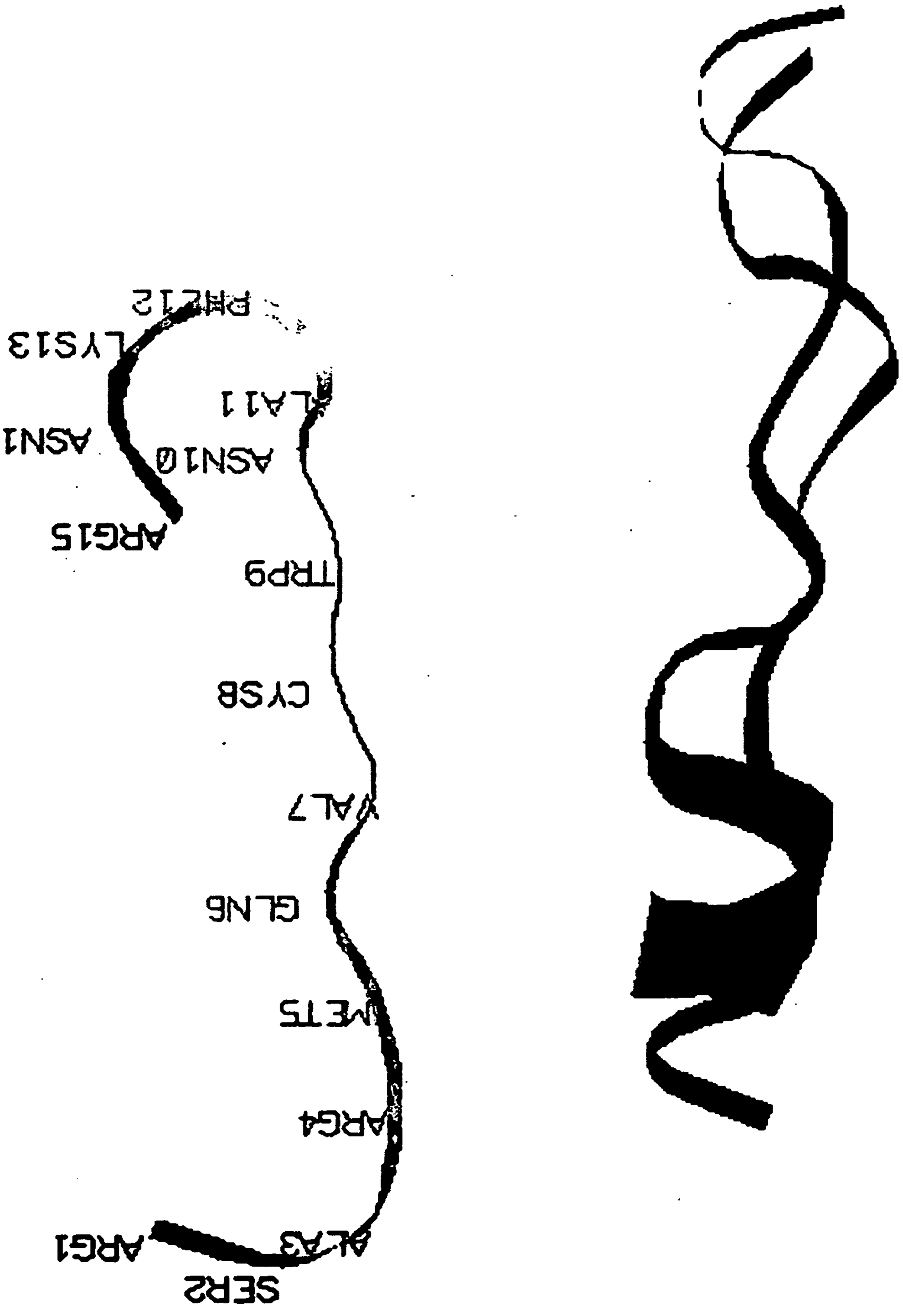

Figure 26 c

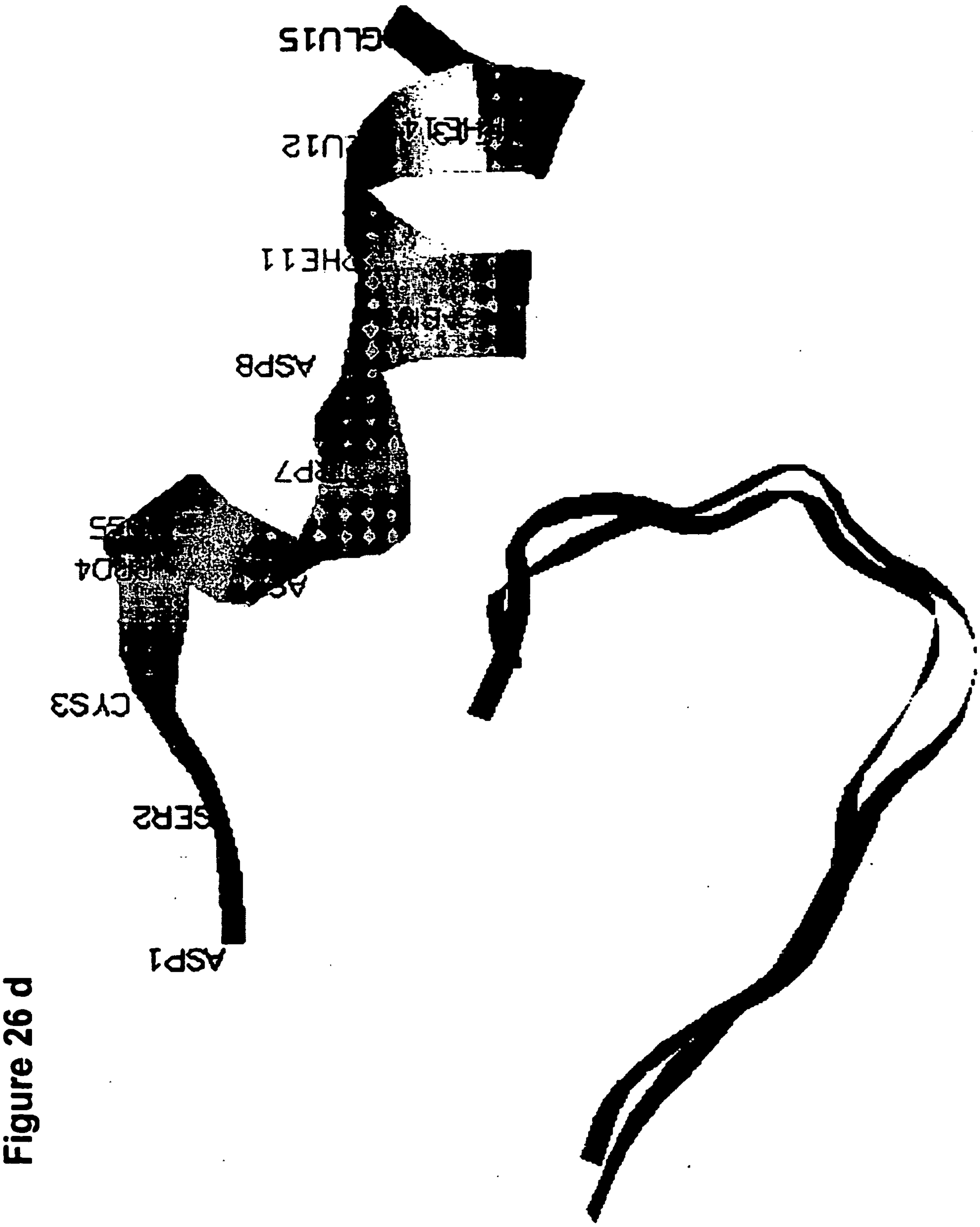


Figure 26 d

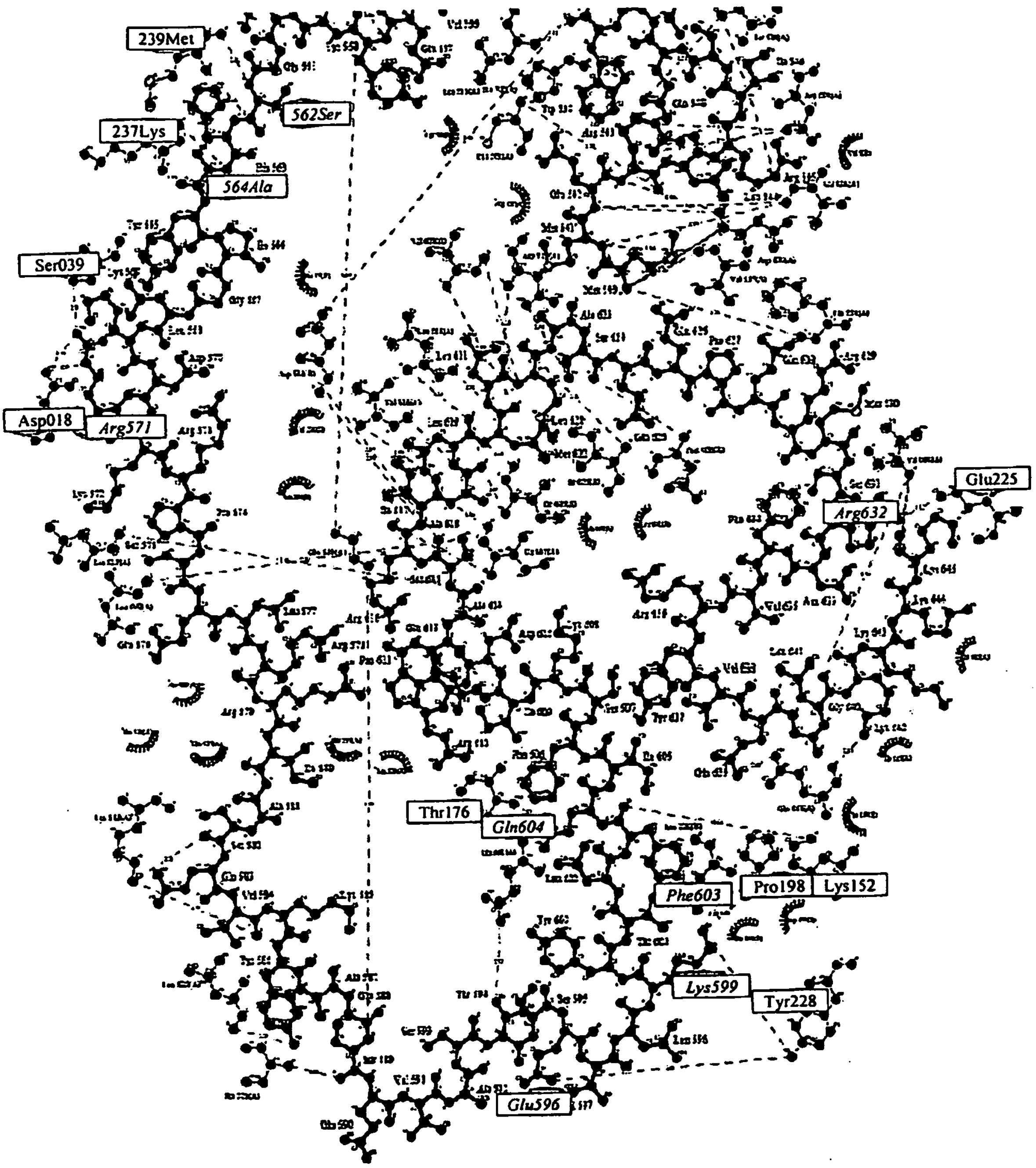
239Met
562Ser
237Lys
564Ala
Ser039
Asp018
Arg571
Glu225
Arg632
Thr176
Gln604
Phe603
Pro198
Lys152
Lys599
Tyr228
Glu596

**Figure 27**

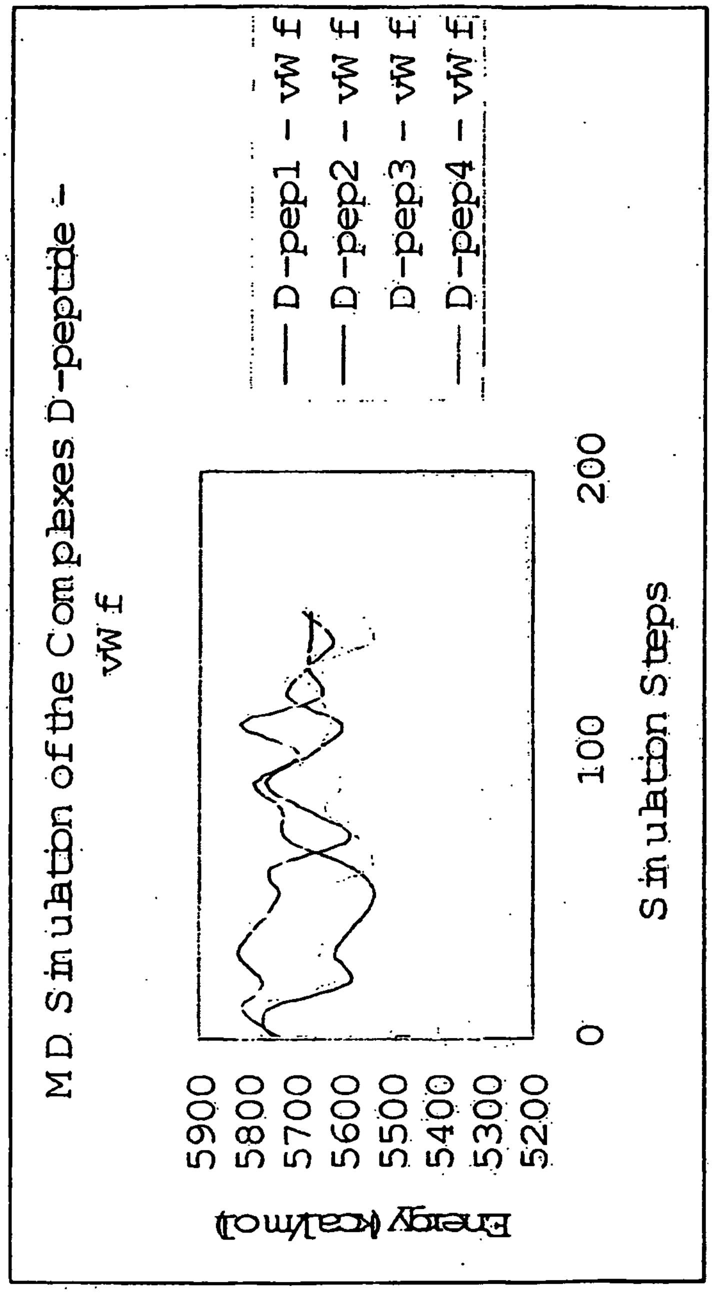
MD Simulation of the Complexes D-peptide - vWf
Energy (kcal/mol)
5900
5800
5700
5600
5500
5400
5300
5200
0
100
200
Simulation Steps
D-pep1 - vWf
D-pep2 - vWf
D-pep3 - vWf
D-pep4 - vWf

Figure 28

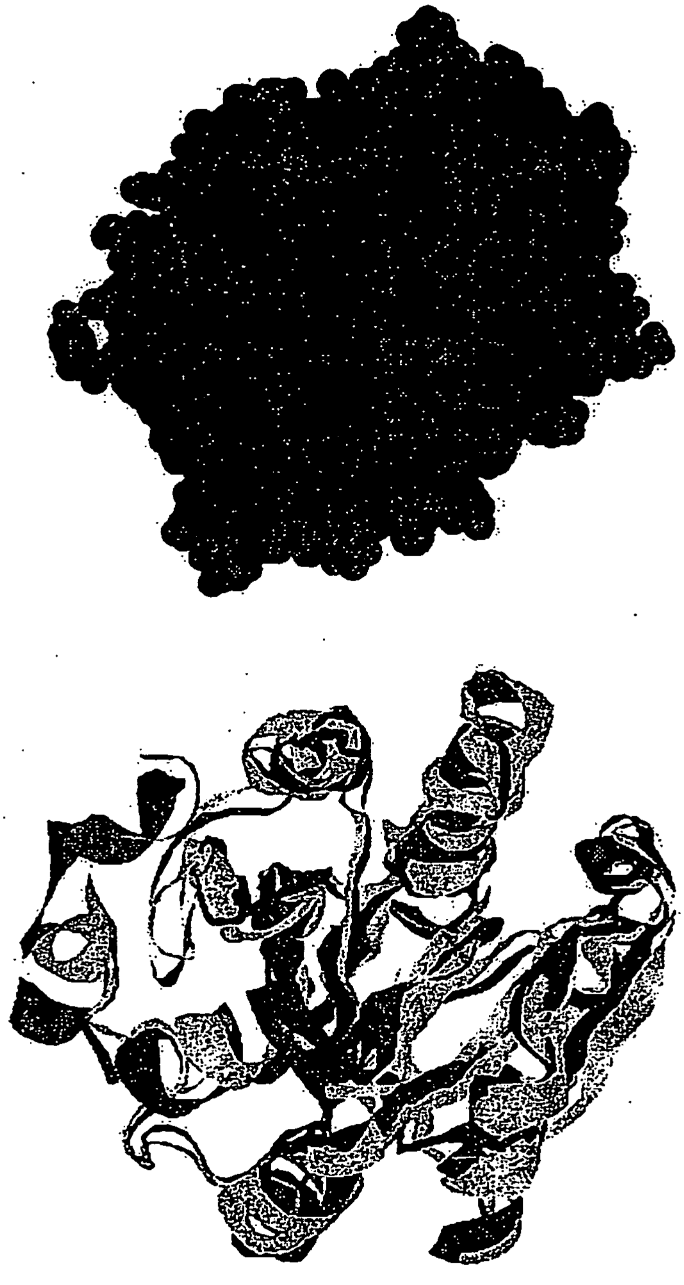

a)

b)

Figure 29a-29b

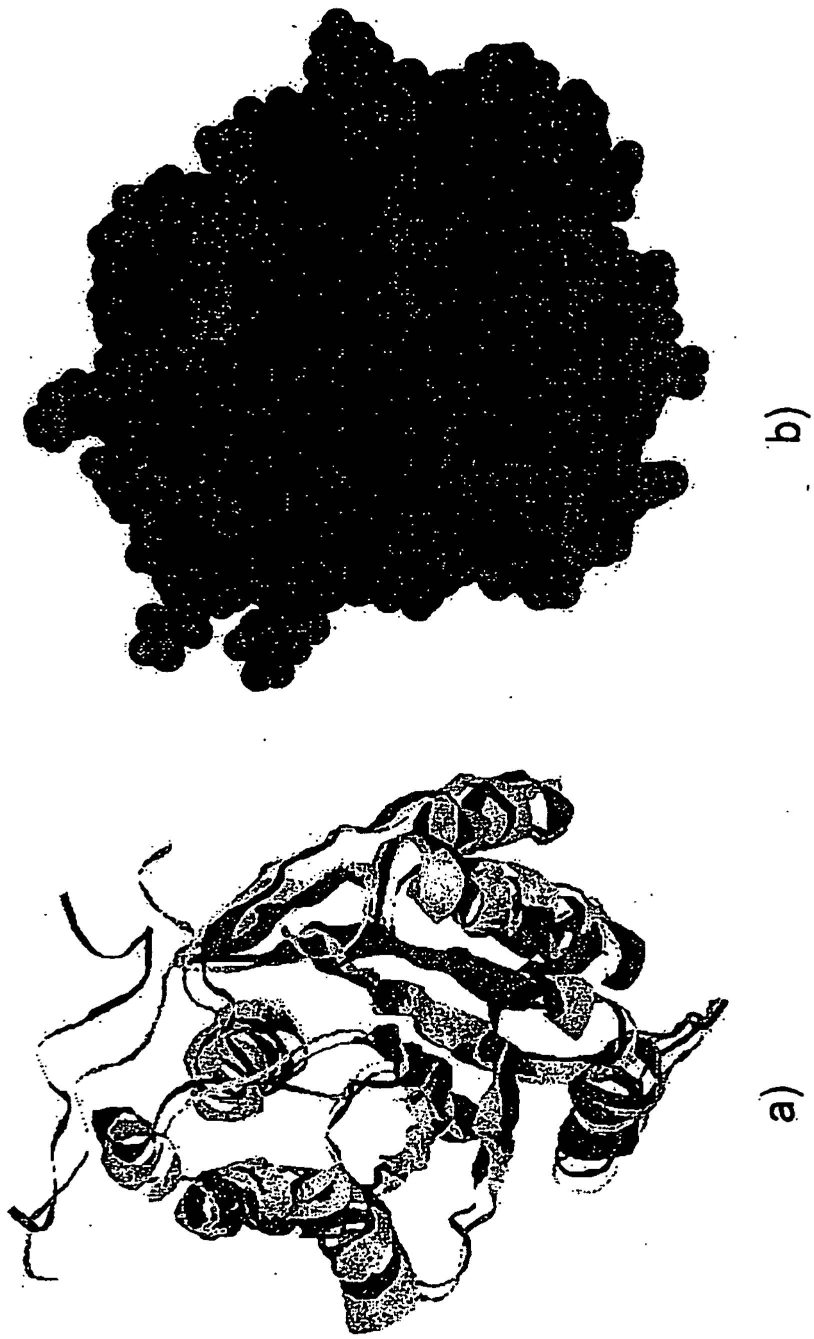


Figure 30a-30b

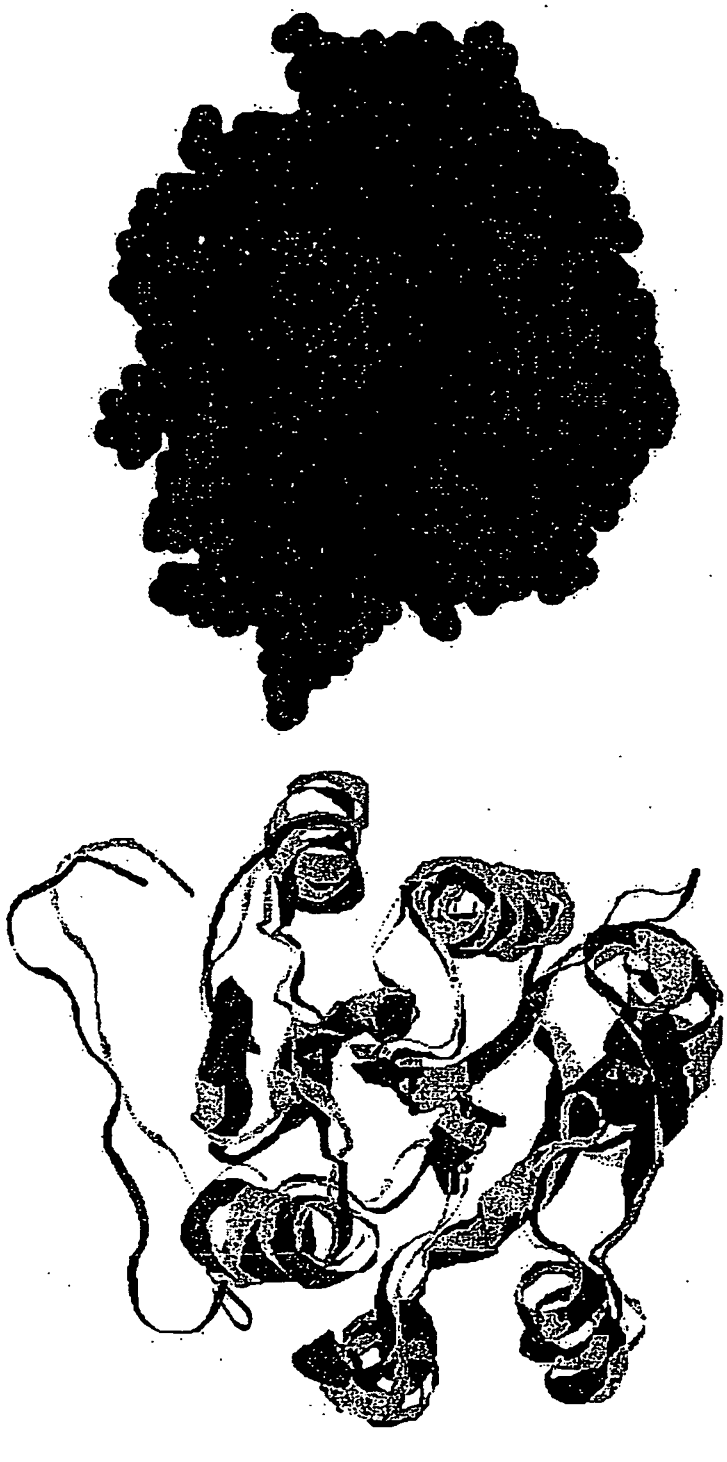

b)

a)

Figure 31a-31b

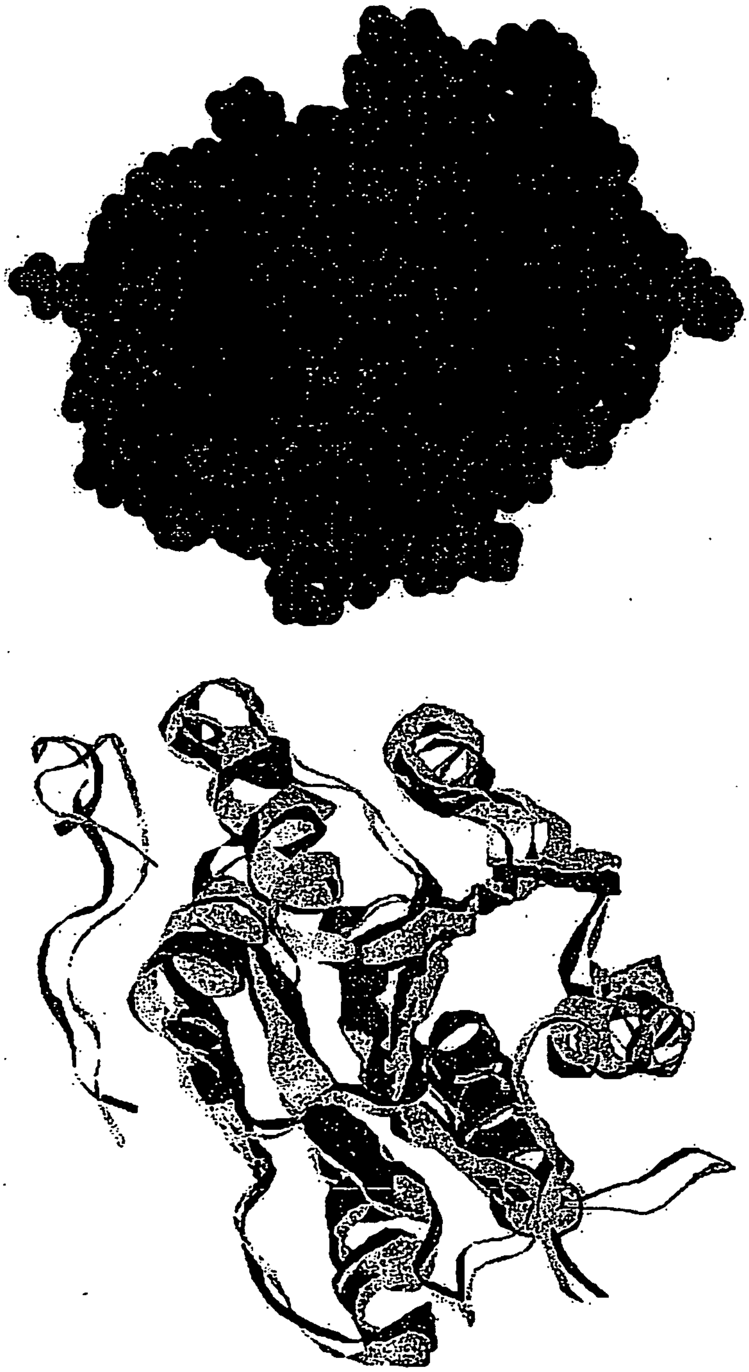

a) b)

Figure 32a-32b

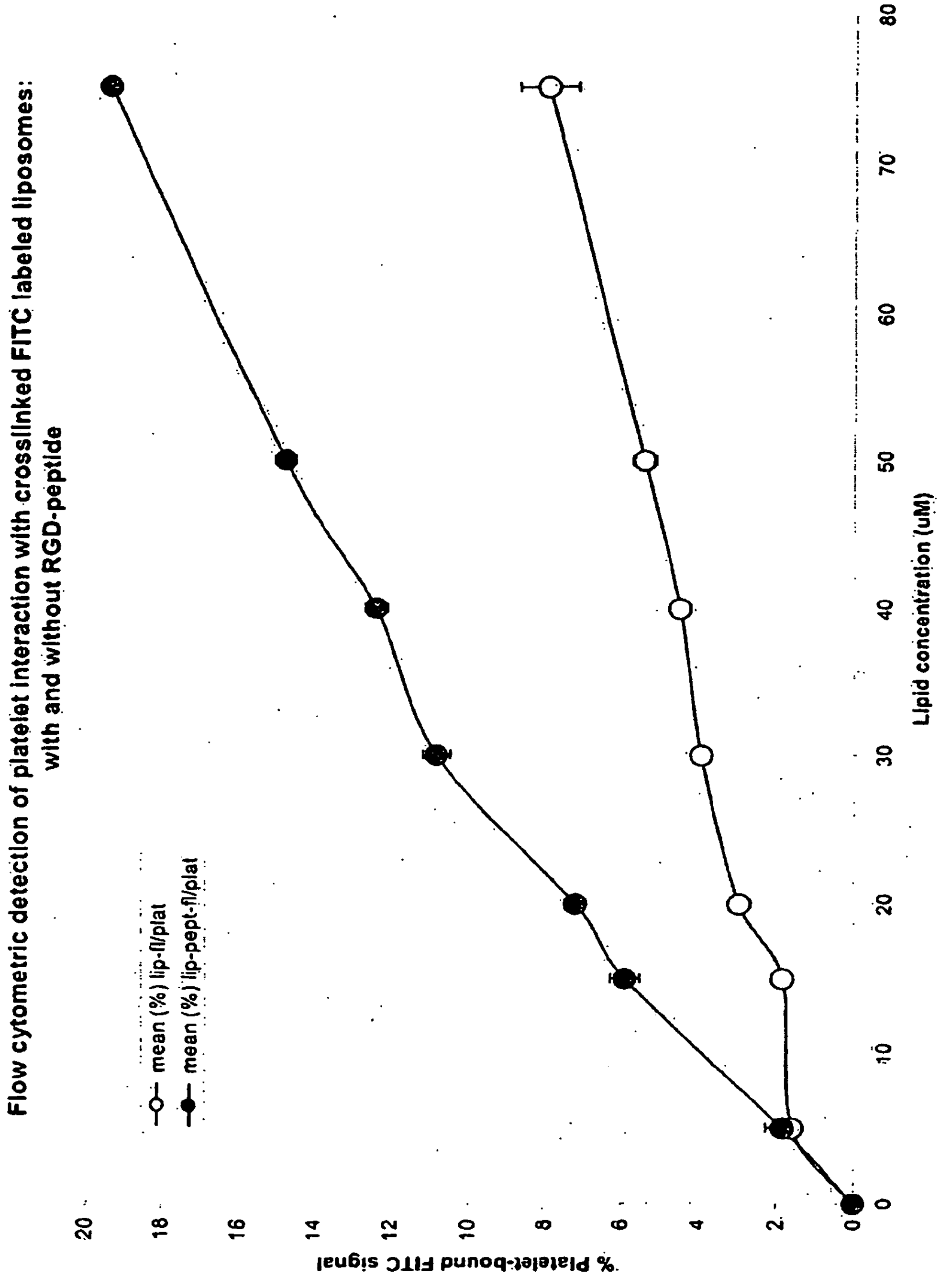
Flow cytometric detection of platelet interaction with crosslinked FITC labeled liposomes:
with and without RGD-peptide
mean (%) lip-fl/plat
mean (%) lip-pept-fl/plat
% Platelet-bound FITC signal
0
2
4
6
8
10
12
14
16
18
20
0
10
20
30
40
50
60
70
80
Lipid concentration (uM)

Figure 33

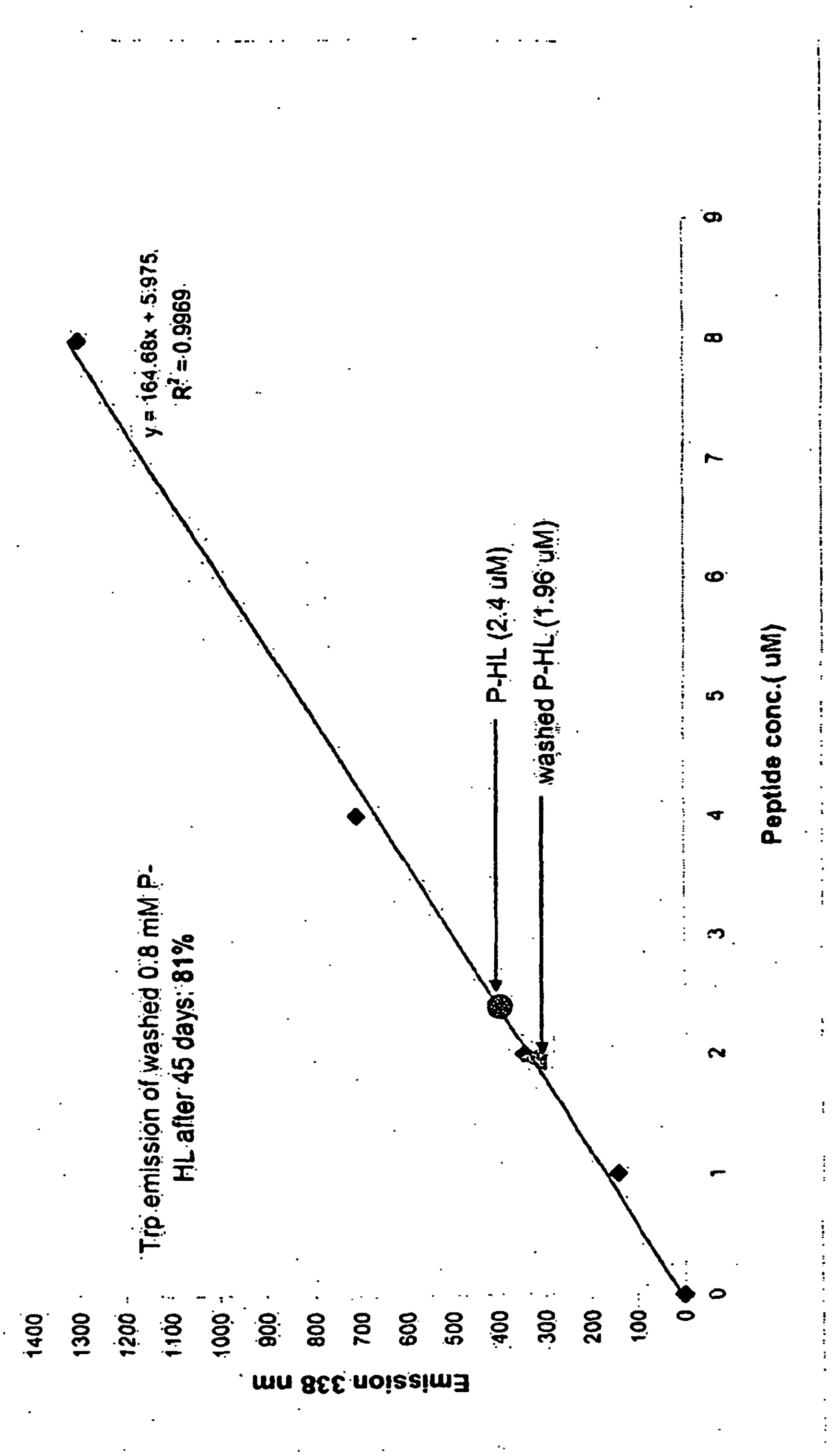
Trp emission of washed 0.8 mM P-HL after 45 days: 81%
y = 164.68x + 5.975
$R^2$ = 0.9969
P-HL (2.4 uM)
washed P-HL (1.96 uM)
Emission 338 nm
0
100
200
300
400
500
600
700
800
900
1000
1100
1200
1300
1400
Peptide conc.( uM)
0
1
2
3
4
5
6
7
8
9

Figure 34

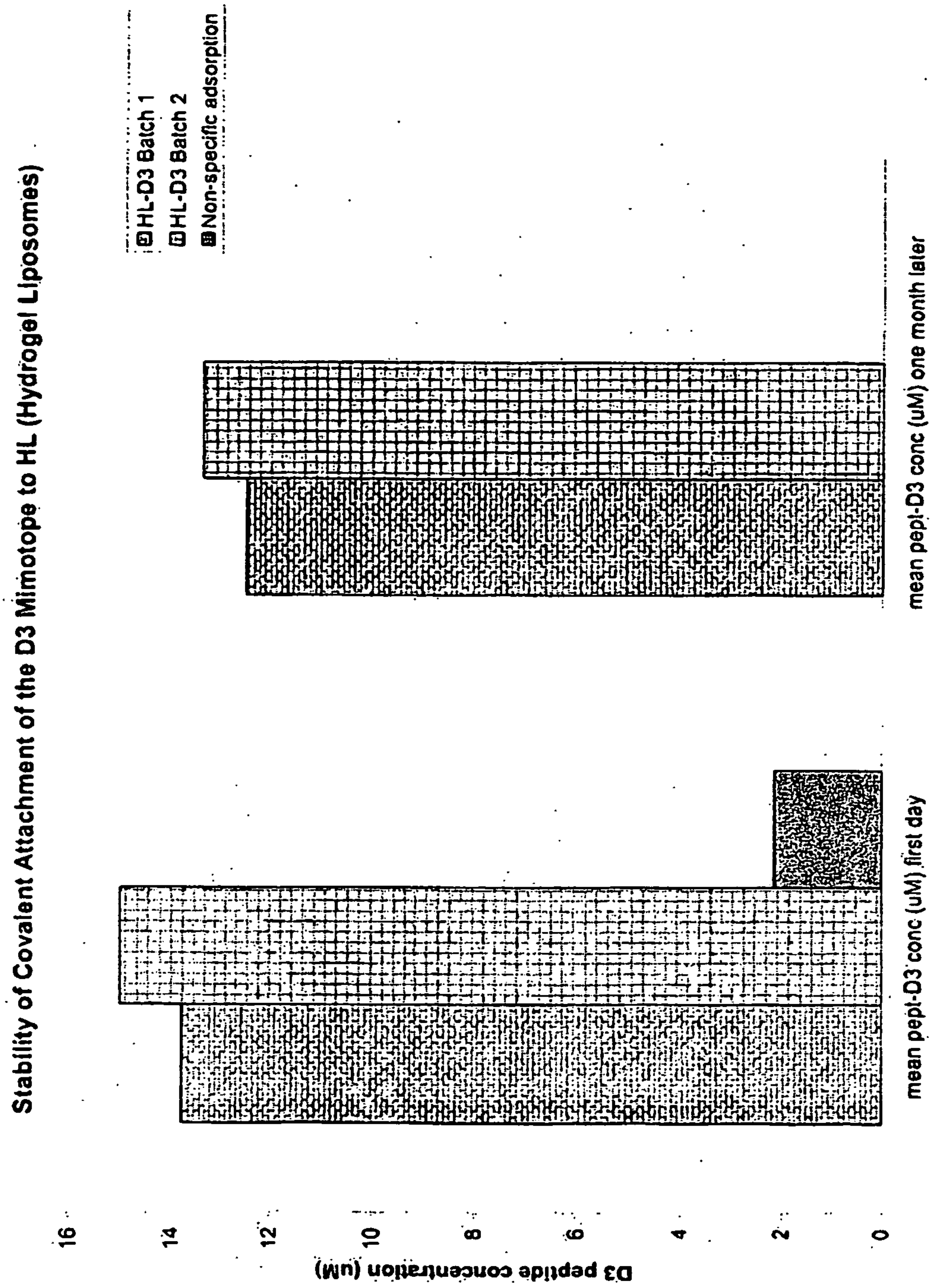

Stability of Covalent Attachment of the D3 Mimotope to HL (Hydrogel Liposomes)
HL-D3 Batch 1
HL-D3 Batch 2
Non-specific adsorption
D3 peptide concentration (uM)
16
14
12
10
8
6
4
2
0
mean pept-D3 conc (uM) first day
mean pept-D3 conc (uM) one month later


Figure 35

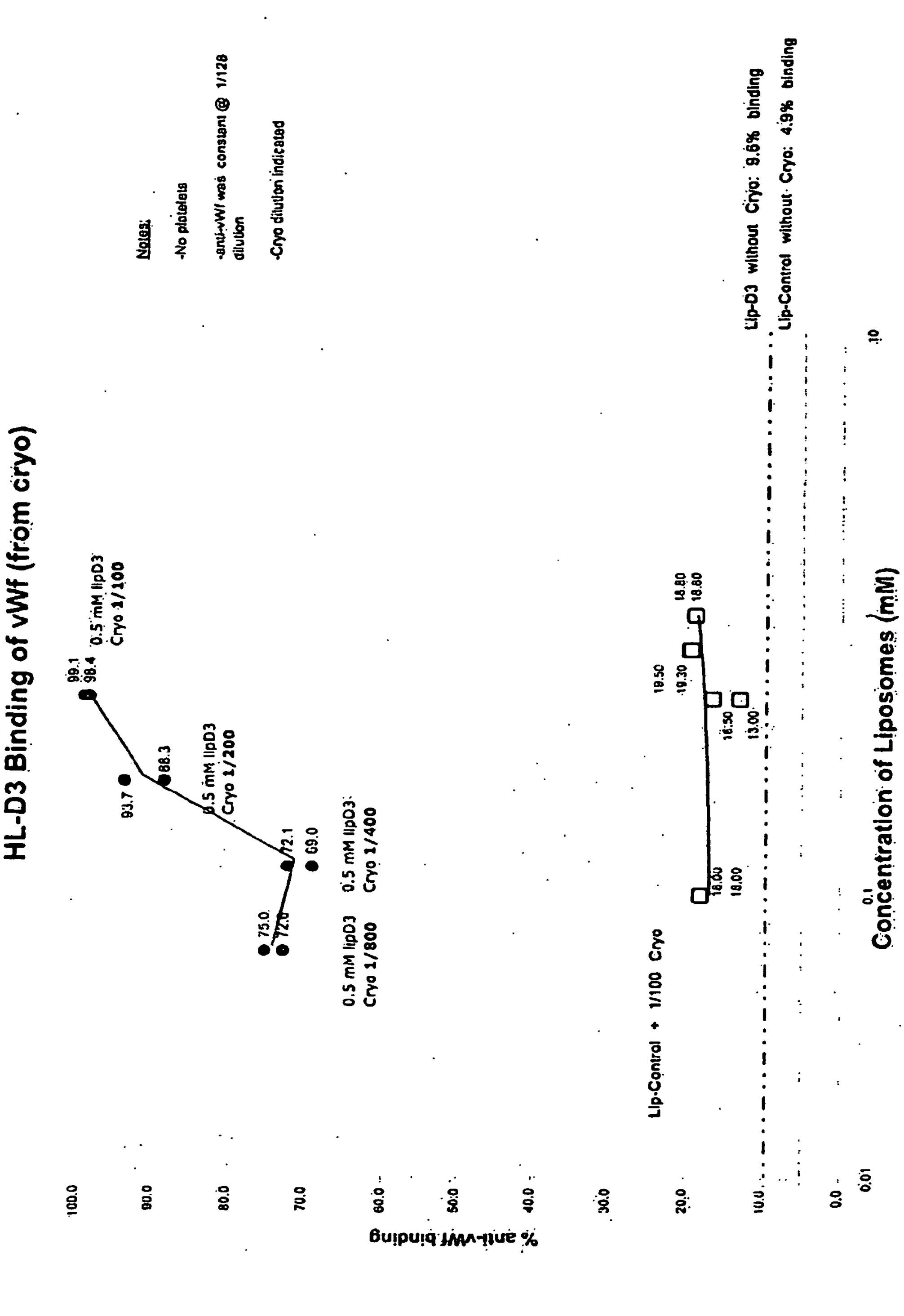
HL-D3 Binding of vWf (from cryo)
% anti-vWf binding
100.0
90.0
80.0
70.0
60.0
50.0
40.0
30.0
20.0
10.0
0.0
99.1
98.4
0.5 mM lipD3
Cryo 1/100
93.7
88.3
0.5 mM lipD3
Cryo 1/200
72.1
69.0
0.5 mM lipD3
Cryo 1/400
75.0
72.0
0.5 mM lipD3
Cryo 1/800
Lip-Control + 1/100 Cryo
19.50
19.30
18.80
18.80
18.50
13.00
18.00
18.00
Lip-D3 without Cryo: 9.6% binding
Lip-Control without Cryo: 4.9% binding
Notes:
-No platelets
-anti-vWf was constant @ 1/128 dilution
-Cryo dilution indicated
0.01
0.1
10
Concentration of Liposomes (mM)

Figure 36

Platelets / Cd42-PE/ anti vWf

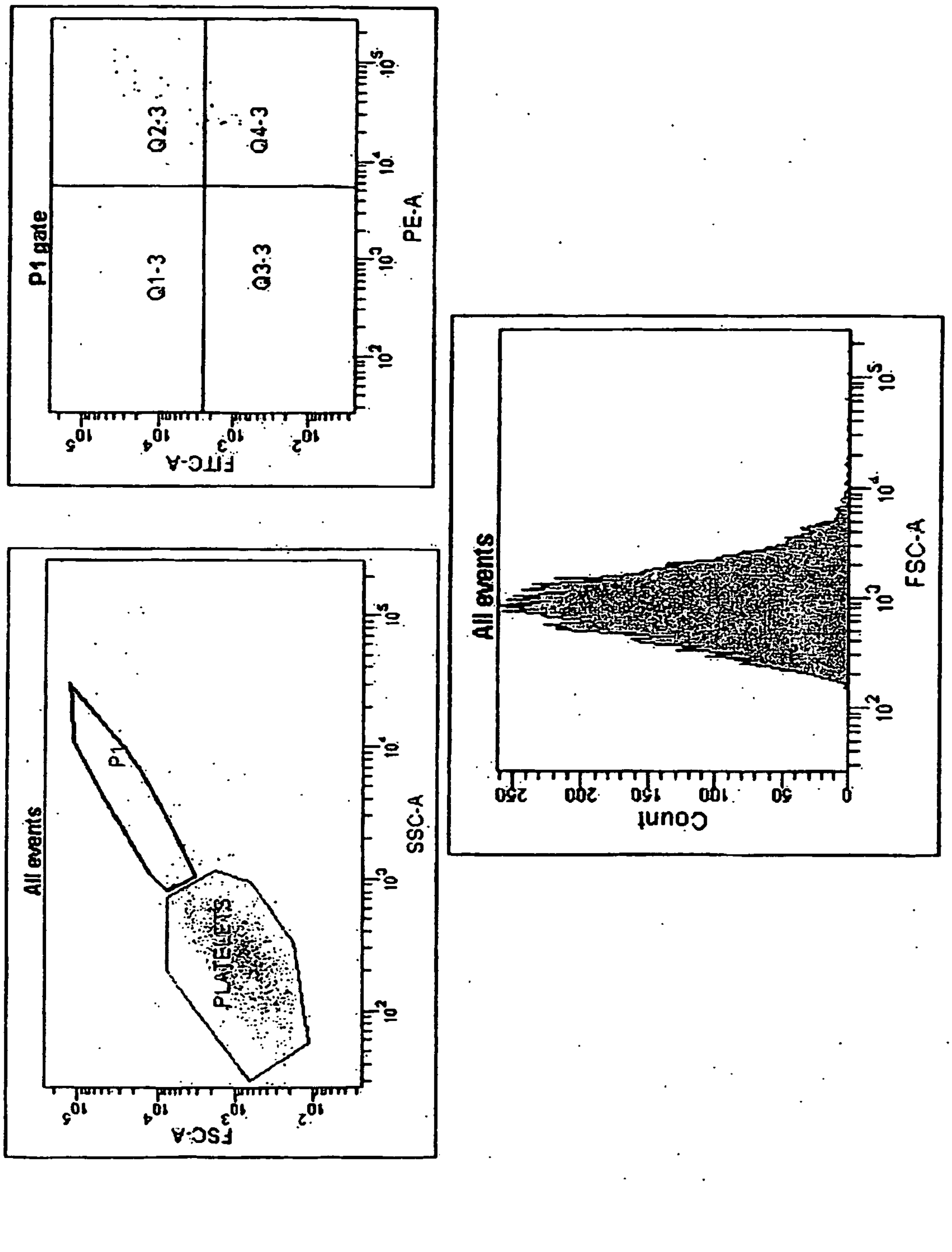


Figure 37a

Liposomes-control / Cd42-PE/ anti vWf

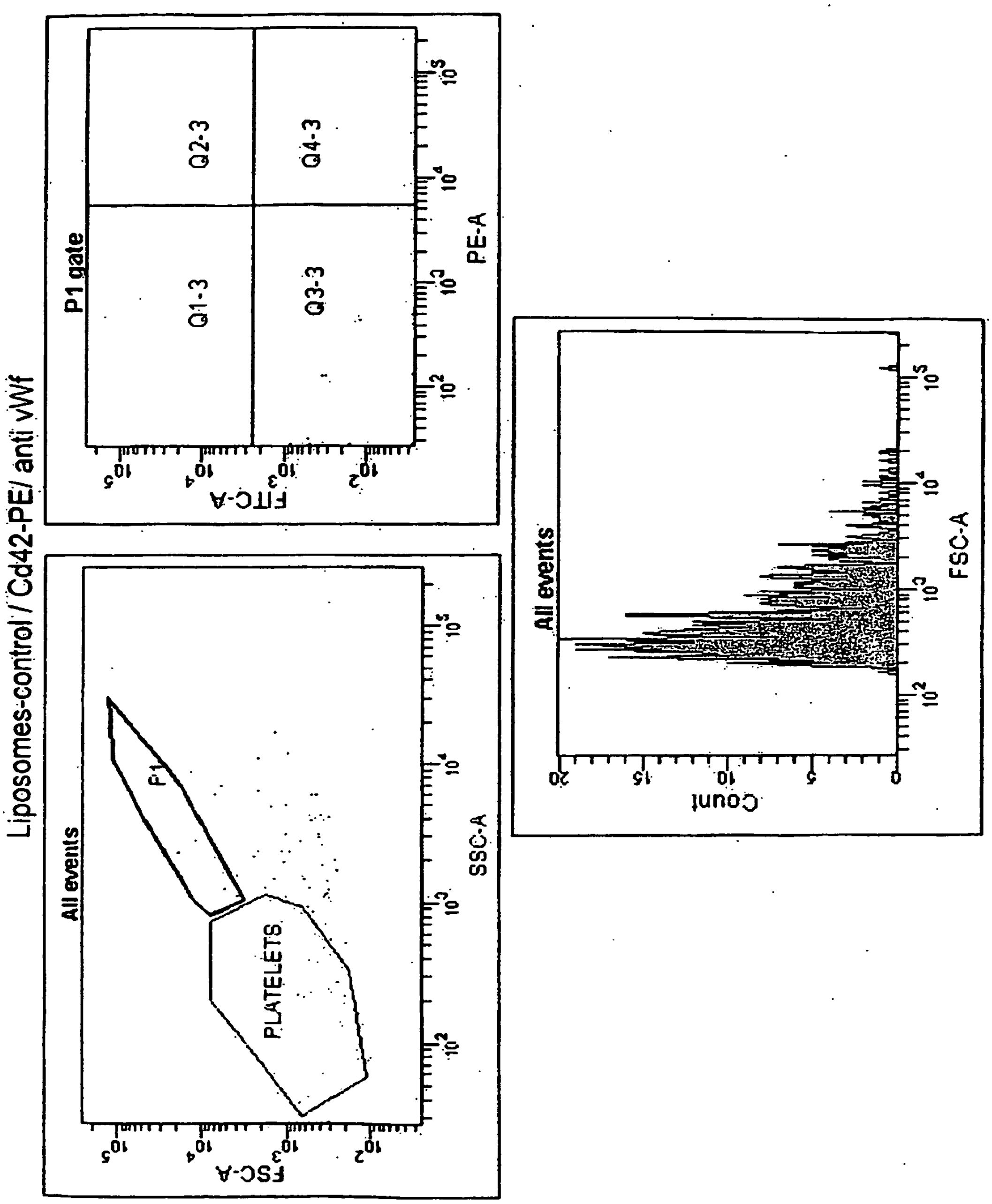


Figure 37b

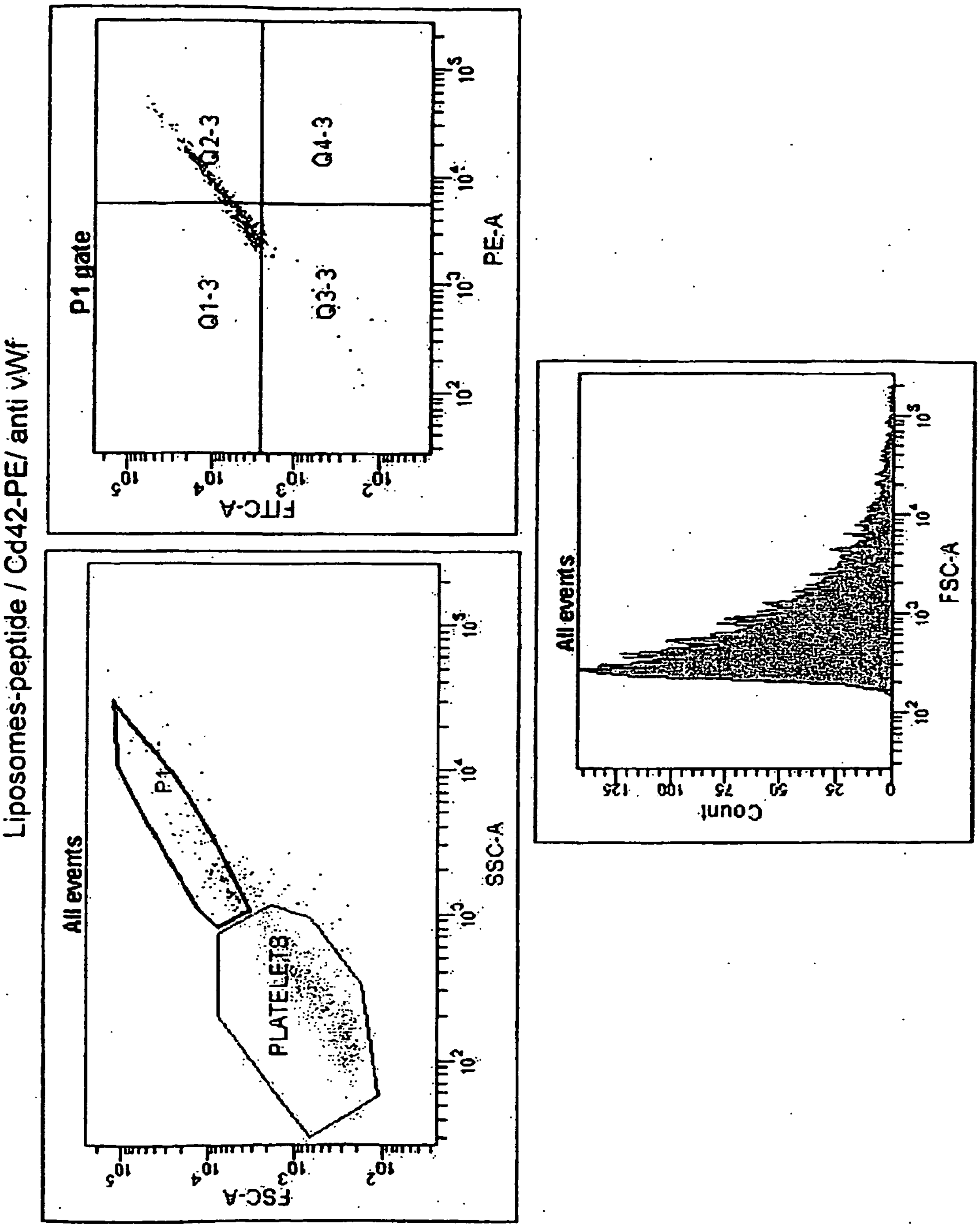
Liposomes-peptide / Cd42-PE/ anti vWf
All events
P1
PLATELETS
FSC-A
SSC-A
P1 gate
Q1-3
Q2-3
Q3-3
Q4-3
FITC-A
PE-A
All events
Count
FSC-A

Figure 37c

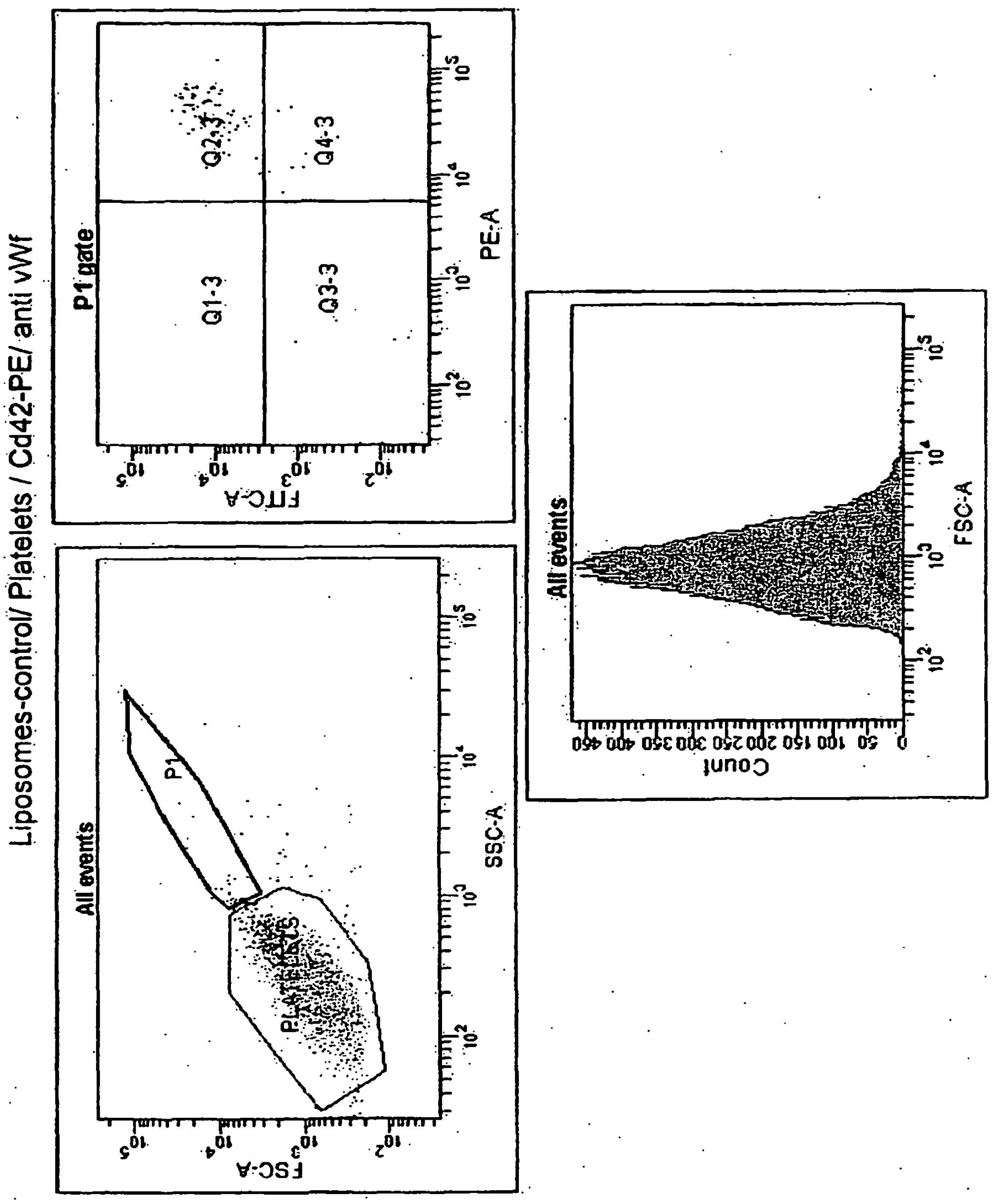
Liposomes-control/ Platelets / Cd42-PE/ anti vWf
All events
P1
PLATELETS
FSC-A
SSC-A
P1 gate
Q1-3
Q2-3
Q3-3
Q4-3
FITC-A
PE-A
All events
Count
FSC-A

**Figure 37d**

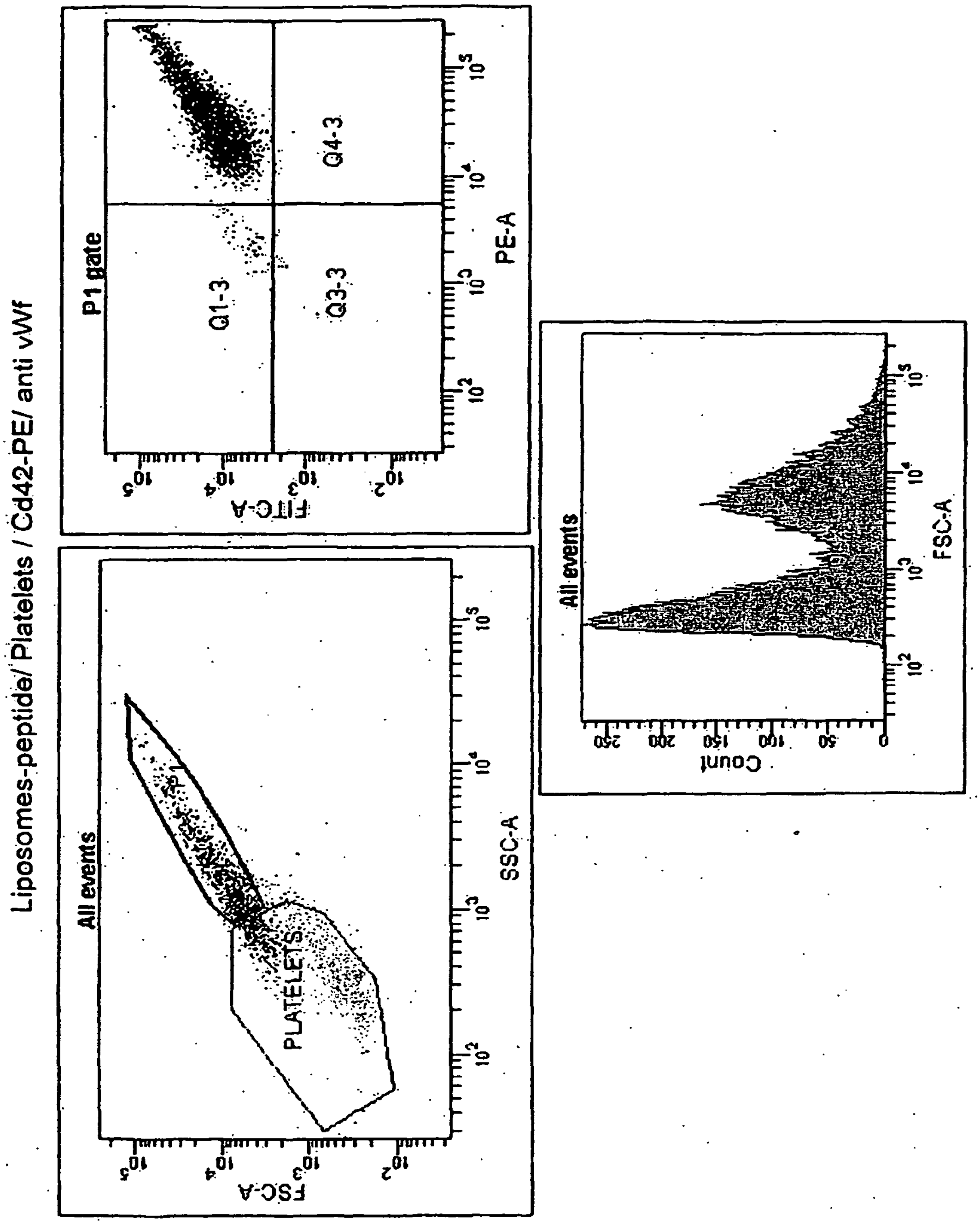
Liposomes-peptide/ Platelets / Cd42-PE/ anti vWf
All events
PLATELETS
P1
FSC-A
SSC-A
P1 gate
Q1-3
Q3-3
Q4-3
FITC-A
PE-A
All events
Count
FSC-A

Figure 37e

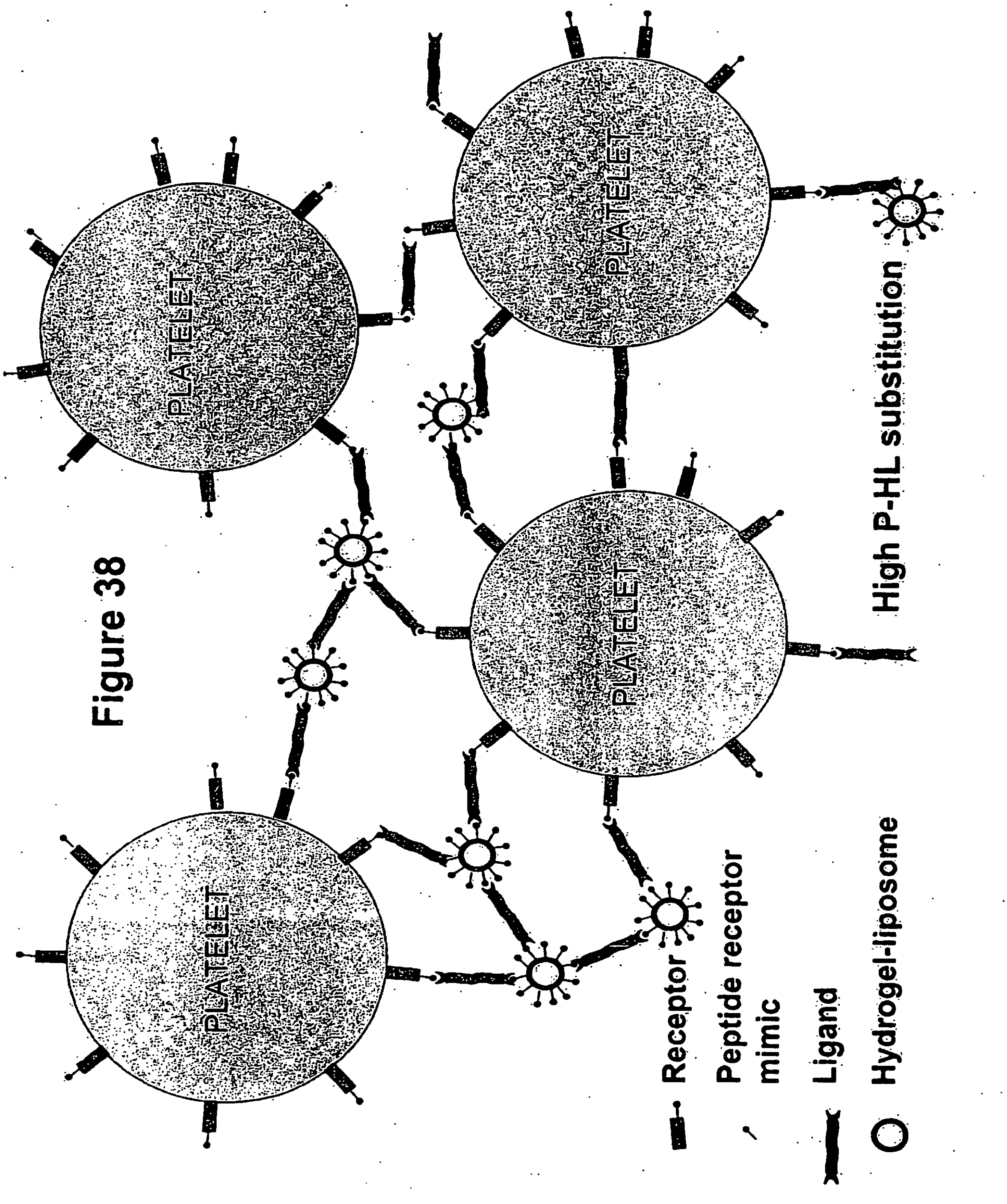

PLATELET
PLATELET
PLATELET
PLATELET
Receptor
Peptide receptor mimic
Ligand
Hydrogel-liposome
High P-HL substitution


Figure 38

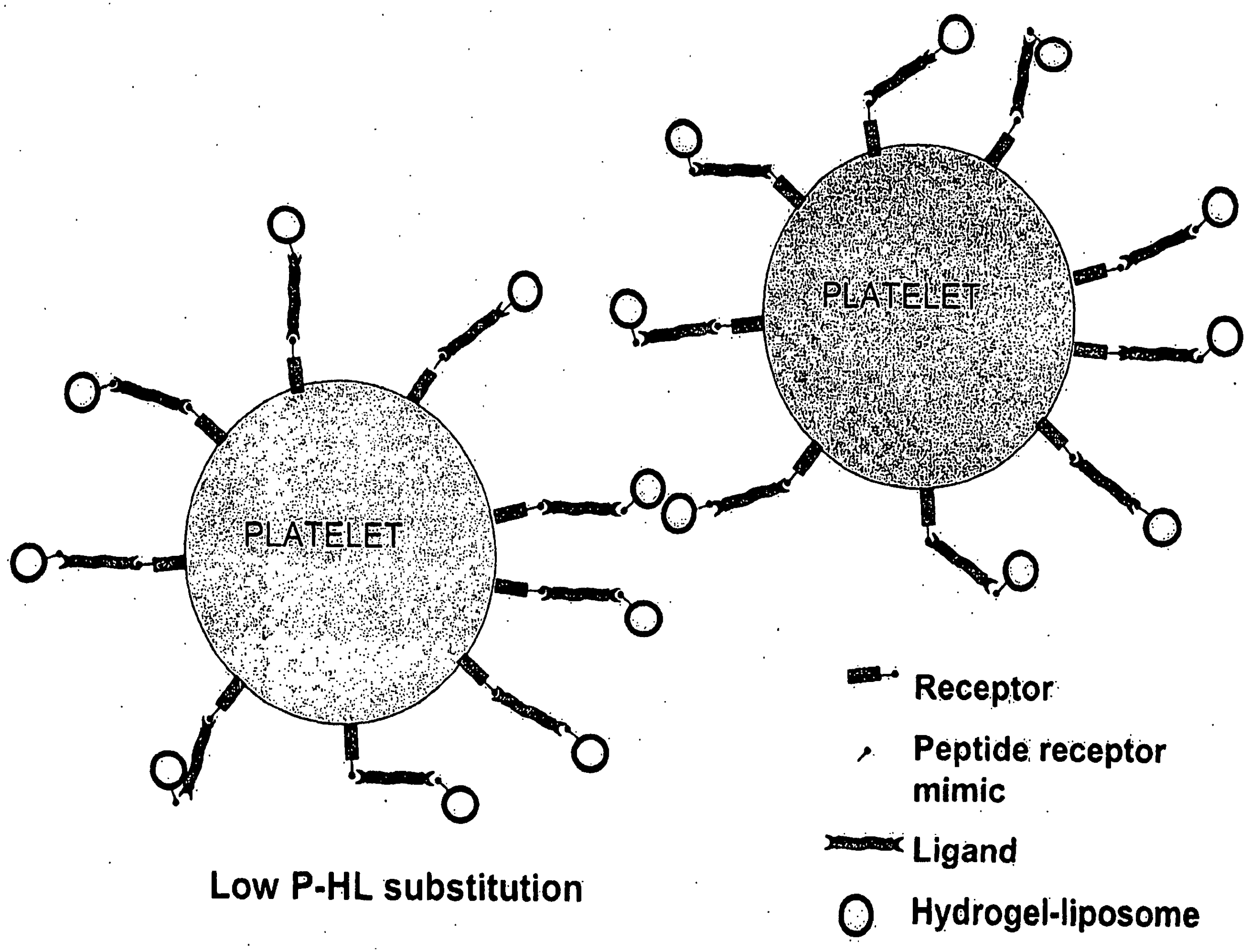
PLATELET
PLATELET
Receptor
Peptide receptor mimic
Ligand
Hydrogel-liposome
Low P-HL substitution

Figure 39

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6958212 B
- US 60842647 B


### Non-patent literature cited in the description


- **Ponder J.A. ; Case DA.** Force fields for protein simulations. *Adv. Prot. Chem.,* 2003, vol. 66, 27-85
- **Richichi A. Percheron 1.** CHARM: A catalog of high angular resolution measurements. *A&A,* 2002, vol. 386, 492-503
- **Reinish et al.** *Thromb. & Haemostas.,* 1988, vol. 60, 518-523
- **Fiske et al.** *J. Biol. Chem.,* 1935, vol. 66, 375-389
- **Yang et al.** *J. Am. Chem. Soc.,* 1995, vol. 117, 4843-4850
- **Bonte et al.** *Biochim. Biophys. Acta.,* 1987, vol. 900, 1-9
- **McGann et al.** *Cryobiology,* 1976, vol. 13, 261-268
- **Olson et al.** *Biochim. Biophys. Acta.,* 1979, vol. 557, 9-23
- **Muhlen, A. et al.** *Pharm. Res.,* 1996, vol. 13, 1411-1416
- **Radmacher et al.** *Science,* 1992, vol. 257, 1900-1905
- **Anabousi et al.** *European Journal of Pharmaceutics and Biopharmaceutics,* vol. 60, 295-303
- **Ruozi et al.** *European Journal of Pharmaceutical Sciences,* 2005, vol. 25, 81-89
- **Constantinescu et al.** *Artificial Cells, Blood Substitutes and Biotechnology,* 2003, vol. 31, 394-424
- **Mordon et al.** *Microvascular Research,* 2001, vol. 63, 315-325
- **van Oss.** *J. Mol. Recognit,* 2003, vol. 16, 177-190
- **Lal et al.** *Eur. Phys. J.,* 2004, vol. E15, 217-223
- **van Oss.** *J. Mol. Recognit.,* 2003, vol. 16, 177-190
- **Amsden.** *Macromolecules,* 1998, vol. 31, 8382-8395
- **Garbuzenko et al.** *Chemistry and Physics of Lipids,* 2005, vol. 135, 117-129
- **Ohki et al.** *Biochemistry,* 1998, vol. 37, 7496-7503
- **Melikyan et al.** *Biophys. J.,* 1996, vol. 71, 2680-2691
- **Behravesh et al.** *Biomaterials,* 2003, vol. 24, 4365-4374
- **Stringer et al.** *J. Controlled Release,* 1996, vol. 42, 195-202
- **Cruise et al.** *Biomaterials,* 1998, vol. 19, 1287-1294
- **Baba et al.** *J. Chromatography A,* 2004, vol. 1040, 45-51
- **Goni et al.** *Eur. J. Biochem.,* 1986, vol. 160, 659-665
- **Meryman.** *Cryobiology,* 1971, vol. 8, 489-500
- **Bangham et al.** *Chemistry and Physics of Lipids,* 1967, vol. 1, 225-246
- **Arnold et al.** *Biochim. Biophys. Acta,* 1983, vol. 728, 121-128
- **Brasseur R.** Differentiation of lipid-associating helices by use of 3-dimensional molecular hydrophobicty potential. *J. Biol. Chem.,* 1991, vol. 266-24, 16120-16127
- **Kohonen T.** *The Self-Organizing Map. Proceedings IEEE,* 1990, vol. 78, 1464-80
- **Del Carpio CA.** A parallel genetic algorithm for polypeptide three dimensional structure prediction. A transputer implementation. *J. Chem. Inf. and Comp. Sci.,* 1996, vol. 36, 258-269
- **Ponder JA. ; Case DA.** Force fields for protein simulations. *Adv. Prot. Chem.,* 2003, vol. 66, 27-85
- **Richichi A. ; Percheron I.** CHARM: A catalog of high angular resolution measurements. *A&A,* 2002, vol. 386, 492-503
- **Del Carpio CA. ; Qiang P. ; Ichiishi E. ; Koyama M. ; Kubo M. ; Endou A. ; Takaba H. ; Miyamoto A.** Robotic path planning and protein complex modeling considering low frequency intra-molecular loop and domain motions. *Genome Informatics,* 2006, vol. 17, 270-278
- **Del Carpio CA ; Ichiishi E. ; Yoshimori A. ; Yoshikawa T.** MIAX: A new paradigm to model bio-molecular interaction and complex formation in condensed phases. *Proteins: Structure, Function and Genetics,* 2002, vol. 48, 696-732

- **Ponder JA ; Case DA.** Force fields for protein simulations. *Adv. Prot. Chem.,* 2003, vol. 66, 27-85
- **Wallace AC ; Laskowski RA. ; Thomton J M.** LIGPLOT: A program to generate schematic diagrams of protein-ligand interactions. *Prot. Eng.,* 1995, vol. 8, 127-134
- **Del Carpio CA ; Ichiishi E. ; Yoshimori A. ; Yoshikawa T.** MIAX: A new paradigm to model bio-molecular interaction and complex formation in condensed phases. *Proteins: Structure, Function and Genetics,* 2002, vol. 48, 696-732
- **Shimizu A. ; Matsushita T. ; Kondo T. ; Inden Y. ; Kojima T. ; Saito H. ; Hirai M.** Identification of the amino residues of the platelet glycoprotein Ib (GPIb) essential for the von Willebrand Factor binding by clustered charged-to alanine scanning mutagenesis. *Journal of Biol. Chem.,* 2004, vol. 279-16, 16285-16294
- **Hauert J. ; Femandez-Carneado J. ; Michielin O. ; Mathieu S. ; Grell D. ; Schapira M. ; Spertini O. ; Mutter M. ; Tuchscherer G. ; Kovacsovics T.** A template-assembled synthetic protein surface mimetic of the von Willebrand factor A1 domain inhibits botrocetin-induced platelet aggregation. *Chembiochem,* 2001, vol. 5, 856-64
- **Xu D. ; Tsai CJ. ; Nussinov R.** Hydrogen bonds and salt bridges across protein-protein interfaces. *Protein Engineering,* 1997, vol. 10, 999-1012
- **Wallace AC ; Laskowski RA. ; Thomton J M.** LIGPLOT: A program to generate schematic diagrams of protein-ligand interactions. *Prot. Eng.,* 1995, vol. 8, 127-134
- **Del Carpio CA ; Rajjack SA ; Koyama M ; Kubo M ; Ichiishi E ; Miyamoto A.** A graph theoretical approach for analysis of protein flexibility change at protein complex formation. *Genome Informatics,* 2005, vol. 16, 148-160
- **Mather B.D. et al.** Michael addition reactions in macromolecular design for emerging technologies. *Prog. Polym. Sci.,* 2006, vol. 31, 487-531

SEQUENCE LISTING

<110> Canadian Blood Services

<120> SYNTHETIC PLATELETS

<130> 13453-91PCT

<150> US 60/842,647
<151> 2006-09-07

<160> 22

<170> PatentIn version 3.4

<210> 1
<211> 15
<212> PRT
<213> Artificial

<220>
<223> peptide

<400> 1

```
Val Ser Arg Gln Asn Gly Lys Gln Tyr Trp Ala Ile Lys Glu Gly
1               5                   10                  15
```

<210> 2
<211> 15
<212> PRT
<213> Artificial

<220>
<223> peptide

<400> 2

```
Trp Gln Asn Glu Gly Thr His Val Leu Ser Arg Cys Tyr Glu Cys
1               5                   10                  15
```

<210> 3
<211> 15
<212> PRT
<213> Artificial

<220>
<223> peptide

<400> 3

Arg Ser Ala Arg Met Gln Val Cys Trp Asn Ala Phe Lys Asn Arg
1 5 10 15

<210> 4
<211> 15
<212> PRT
<213> Artificial

<220>
<223> peptide

<400> 4

Asp Ser Cys Pro Arg Asp Trp Asp Asn Asn Phe Leu Phe Phe Glu
1 5 10 15

<210> 5
<211> 15
<212> PRT
<213> Artificial

<220>
<223> peptide

<400> 5

Gly Glu Lys Ile Ala Trp Tyr Gln Lys Gly Asn Gln Arg Ser Val
1 5 10 15

<210> 6
<211> 15
<212> PRT
<213> Artificial

<220>
<223> peptide

<400> 6

Cys Glu Tyr Cys Arg Ser Leu Val His Thr Gly Glu Asn Gln Trp
1 5 10 15

<210> 7
<211> 15
<212> PRT
<213> Artificial

<220>
<223> peptide

<400> 7

Arg Asn Lys Phe Ala Asn Trp Cys Val Gln Met Arg Ala Ser Arg
1 5 10 15

<210> 8
<211> 15
<212> PRT
<213> Artificial

<220>
<223> peptide

<400> 8

Glu Phe Phe Leu Phe Asn Asn Asp Trp Asp Arg Pro Cys Ser Asp
1 5 10 15

<210> 9
<211> 18
<212> PRT
<213> Artificial

<220>
<223> peptide

<220>
<221> MISC_FEATURE
<222> (3)..(5)
<223> Xaa is an uncharged amino acid

<220>
<221> MISC FEATURE
<222> (7)..(8)
<223> Xaa is an uncharged amino acid

<220>
<221> MISC_FEATURE
<222> (10)..(11)
<223> Xaa is an uncharged amino acid

<220>
<221> MISC_FEATURE
<222> (16)..(16)
<223> Xaa is an uncharged amino acid

<400> 9

Val Ala Xaa Xaa Xaa Lys Xaa Xaa Phe Xaa Xaa Glu Asp Val Lys Xaa
1 5 10 15

Met Thr

<210> 10
<211> 18
<212> PRT
<213> Artificial

<220>
<223> peptide

<220>
<221> MISC_FEATURE
<222> (3)..(3)
<223> Xaa is an uncharged amino acid

<220>
<221> MISC_FEATURE
<222> (8)..(9)
<223> Xaa is an uncharged amino acid

<220>
<221> MISC_FEATURE
<222> (11)..(12)
<223> Xaa is an uncharged amino acid

<220>
<221> MISC_FEATURE
<222> (14)..(16)
<223> Xaa is an uncharged amino acid

<400> 10

Thr Met Xaa Lys Val Asp Glu Xaa Xaa Phe Xaa Xaa Lys Xaa Xaa Xaa
1 5 10 15

Ala Val

<210> 11
<211> 10
<212> PRT
<213> Artificial

<220>
<223> peptide

<400> 11

Ser His Ala Tyr Ile Gly Leu Lys Asp Arg
1 5 10

<210> 12
<211> 10
<212> PRT
<213> Artificial

<220>
<223> peptide

<400> 12

Glu Val Leu Lys Tyr Thr Leu Phe Gln Ile
1 5 10

<210> 13
<211> 10
<212> PRT
<213> Artificial

<220>
<223> peptide

<400> 13

Arg Asp Lys Leu Gly Ile Tyr Ala His Ser
1 5 10

<210> 14
<211> 10
<212> PRT
<213> Artificial

<220>
<223> peptide

<400> 14

Ile Gln Phe Leu Thr Tyr Lys Leu Val Glu
1 5 10

<210> 15
<211> 15
<212> PRT
<213> Artificial

<220>
<223> peptide

<400> 15

Ser Met Thr Ser Met Cys Tyr Leu Ile Gly Ala Pro Lys Tyr Lys
1 5 10 15

<210> 16
<211> 15
<212> PRT
<213> Artificial

<220>
<223> peptide

<400> 16

Lys Tyr Gln Cys Tyr Ala Pro Ala His Pro Ser Tyr Val Asn Tyr
1 5 10 15

<210> 17
<211> 15
<212> PRT
<213> Artificial

<220>
<223> peptide

<400> 17

Phe Lys Trp Ser Trp Glu Trp Gln Gly Gln Glu Ala Tyr Tyr Asp
1 5 10 15

<210> 18
<211> 15
<212> PRT
<213> Artificial

<220>
<223> peptide

<400> 18

Phe Arg Ile Tyr Tyr Val Tyr Thr Thr Ser Gln Gln Asp Ser Cys
1 5 10 15

<210> 19
<211> 15
<212> PRT
<213> Artificial

<220>
<223> peptide

<400> 19

Lys Tyr Lys Pro Ala Gly Ile Leu Tyr Cys Met Ser Thr Met Ser
1 5 10 15

<210> 20
<211> 15
<212> PRT
<213> Artificial

<220>
<223> peptide

<400> 20

Tyr Asn Val Tyr Ser Pro His Ala Pro Ala Tyr Cys Gln Tyr Lys
1 5 10 15

<210> 21
<211> 15
<212> PRT
<213> Artificial

<220>
<223> peptide

<400> 21

Asp Tyr Tyr Ala Glu Gln Gly Gln Trp Glu Trp Ser Trp Lys Phe
1 5 10 15

<210> 22
<211> 15
<212> PRT
<213> Artificial

<220>
<223> peptide

<400> 22

Cys Ser Asp Gln Gln Ser Thr Thr Tyr Val Tyr Tyr Ile Arg Phe
1 5 10 15